# EUROPEAN PATENT APPLICATION

(11) **EP 4 831 193 A1**
(43) Date of publication of application: **28.10.2026**
(21) Application number: 25382412.2
(22) Date of filing: 24.04.2025
(51) Int. Cl.: C07K 14/47, A61K 40/11, A61K 40/32, A61P 35/00, C07K 14/705, C07K 14/725, C07K 14/82

(54) **T CELL RECEPTORS AND USES THEREOF**

(71) Applicant: Fundació Privada Institut d'Investigació Oncològica de Vall Hebron, 08035 Barcelona (ES)
(72) Inventor: LOZANO RABELLA, Maria, E-08035 Barcelona (ES); GARCIA GARIJO, Andrea, E-08035 Barcelona (ES); YUSTE ESTEVANEZ, Anna, E-08035 Barcelona (ES); GROS VIDAL, Alena, E-08035 Barcelona (ES)
(74) Representative: ABG Intellectual Property Law, S.L.

(57) **Abstract**

The invention relates to the field of immunotherapeutics and, more particularly, to an antigen recognizing construct specifically binding to a complex of an antigenic peptide and a HLA molecule, wherein said antigen recognizing construct is selected from a T cell receptor or an antigen-binding fragment thereof. The invention also relates to use of the antigen recognizing constructs for diagnosis and therapy.

## Description

### FIELD OF THE INVENTION

The present invention pertains to novel antigen recognizing constructs according to the appended set of claims, which are T cell receptors. The constructs of the invention are particularly useful for the diagnosis, prevention or therapy of tumorous diseases. Furthermore provided are nucleic acids, vectors and host cells which encode, comprise or present the antigen recognizing constructs of the invention. The invention thus provides new means for immune therapy, specifically adoptive T cell therapy, for treating cancer.

### BACKGROUND ART

The identification of particular TCR is advantageous for the development of novel immunotherapies. Methods for the identification of TCRs with specificity characteristics amenable to therapeutic use have a high attrition rate and thus do not provide the skilled person with a reasonable expectation of success.

In the first instance, the skilled person needs to identify a suitable starting, or scaffold, sequence. Typically such sequences are obtained from natural sources e.g. from antigen responding T cells extracted from donor blood. Given the rarity of cancer specific T cells in the natural repertoire, it is often necessary to screen many donors before a responding T cell may be found. The screening process may take several weeks or months, and even when a responding T cell is found, it may be unsuitable for immunotherapeutic use. For example, the response may too weak and/or may not be specific for the target antigen, alternatively it may not be possible to generate a clonal T cell population, nor expand or maintain a given T cell line to produce sufficient material to identify the correct TCR chain sequences.

TCRs as they exist in nature have weak affinity for target antigen (low micromolar range) compared with antibodies, and TCRs against cancer antigens typically have weaker antigen recognition than viral specific TCRs. This weak affinity coupled with HLA down-regulation on cancer cells means that therapeutic TCRs for cancer immunotherapy require engineering to increase their affinity for target antigen and thus generate a more potent response. However, to produce significant increases in the affinity of a given TCR against a given target requires the skilled person to select specific mutations and / or combinations of mutations from a large pool of possible alternatives. The specific mutations and/or combinations of mutations that produce significant increases in affinity are not predictable and there is a high attrition rate. In many cases it may not be possible to achieve significant increases in affinity with a given TCR starting sequence.

The skilled person must also select mutations and or combinations of mutations that allow the TCR to retain a high specificity for target antigen and demonstrate a good safety profile in preclinical testing. Again such mutations and/or combinations of mutations are not predictable. The attrition rate at this stage is even higher and in many cases may not be achievable at all from a given TCR starting sequence.

The mutations required for high affinity and high specificity should also produce a TCR that is able to be expressed, refolded and purified at a reasonable yield and that is highly stable in a purified form.

Despite the difficulties described above of identifying TCR sequences with suitable characteristics for therapeutic use, the inventors have surprisingly found TCR sequences that provide an ideal starting point, or scaffold, to produce therapeutic TCRs.

### SUMMARY OF THE INVENTION

By performing personalized immunological screenings in metastatic cancer patients with different tumor types, the authors of the present invention have identified multiple TCRs targeting different tumor antigens. This includes TCRs targeting non-canonical or cryptic antigens, cancer germline antigens and neoantigens derived from hotspot mutations.

The identification of TCR targeting tumor antigens is of great importance for the development of effective T-cell therapies that can exploit their capacity to kill tumor cells and eradicate the tumor.

In a first aspect the present invention relates an antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein:
(a) the antigenic peptide comprises the amino acid sequence FFDETRQL (SEQ ID NO: 1) and the HLA molecule is HLA C*04:01; or
(b) the antigenic peptide comprises the amino acid sequence SCMGGMNQR ( SEQ ID NO: 2) and the HLA molecule is HLA A*11:01.

In a second aspect, the present invention relates to an antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein the antigen recognizing construct comprises:
(a) a TCR alpha chain variable domain comprising a CDR3 comprising SEQ ID NO: 5 or functionally equivalent variant thereof and a TCR beta chain variable domain comprising a CDR3 comprising SEQ ID NO: 8 or a functionally equivalent variant thereof; or
(b) a TCR alpha chain variable domain comprising a CDR3 comprising SEQ ID NO: 17 or functionally equivalent variant thereof and a TCR beta chain variable domain comprising a CDR3 comprising SEQ ID NO: 20 or a functionally equivalent variant thereof; or
(c) a TCR alpha chain variable domain comprising a CDR3 comprising SEQ ID NO: 29 or functionally equivalent variant thereof and a TCR beta chain variable domain comprising a CDR3 comprising SEQ ID NO: 32 or a functionally equivalent variant thereof; or
(d) a TCR alpha chain variable domain comprising a CDR3 comprising SEQ ID NO: 41 or a functionally equivalent variant thereof and a TCR beta chain variable domain comprising a CDR3 comprising SEQ ID NO: 44 or a functionally equivalent variant thereof; or
(e) a TCR alpha chain variable domain comprising a CDR3 comprising SEQ ID NO: 53 or a functionally equivalent variant thereof and a TCR beta chain variable domain comprising a CDR3 comprising SEQ ID NO: 56 or a functionally equivalent variant thereof; or
(f) a TCR alpha chain variable domain comprising a CDR3 comprising SEQ ID NO: 65 or a functionally equivalent variant thereof and a TCR beta chain variable domain comprising a CDR3 comprising SEQ ID NO: 68 or a functionally equivalent variant thereof; or
(g) a TCR alpha chain variable domain comprising a CDR3 comprising SEQ ID NO: 77 or a functionally equivalent variant thereof and a TCR beta chain variable domain comprising a CDR3 comprising SEQ ID NO: 80 or a functionally equivalent variant thereof; or
(h) a TCR alpha chain variable domain comprising a CDR3 comprising SEQ ID NO: 89 or a functionally equivalent variant thereof and a TCR beta chain variable domain comprising a CDR3 comprising SEQ ID NO: 92 or a functionally equivalent variant thereof; or
(i) a TCR alpha chain variable domain comprising a CDR3 comprising SEQ ID NO: 101 or a functionally equivalent variant thereof and a TCR beta chain variable domain comprising a CDR3 comprising SEQ ID NO: 104 or a functionally equivalent variant thereof;
(j) a TCR alpha chain variable domain comprising a CDR3 comprising SEQ ID NO: 113 or a functionally equivalent variant thereof and a TCR beta chain variable domain comprising a CDR3 comprising SEQ ID NO: 116 or a functionally equivalent variant thereof and
(k) a TCR alpha chain variable domain comprising a CDR3 comprising SEQ ID NO: 125 or a functionally equivalent variant thereof and a TCR beta chain variable domain comprising a CDR3 comprising SEQ ID NO: 128 or a functionally equivalent variant thereof.

In a third aspect, the present invention relates to a nucleic acid encoding the antigen recognizing construct according to the previous aspect of the invention.

In a fourth aspect, the present invention relates to an expression vector comprising a nucleic acid according to the third aspect of the invention.

In a fifth aspect, the present invention relates to a nucleic acid composition encoding the antigen recognizing construct according to the first and second aspect of the invention selected from the group consisting of:
(i) a composition comprising a first nucleic acid encoding the alpha chain of a TCR and a second nucleic acid encoding the beta chain of said TCR,
(ii) a composition comprising a first nucleic acid encoding a fusion protein comprising the alpha chain of a TCR and a second nucleic acid encoding the beta chain of said TCR,
(iii) a composition comprising a first nucleic acid encoding the alpha chain of a TCR and a second nucleic acid encoding a fusion protein comprising the beta chain of said TCR and a second polypeptide.

In a sixth aspect, the present invention relates to a cell comprising an antigen recognizing construct according to the first and second aspect of the invention, a nucleic acid construct according to the third aspect of the invention, a vector according to the fourth aspect of the invention or the nucleic acid composition according to the fifth aspect of the invention.

In a seventh aspect, the present invention relates to a pharmaceutical composition comprising an antigen recognizing construct according to the first and second aspect of the invention, a nucleic acid construct according to the third aspect of the invention, a vector according to the fourth aspect of the invention or the nucleic acid composition according to the fifth aspect of the invention, or the cell according to the sixth aspect of the invention together with one or more pharmaceutically acceptable carriers or excipients.

In an eight aspect, the present invention relates to an in vitro method of detecting cancer in a biological sample comprising:
(a) contacting the biological sample with the antigen recognizing construct of any of the previous aspects, and
(b) detecting binding of the antigen recognizing construct to the biological sample which has a cancer that express a tumoral antigen which comprises the antigenic peptide recognized by the antigen recognizing construct when the antigenic peptide is linked with a HLA.

In a ninth aspect, the present invention relates to the antigen recognizing construct according to the first and second aspect of the invention, the nucleic acid construct according to the third aspect of the invention, the vector according to the fourth aspect of the invention, the nucleic acid composition according to the fifth aspect of the invention, the cell according to the sixth aspect of the invention for use in medicine or the pharmaceutical composition according to the seventh aspect of the invention.

In a tenth aspect, the present invention relates to the antigen recognizing construct according to the first and second aspect of the invention, the nucleic acid construct according to the third aspect of the invention, the vector according to the fourth aspect of the invention, the nucleic acid composition according to the fifth aspect of the invention, the cell according to the sixth aspect of the invention or the pharmaceutical composition according to the seventh aspect of the invention for use in the treatment of cancer.

### DESCRIPTION OF THE FIGURES

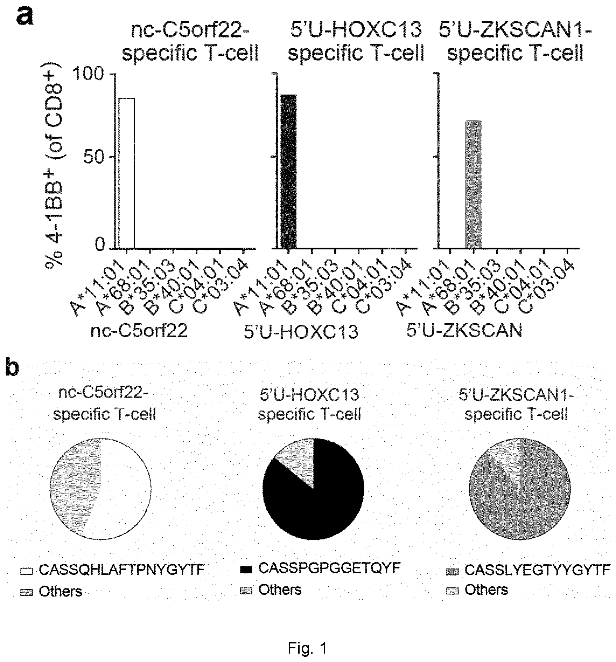

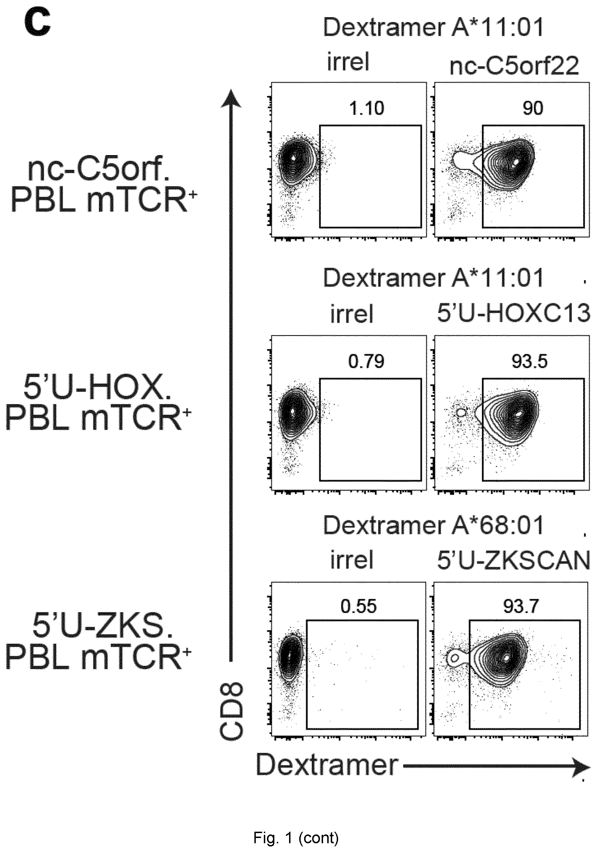

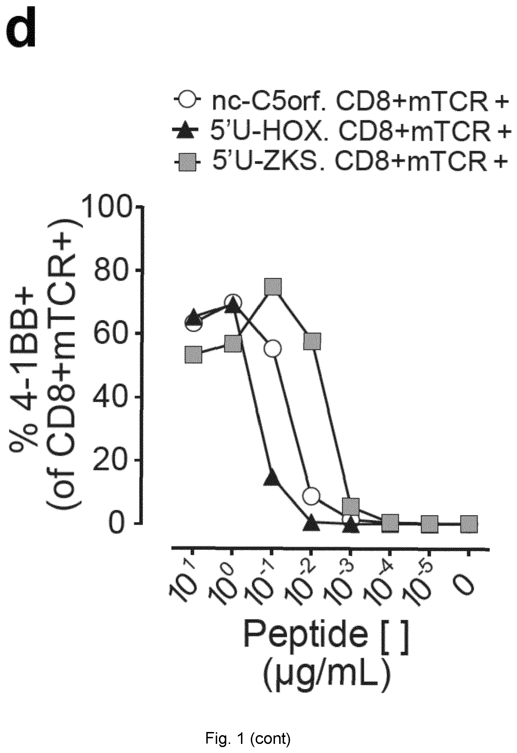

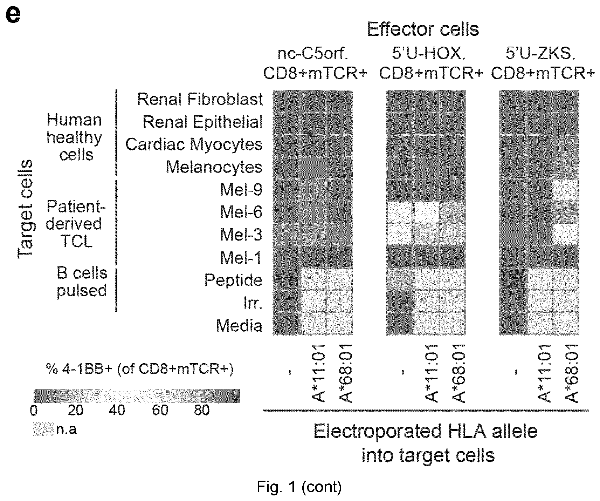

**Figure 1****: Identification of TCRs targeting cryptic antigens.** a) Restriction element was evaluated by coculturing enriched T-cell populations recognizing cryptic antigens with COS-7 cells expressing the donor HLA alleles and pulsed with the corresponding peptides. b) Pie charts show the frequency of the most dominant TCR identified by single-cell RNA sequencing of T-cell populations enriched for the recognition of cryptic antigens. The sequence of the CDR3 beta chain is depicted. c) FACS plots showing the staining of TCR-transduced cells with HLA-dextramers generated loading each cryptic peptide with the respective HLA restriction element. Irrelevant peptides were loaded in HLA dextramers as a negative control. d) TCR-transduced T cells purified by FACS sorting (CD8+mTCR+) were cocultured with B cells pulsed with serial dilutions of the corresponding peptide. e) Expression and translation of cryptic antigens in healthy human cells and selected tumor cell lines (TCLs) was indirectly evaluated by coculturing control and electroporated target cells with RNA encoding the specified HLA alleles with sort purified TCR-transduced T cells. T-cell activation was assessed by measuring 4-1BB expression on CD8+mTCR+. B cells were pulsed with the peptide of interest as a positive control. Mel. Melanoma cell line. n.a. not assessed.

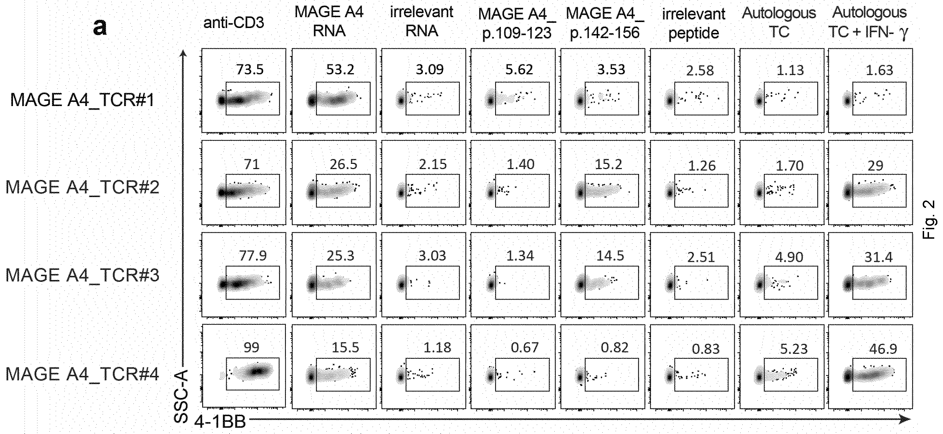

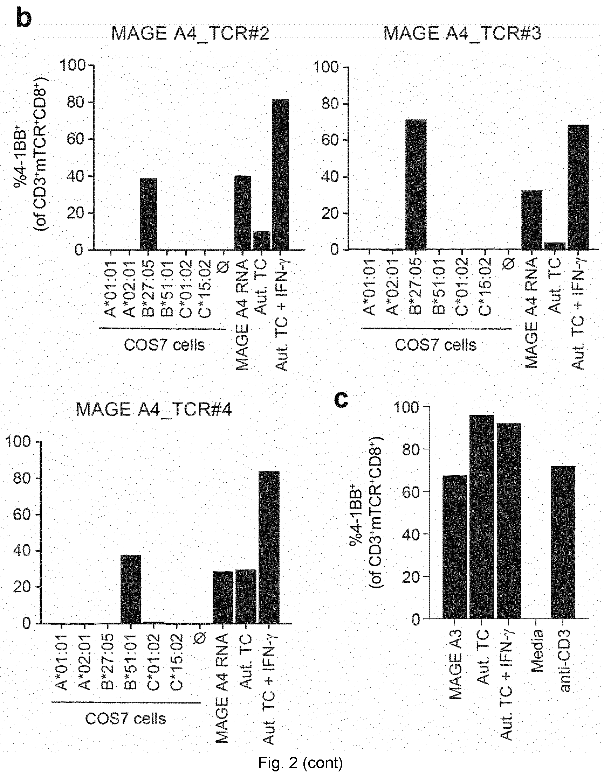

**Figure 2****: Identification of TCRs targeting cancer germline antigens.** a) Flow cytometry plots show the reactivity of TCR-transduced cells with the four different TCRs recognizing MAGE-A4. b) Restriction element was evaluated by coculturing T cells transduced with the different TCRs recognizing MAGE-A4 with COS-7 cells co-transfected with the donor HLA alleles and MAGE A4 DNA. c) T cells transduced with a TCR recognizing MAGE-A3 were co-cultured with autologous CD4+ T cells pulsed with MAGE-A3 peptide and with the autologous tumor cell line, treated or not with IFN-γ. Media was used as a negative control while anti-CD3 was used as a positive control.TC = tumor cell line. Data is representative from two independent experiments.

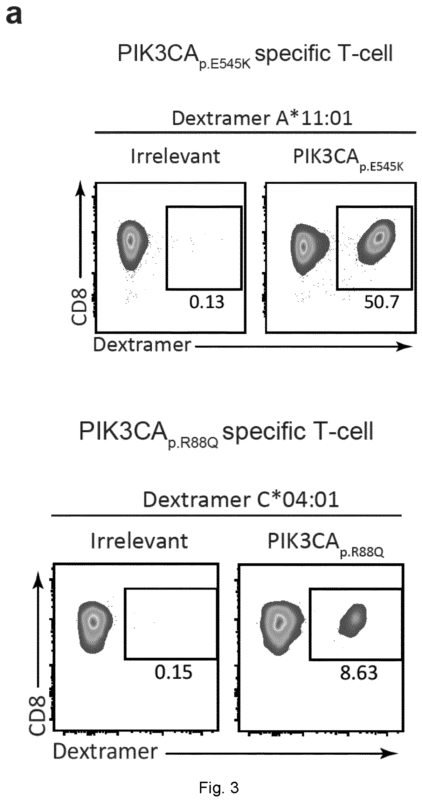

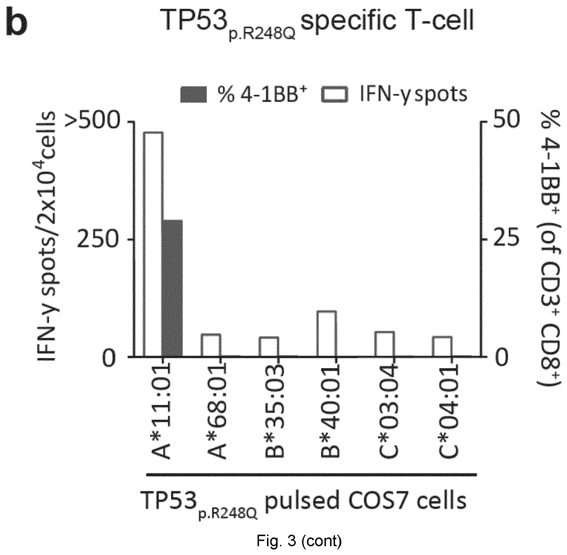

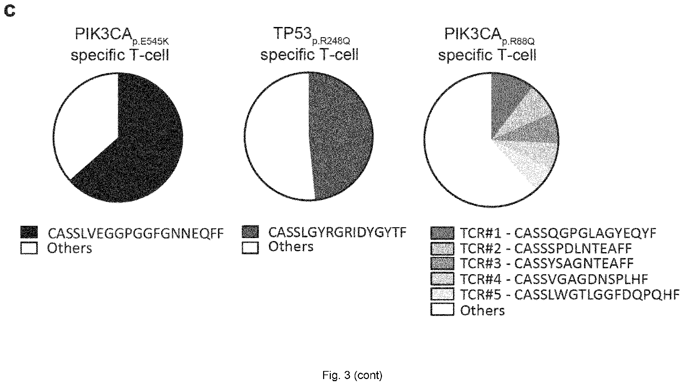

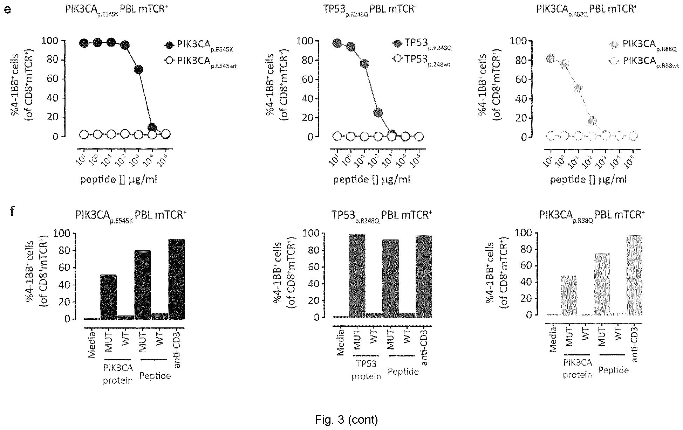

**Figure 3****. Identification of TCRs targeting shared neoantigens.** a) Flow cytometry analysis of enriched T-cell populations binding to HLA-dextramers loaded with hotspot-derived peptides. FACS plots show the frequency of T cells binding to HLA-A*11:01 dextramers loaded with PIK3CAp.E545K (left) or to HLA-C*04:01 dextramers loaded with PIK3CAp.R88Q (right). Irrelevant peptides loaded in HLA dextramers served as negative controls. b) Restriction element of TP53p.R248Q peptide was evaluated by coculturing the enriched T-cell population recognizing TP53p.R248Q with COS7 cells individually expressing one of the donor HLA alleles and pulsed with the targeted peptide derived from TP53p.R248Q. The number of IFN-γ spots and frequency of 4-1BB+ cells gated on CD3+CD8+ T cells after 20h coculture is shown. c) Pie charts showing the frequency of the most dominant TCR-β chains identified by TCR-β deep sequencing of T-cell populations recognizing PIK3CAp.E545K, TP53p.R248Q and PIK3CAp.R88Q. The sequence of the CDR3 beta chains is depicted. d) Heat map summarizing the frequency of HLA-dextramer positive cells of CD8+ from TCR-transduced T cells with the candidate TCRs identified from PIK3CAp.R88Q specific T-cell population. HLA-C*04:01-dextramers were loaded with the targeted PIK3CAp.R88Q peptide or an irrelevant peptide that was used as a negative control e) TCR-transduced T cells were cocultured with B cells or tumor cells pulsed with serial dilutions of the corresponding mutated peptide. Serial dilutions of the wildtype counterpart were also pulsed into the same target cells to determine if the TCR recognized the mutated peptide in a mutation-specific manner. f) Reactivity of TCR-transduced T cells against an HLA-A*11:01+HLA-C*04:01+ patient-derived tumor cell line expressing the cognate mutant or wildtype hotspot-derived neoantigens. T cells transduced with TCRs recognizing PIK3CAp.E545K (left), TP53p.R248Q (middle) or PIK3CAp.R88Q were cocultured with a tumor cell line electroporated with the full-length mutant protein or pulsed with the corresponding mutant peptide. The wild-type (WT) versions of the full-length protein and the peptide were also included. Media and anti-CD3 are negative and positive controls, respectively. The frequency of 4-1BB+ cells gated on CD8+mTCR+ after 20h of coculture with the specified targets is shown. Data is representative from two independent experiments.

### DETAILED DESCRIPTION OF THE INVENTION

### Antigen recognizing construct

In a first aspect the present invention relates an antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein:
(a) the antigenic peptide comprises the amino acid sequence FFDETRQL (SEQ ID NO: 1) and the HLA molecule is HLA C*04:01; or
(b) the antigenic peptide comprises the amino acid sequence SCMGGMNQR ( SEQ ID NO: 2) and the HLA molecule is HLA A*11:01.

The term "specifically binding", as used herein, refer to the recognition and binding of two entities (for example, the TCR variable domain and the complex of an antigenic peptide and a HLA molecule) in the absence of substantially recognition and binding other molecules in a sample. In some embodiments, a TCR can have, for example, an affinity for the antigen-HLA complex which is at least 10, 100, 1000 or 10,000 times greater than the affinity of the TCR for another antigen-HLA complex in a sample. In some embodiments the ligand can have an antigen-HLA complex binding dissociation constant (K_{D}) between about 0.01 nM and about 100 nM, or any value in between such as 0.1 nM.

The term "HLA" or "human leukocyte antigen", as used herein, refers to the major histocompatibility antigens (MHC) of humans, which are encoded by the genes found on the locus that forms the HLA system.

As used herein, the term "antigenic peptide" refers to a protein-based molecule comprising one or more epitopes that are able to stimulate the immune system in an organism in order to generate an antigen-specific humoral or cellular response.

Antigen specificity is conferred by the variable regions of the alpha and beta chain. Both variable domains of the TCR alpha chain and beta chain comprise three hypervariable or complementarity determining regions (CDR1alpha/beta, CDR2alpha/beta and CDR3 alpha/beta) surrounded by framework (FR) regions. CDR3 is the prime determinant of antigen recognition and specificity (i.e. the ability to recognize and interact with a specific antigen), whereas CDR1 and CDR2 mainly interact with the MHC molecule presenting the antigenic peptide.

Each variable region, herein referred to as alpha variable domain and beta variable domain, comprises three "complementarity determining regions" (CDRs) embedded in a framework sequence, one being the hypervariable region named CDR3. The TCR alpha variable domain CDRs are referred to as CDRa1, CDRa2, CDRa3, and the TCR beta variable domain CDRs are referred to as CDRb1, CDRb2, CDRb3. There are several types of alpha chain variable (Valpha) regions and several types of beta chain variable (Vbeta) regions distinguished by their framework, CDR1 and CDR2 sequences, and by a partly defined CDR3 sequence.

In some embodiments of the present invention, the antigen recognizing construct defined in the first aspect of the invention is selected from a group consisting of:
- a T cell receptor (TCR)
- an antigen-biding fragment of a TCR
- a fusion protein comprising a TCR or an antigen-biding fragment of a TCR
- a single chain TCR

The term "T cell receptor" or "TCR", as used herein, includes native TCRs as well as TCR variants, fragments and constructs. The term thus includes heterodimers comprising TCR alpha and beta chains as well as multimers; optionally comprising further domains and/or moieties. T-cell receptors (TCR) are protein complexes formed by six different polypeptides. In most T cells, TCRs are composed of αβ subunits displaying immunoglobulin-like variable domains that recognize peptide antigens associated with HLA molecules expressed on the presenting cells. These TCR αβ subunits are then associated with the CD3 complex formed by the γ, δ, ε and ζ subunits generating the final complex of six polypeptides.

In its native form, the TCR exists as a complex of several proteins on the surface of T cells. The T cell receptor is composed of two (separate) protein chains, which are produced from the independent T cell receptor alpha and beta (TCR alpha and TCR beta) genes and are called alpha and beta chains. Each chain of the TCR possesses one N-terminal immunoglobulin-like (Ig)-variable (V) domain/region, one Ig-constant-like (C) domain/region, a transmembrane/cell membrane-spanning region anchoring the chain in the plasma membrane, and a short cytoplasmic tail at the C-terminal end.

The alfa and beta chains of a beta TCR's are generally regarded as each having two "domains" or "regions", namely variable and constant domains/regions. The terms "domain(s)" and "region(s)" are used interchangeably herein. The variable domain consists of a concatenation of variable region and joining region. In the present specification and claims, the term "TCR alpha variable domain" therefore refers to the concatenation of TRAV and TRAJ regions, and the term TCR alpha constant domain refers to the extracellular TRAC region, or to a C-terminal truncated TRAC sequence. Likewise the term "TCR beta variable domain" refers to the concatenation of TRBV and TRBD/TRBJ regions, and the term TCR beta constant domain refers to the extracellular TRBC region, or to a C-terminal truncated TRBC sequence. The variable regional and the constant region are connected by a J (joining) domain.

In the present specification and claims, the term "TCR alpha extracellular domain" refers to the part of the TCR alpha chain which is exposed outside the cell and which is formed by the alpha chain variable region and the TCR alpha chain constant region but lackng the alpha chain transmembrane and cytosolic regions. Likewise the term "TCR beta extracellular domain" refers to the part of the TCR beta chain which is exposed outside the cell and which is formed by the TCR beta chain variable region and the beta chain constant region but lacking the TCR beta transmembrane and cytosolic regions.

As used herein, the term "antigen-binding fragment", "antigen-binding region" or "antigen-binding portion" are used interchangeably and refer to any oligopeptide or polypeptide that can bind to a certain antigen. In the present invention the antigen-binding fragment is the variable domain of the TCR. Concretely, the antigen-biding fragment comprises the variable domains of the TCR alpha and the TCR beta.

In some instances, the TCR is an antigen-binding fragment that is less than a full-length TCR but that binds to a specific peptide bound in a MHC molecule, such as binds to a MHC-peptide complex. In some cases, an antigen-binding fragment of a TCR can contain only a portion of the structural domains of a full-length or intact TCR, but yet is able to bind the peptide epitope, such as MHC-peptide complex, to which the full TCR binds. In some cases, an antigen-binding fragment contains the variable domains of a TCR, such as variable a chain and variable beta chain of a TCR, sufficient to form a binding site for binding to a specific MHC-peptide complex. Generally, the variable chains of a TCR contain complementarity determining regions involved in recognition of the peptide, MHC and/or MHC-peptide complex.

A conventional TCR antigen-binding site usually includes six CDRs, comprising the CDR set from each of an alpha and a beta chain variable region, wherein CDR1 and CDR3 sequences are relevant to the recognition and binding of the peptide antigen that is bound to the MHC protein and the CDR2 sequences are relevant to the recognition and binding of the MHC protein. Analogous to antibodies, TCRs comprise framework regions which are amino acid sequences interposed between CDRs, i.e. to those portions of TCR alpha and beta chain variable regions that are relatively conserved among different TCRs. The alpha and beta chains of a TCR each have four FRs, herein designated FR1-a, FR2-a, FR3-a, FR4-a, and FR1-b, FR2-b, FR3-b, FR4-b, respectively. Accordingly, the alpha chain variable domain may thus be designated as (FR1-a)-(CDRa1)-(FR2-1)-(CDRa2)-(FR3-a)-(CDRa3)-(FR4-a) and the beta chain variable domain may thus be designated as (FR1-b)-(CDRb 1)-(FR2-b)-(CDRb2)-(FR3-b)-(CDRb3)-(FR4-b).

As used herein, the term "fusion protein" relates to proteins which consist of two or more functional domains derived from different proteins. A fusion protein may be obtained by conventional means, e.g., by means of gene expression of the nucleotide sequence encoding for said fusion protein in a suitable cell. The fusion protein, per definition, is never found in nature as such. In a particular embodiment, the antigen recognizing construct according to the present invention is a TCR or a fragment thereof.

In another embodiment, the antigen recognizing construct according to the present invention is soluble TCR.

The TCR according to the present invention is formed by an alpha and beta chains wherein the alpha and beta chain contain, from the N- to the C-terminus: a variable region, a constant region, a J region, a C region or connecting domain, a transmembrane region and a cytoplasmic region.

As used herein, a "soluble TCR" or sTCR refers to a molecule which contains at least the variable parts of the TCR alpha and TCR beta chains but which lacks the elements that are needed for its anchoring into the plasma membrane, i.e. it lacks at least the transmembrane domain and the cytosolic domain.

In certain embodiments, the soluble TCR is a molecule selected from the group consisting of
(i) a fusion protein comprising the TCR alpha chain variable region and the TCR beta chain variable region,
(ii) a fusion protein comprising a first region comprising the TCR alpha chain extracellular region and a second region comprising the TCR beta chain extracellular sequence,
(iii) A first polypeptide comprising the TCR alpha chain variable region and a second polypeptide comprising the TCR beta chain variable region wherein the first and second polypeptides are connected by a disulfide bond or
(iv) A first polypeptide comprising the TCR alpha chain extracellular region and a second polypeptide comprising the TCR beta chain extracellular region wherein the first and second are connected by a disulfide bond

Both the soluble TCRs defined under (i) and (ii) are single chain TCR in that they consist of a single polypeptide chain which comprises regions derived from the alpha chain and form the beta chain. The soluble TCRs defined under (iii) and (iv) are heterodimers which comprise a first polypeptide containing elements from the alpha chain and elements from the beta chain.

The sTCRs according to the present invention may further comprise additional modifications aimed at stabilizing the interactions between the variable regions of the alpha and beta chains (in the case of the sTCRs as defined in (i) to (iv)) or modifications aimed at stabilizing the interaction between the polypeptide comprising elements of the alpha chain and the polypeptide comprising elements of the beta chain. Suitable modifications include all those shown in figure 2 in Robinson et al. (FEBS J., 2021, 288(21):6159-6173. doi: 10.1111/febs.15780) and which include, without limitation:
- sTCRs in which the transmembrane domain of both chains has been replaced by a leucine zipper, thereby resulting in the generation of alpha-beta heterodimers
- sTCRs in which the interaction between the alpha and beta chains is stabilized by the introduction of Boulter disulphide bonds or additional Cα:Cβ disulphide bonds.
- sTCRs in which containing stabilizing mutations on surface of constant domains.
- sTCRs containing additional V-C disulphides.
- sTCRs containing mutations that stabilize Vα:Cα or Vβ:Cβ interactions or Vα:Vβ interactions

A first type of sTCRs is formed by single chain TCRs which consist of a single polypeptide chain which comprises the alpha chain variable region and the beta chain variable region of the TCR.

It will be understood that in this type of sTCR, the regions of the single polypeptide comprising the alpha chain variable region and the beta chain variable region of the TCR may be directly connected or connected by spacer or linker regions.

Amino acid linker regions are sequences within a polypeptide chain that connect distinct protein domains, providing flexibility and facilitating proper folding and function. These linkers often lack a defined secondary structure, adopting random coil configurations. Suitable linkers can be readily selected and can be of any of a suitable of different lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids. Commonly used amino acids in linkers include glycine, serine, and proline, which contribute to the flexibility and conformational freedom of the linker. Exemplary flexible linkers include the linker having the sequence TGSTSGSGKPGSGEGS. Suitable linkers include as glycine polymers (G)ₙ where (n) can be adjusted to create longer linkers, glycine-serine polymers (including, for example, (GS)ₙ, (GSGGS)ₙ and (GGGS)ₙ, where n is an integer of at least one), glycine-alanine polymers, alanine-serine polymers, and other flexible linkers known in the art. In a particular embodiment the linker comprises a glycine polymer of formula (G₄S)₃. Glycine and glycine-serine polymers are of interest since both of these amino acids are relatively unstructured, and therefore may serve as a neutral tether between components. Glycine polymers are of particular interest since glycine accesses significantly more phi-psi space than even alanine, and is much less restricted than residues with longer side chains. Exemplary flexible linkers include, but are not limited to GGSG, GGSGG, GSGSG, GSGGG, GGGSG, GSSSG, and the like. The ordinarily skilled artisan will recognize that design of a peptide conjugated to any elements described above can include linkers that are all or partially flexible, such that the linker can include a flexible linker as well as one or more portions that confer less flexible structure.

For the scTCR to bind an MHC-peptide complex, the α and β segments must be paired so that the variable region sequences thereof are orientated for such binding. Hence the linker should have sufficient length to span the distance between the C terminus of the α segment and the N terminus of the β segment, or vice versa. On the other hand excessive linker length should preferably be avoided, in case the end of the linker at the N-terminal variable region sequence blocks or reduces bonding of the scTCR to the target peptide-MHC complex.

In the first type of sTCRs according to the present invention, the alpha chain variable region may be located N-terminally with respect to the beta chain variable region of the TCR. In another embodiment, the beta chain variable region may be located N-terminally with respect to the alpha chain variable region of the TCR.

A second type of sTCRs is formed by single chain TCRs which consist of a single polypeptide chain which comprises the TCR alpha chain extracellular region and a second region comprising TCR beta chain extracellular region. These regions may be directly connected or connected by spacer or linker regions..

The extracellular region sequences present in the sTCRs according to the present invention preferably correspond to those of a human TCR, as do the variable region sequences present in the α and β segments. However, the correspondence between such sequences need not be 1:1 on an amino acid level. N- or C-truncation, and/or amino acid deletion and/or substitution relative to the corresponding human TCR sequences is acceptable, provided the overall result is mutual orientation of variable region sequences of the α and β segments as in native αβ T cell receptors and retention of peptide-MHC binding functionality.

In the second type of sTCRs according to the present invention, the alpha chain extracellular region may be located N-terminal with respect to the beta chain extracellular region of the TCR. In another embodiment, the beta chain extracellular region may be located N-terminal with respect to the alpha chain extracellular region of the TCR

A third type of sTCRs is formed by heterodimers which comprise a first polypeptide comprising the TCR alpha chain variable region and a second polypeptide comprising the TCR beta chain variable region wherein the first and second polypeptides, wherein the first and second polypeptide are connected by a disulfide bond. The interaction of the first and second polypeptide may be stabilized by the presence of additional regions in the first and second polypeptides containing dimerization motifs. The dimerization motifs in the first and second polypeptides may, be virtue of their interaction, strengthen the interaction between both polypeptides.

Dimerization motifs or regions are specific sequences within polypeptide chains that facilitate and stabilize the interaction between two protein molecules, forming a dimer. These regions often rely on non-covalent interactions such as hydrogen bonds, ionic interactions, and van der Waals forces, which allow for reversible and dynamic regulation of protein activity. Suitable dimerization regions that can be used in the sTCRs include, without limitation, leucine zippers, such as those found in transcription factors like c-Fos and c-Jun1, Helix-Loop-Helix (HLH) Domains such as those found in MyoD and E47, coiled-coil motifs, such as those found in kinesin motor proteins and PDZ Domains, such as those found in the PSD-95 protein

A fourth type of sTCR are heterodimers formed by a first polypeptide comprising the TCR alpha chain extracellular region and a second polypeptide comprising the TCR beta chain extracellular constant, wherein the first and second are connected by a disulfide bond.

Similarly as above, the heterodimer formed by the first polypeptide comprising the TCR alpha chain extracellular region and the second polypeptide comprising the TCR beta chain extracellular region may be stabilized by dimerization motifs. Suitable dimerization motifs that can be used for this type of sTCRs are those defined above for the previous type of sTCR.

In an embodiment, the antigen recognizing construct according to the first aspect of the invention comprises:
(a) a TCR alpha chain variable domain comprising a complementarity determining region 3 (CDR3) comprising SEQ ID NO: 5 or functionally equivalent variant thereof and a TCR beta chain variable domain comprising a complementarity determining region 3 (CDR3) comprising SEQ ID NO: 8 or a functionally equivalent variant thereof; or
(b) a TCR alpha chain variable domain comprising a CDR3 comprising SEQ ID NO: 17 or a functionally equivalent variant thereof and a TCR beta chain variable domain comprising a CDR3 comprising SEQ ID NO: 20 or a functionally equivalent variant thereof.

The expression "functionally equivalent variant", when referred to a CDR refers to a peptide sequence which, when present in the TCR variable domain alpha or beta in combination with the other CDRs of the TCR alpha and beta variable domains, results in a TCR which substantially preserves the ability of the TCR to specifically bind to the antigen of the TCR containing the non-variant TCR. It will be understood that different combinations of non-modified and variant CDRs can be found in the TCRs of the invention. In particular:
- The TCR contains a variant of one or more of the alpha chain variable domain CDR1, CDR2 and CDR3 but the beta chain variable domain CDR1, CDR2 and CDR3 are not modified.
- The TCR contains a variant of one or more of the beta chain variable domain CDR1, CDR2 and/or CDR3 but the alpha chain variable domain CDR1, CDR2 and CDR3 are not modified.
- The TCR contains a variant of one or more of the alpha chain variable domain CDR1, CDR2 and CDR3 and a variant of one or more of the beta chain variable domain CDR1, CDR2 and/or CDR3 but the alpha chain variable domain CDR1, CDR2 and CDR3 are not modified.

Suitable functionally equivalent variants of the CDRs of the antigen recognizing constructs according to the present invention include those which, if n if the total number of amino acids of the CDR, contain amino acid substitution at n-1, n-2, n-3, n-4, n-5 and so forth positions of the reference CDR. For instance, if the reference CDR consists of 10 residues, then the functionally equivalent variant of the CDR contains 9, 8, 7 6, 5, 4, 3 2 or 1 substitutions. The substitutions can be of conservative nature.

"Conservative amino acid substitutions" result from replacing one amino acid with another having similar structural and/or chemical properties. For example, the following six groups each contain amino acids that are conservative substitutions for one another:
- Alanine (A), Serine (S), Threonine (T);
- Aspartic acid (D), Glutamic acid (E);
- Asparagine (N), Glutamine (Q);
- Arginine (R), Lysine (K);
- Isoleucine (I), Leucine (L), Methionine (M), Valine (V); and
- Phenylalanine (F), Tyrosine (Y), Tryptophan (W).

The terms "specific binding," "selective binding," "selectively binds," and "specifically binds," refer to a TCR binding to an antigen complexed to an HLA molecule with high affinity.

As used herein, the term "high affinity" for a TCR refers to a TCR having a K_{D} of 10⁻⁷ M or less, preferably 10⁻⁸ M or less, more preferably 10⁻⁹ M or less and even more preferably 10⁻¹⁰ M or less for a target antigen when complexed with the HLA molecule. Typically, the TCR (i) binds with an equilibrium dissociation constant (K_{D}) of approximately less than 10⁻⁷ M, such as approximately less than 10⁻⁸ M, 10⁻⁹ M or 10⁻¹⁰ M or even lower when determined by, e.g., surface plasmon resonance (SPR) technology in a BIACORE instrument using the predetermined antigen, e.g., hBCMA, as the analyte and the sdAb as the ligand, or Scatchard analysis of binding of the antibody to antigen positive cells, and (ii) binds to the predetermined antigen with an affinity that is at least two-fold greater than its affinity for binding to a non-specific antigen (e.g., BSA, casein) other than the predetermined antigen or a closely-related antigen. Accordingly, a TCR that "specifically binds to its target antigen" refers to a TCR that binds to the antigen in a complex with an HLA molecule with a K_{D} of 10⁻⁷ M or less, such as approximately less than 10⁻⁻⁸ M, 10⁻⁹ M or 10⁻¹⁰ M or even lower.

The functionality of the TCR carrying the functionally equivalent variant of the CDRs according to the invention can also be determined by measuring their "half maximum effective concentration" (EC₅₀), which is refers to the half-maximal effect concentration (concentration for 50% of maximal effect, EC₅₀), which refers to the concentration that elicits 50% of the maximal effect. In a preferred embodiment the TCR according to the invention have an EC₅₀ of at least 30 nM, at least 29 nM, at least 28 nM, at least 27 nM, at least 26 nM, at least 25 nM, at least 24 nM, at least 23 nM, at least 22 nM, at least 21 nM, at least 20 nM, at least 19 nM, at least 18 nM, at least 17 nM, at least 16 nM, at least 15 nM, at least 14 nM, at least 13 nM, at least 12 nM, at least 11 nM, at least 10 nM. It will be understood that the functionally equivalent variants of the sdAbs will maintain at least 50%, at least 60%, at least 70%, at least 80%, at least 90%, at least 100% of the EC50 of the sdAb to which the variant refers to.

In a particular embodiment, the antigen recognizing construct according to the first aspect of the invention, wherein:
(a) the TCR alpha chain variable domains CDR1, CDR2 and CDR3 comprise, respectively, SEQ ID NO: 3, 4 and 5 or functionally equivalent variants thereof and the TCR beta chain variable domains CDR1, CDR2 and CDR3 comprise respectively, SEQ ID NO: 6, 7 and 8 or functionally equivalent variants thereof; or
(b) the TCR alpha chain variable domains CDR1, CDR2 and CDR3 comprise, respectively, SEQ ID NO: 15, 16 and 17 or functionally equivalent variants thereof and the TCR beta chain variable domains CDR1, CDR2 and CDR3 comprise respectively, SEQ ID NO: 18, 19 and 20 or functionally equivalent variants thereof.

In another particular embodiment, the antigen recognizing construct according to the first aspect of the invention, wherein the TCR comprises:
(a) an alpha chain variable region having the amino acid sequence of SEQ ID NO: 9 and a beta chain variable region having the amino acid sequence of SEQ ID NO: 10; or
(b) an alpha chain variable region having the amino acid sequence of SEQ ID NO: 21 and a beta chain variable region having the amino acid sequence of SEQ ID NO: 22.

In another particular embodiment, the antigen recognizing construct according to the first aspect of the invention, wherein the TCR comprises:
(a) an alpha chain extracellular region having the amino acid sequence of SEQ ID NO: 11 and a beta chain extracellular region having the amino acid sequence of SEQ ID NO: 12; or
(b) an alpha chain extracellular region having the amino acid sequence of SEQ ID NO: 23 and a beta chain extracellular region having the amino acid sequence of SEQ ID NO: 24.

In a more particular embodiment, the antigen recognizing construct according to the first aspect of the invention, wherein the TCR comprises:
(a) an alpha chain having the amino acid sequence of SEQ ID NO: 13 and a beta chain having the amino acid sequence of SEQ ID NO: 14; or
(b) an alpha chain having the amino acid sequence of SEQ ID NO: 25 and a beta chain having the amino acid sequence of SEQ ID NO: 26.

In a second aspect, the present invention relates to an antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein the antigen recognizing construct comprises:
(a) a TCR alpha chain variable domain comprising a CDR3 comprising SEQ ID NO: 5 or functionally equivalent variant thereof and a TCR beta chain variable domain comprising a CDR3 comprising SEQ ID NO: 8 or a functionally equivalent variant thereof; or
(b) a TCR alpha chain variable domain comprising a CDR3 comprising SEQ ID NO: 17 or functionally equivalent variant thereof and a TCR beta chain variable domain comprising a CDR3 comprising SEQ ID NO: 20 or a functionally equivalent variant thereof; or
(c) a TCR alpha chain variable domain comprising a CDR3 comprising SEQ ID NO: 29 or functionally equivalent variant thereof and a TCR beta chain variable domain comprising a CDR3 comprising SEQ ID NO: 32 or a functionally equivalent variant thereof; or
(d) a TCR alpha chain variable domain comprising a CDR3 comprising SEQ ID NO: 41 or a functionally equivalent variant thereof and a TCR beta chain variable domain comprising a CDR3 comprising SEQ ID NO: 44 or a functionally equivalent variant thereof; or
(e) a TCR alpha chain variable domain comprising a CDR3 comprising SEQ ID NO: 53 or a functionally equivalent variant thereof and a TCR beta chain variable domain comprising a CDR3 comprising SEQ ID NO: 56 or a functionally equivalent variant thereof; or
(f) a TCR alpha chain variable domain comprising a CDR3 comprising SEQ ID NO: 65 or a functionally equivalent variant thereof and a TCR beta chain variable domain comprising a CDR3 comprising SEQ ID NO: 68 or a functionally equivalent variant thereof; or
(g) a TCR alpha chain variable domain comprising a CDR3 comprising SEQ ID NO: 77 or a functionally equivalent variant thereof and a TCR beta chain variable domain comprising a CDR3 comprising SEQ ID NO: 80 or a functionally equivalent variant thereof; or
(h) a TCR alpha chain variable domain comprising a CDR3 comprising SEQ ID NO: 89 or a functionally equivalent variant thereof and a TCR beta chain variable domain comprising a CDR3 comprising SEQ ID NO: 92 or a functionally equivalent variant thereof; or
(i) a TCR alpha chain variable domain comprising a CDR3 comprising SEQ ID NO: 101 or a functionally equivalent variant thereof and a TCR beta chain variable domain comprising a CDR3 comprising SEQ ID NO: 104 or a functionally equivalent variant thereof; or
(j) a TCR alpha chain variable domain comprising a CDR3 comprising SEQ ID NO: 113 or a functionally equivalent variant thereof and a TCR beta chain variable domain comprising a CDR3 comprising SEQ ID NO: 116 or a functionally equivalent variant thereof.
(k) a TCR alpha chain variable domain comprising a CDR3 comprising SEQ ID NO: 125 or a functionally equivalent variant thereof and a TCR beta chain variable domain comprising a CDR3 comprising SEQ ID NO: 128 or a functionally equivalent variant thereof.

In an embodiment, the antigen recognizing construct comprising a TCR alpha chain variable domain comprising a CDR3 comprising SEQ ID NO: 5 or functionally equivalent variant thereof and a TCR beta chain variable domain comprising a CDR3 comprising SEQ ID NO: 8 or a functionally equivalent variant thereof corresponds to the TCR having specificity for a peptide derived from the PIK3CA p.R88Q protein as defined in Table 2 below, wherein said peptide preferably comprises or consists of the sequence FFDETRQL, when complexed to HLA C*04:01.

In an embodiment, the antigen recognizing construct comprising a TCR alpha chain variable domain comprising a CDR3 comprising SEQ ID NO: 17 or functionally equivalent variant thereof and a TCR beta chain variable domain comprising a CDR3 comprising SEQ ID NO: 20 or a functionally equivalent variant thereof corresponds to the TCR having specificity for a peptide derived from the TP53 p.R248Q protein as defined in Table 2 below, wherein said peptide preferably comprises or consists of the sequence SCMGGMNQR, when complexed to HLA A*11:01.

In an embodiment, the antigen recognizing construct comprising a TCR alpha chain variable domain comprising a CDR3 comprising SEQ ID NO: 29 or functionally equivalent variant thereof and a TCR beta chain variable domain comprising a CDR3 comprising SEQ ID NO: 32 or a functionally equivalent variant thereof corresponds to the TCR having specificity for an antigen derived from ncRNA C5orf22 as defined in Table 2 below, wherein said peptide preferably comprises or consists of the sequence STDLPILLK and wherein the peptide is complexed to HLA A11*01.

In an embodiment, the antigen recognizing construct comprising a TCR alpha chain variable domain comprising a CDR3 comprising SEQ ID NO: 41 or functionally equivalent variant thereof and a TCR beta chain variable domain comprising a CDR3 comprising SEQ ID NO: 44 or a functionally equivalent variant thereof corresponds to the TCR having specificity for a peptide derived from the polypeptide encoded by the 5'UTR of the HOXC13 gene as defined in Table 2 below, wherein the peptide preferably comprises or consists of the sequence SSLPLASGVFKK and wherein the peptide is complexed to HLA A11*01.

In an embodiment, the antigen recognizing construct comprising a TCR alpha chain variable domain comprising a CDR3 comprising SEQ ID NO: 53 or functionally equivalent variant thereof and a TCR beta chain variable domain comprising a CDR3 comprising SEQ ID NO: 56 or a functionally equivalent variant thereof corresponds to the TCR having specificity for a peptide derived from the polypeptide encoded by the 5'UTR region of the ZKSCAN1 gene as defined in Table 2 below, wherein the peptide preferably comprises or consists of the of the sequence RVAEITGIVK and wherein the peptide is complexed to HLA A68*01.

In an embodiment, the antigen recognizing construct comprising a TCR alpha chain variable domain comprising a CDR3 comprising SEQ ID NO: 65 or functionally equivalent variant thereof and a TCR beta chain variable domain comprising a CDR3 comprising SEQ ID NO: 68 or a functionally equivalent variant thereof corresponds to the TCR having specificity for a peptide derived from the region defined by positions 167 to 176 of the MAGE A3 protein or from the region defined by positions 167 to 176 of the E168K MAGEA6 protein as defined in Table 2 below, wherein the peptide preferably comprising or consisting of the sequence MEVDPIGHLY.

In an embodiment, the antigen recognizing construct comprising a TCR alpha chain variable domain comprising a CDR3 comprising SEQ ID NO: 89 or functionally equivalent variant thereof and a TCR beta chain variable domain comprising a CDR3 comprising SEQ ID NO: 92 or a functionally equivalent variant thereof corresponds to the TCR having specificity for an peptide derived from the region defined by positions 142 to 156 in the MAGE A4 protein as defined in Table 2 below, wherein the peptide preferably comprises or consists of the sequence KRCFPVIFGK and wherein the peptide is complexed to HLA B*27:05.

In an embodiment, the antigen recognizing construct comprising a TCR alpha chain variable domain comprising a CDR3 comprising SEQ ID NO: 101 or functionally equivalent variant thereof and a TCR beta chain variable domain comprising a CDR3 comprising SEQ ID NO: 104 or a functionally equivalent variant thereof corresponds to the TCR having specificity for a peptide corresponding to the region defined by positions 142 to 156 of the MAGE A4_protein as defined in Table 2 below, wherein the peptide preferably comprises or consists of the sequence KRCFPVIFGK, wherein said peptide is complexed to HLA B*27:05.

In an embodiment, the antigen recognizing construct comprising a TCR alpha chain variable domain comprising a CDR3 comprising SEQ ID NO: 125 or functionally equivalent variant thereof and a TCR beta chain variable domain comprising a CDR3 comprising SEQ ID NO: 128 or a functionally equivalent variant thereof corresponds to the TCR having specificity for an antigen derived from the MAGE A4 protein as defined in Table 2 below and wherein the peptide is complexed to HLA B*51:01.

In an embodiment, the antigen recognizing construct comprising a TCR alpha chain variable domain comprising a CDR3 comprising SEQ ID NO: 113 or functionally equivalent variant thereof and a TCR beta chain variable domain comprising a CDR3 comprising SEQ ID NO: 116 or a functionally equivalent variant thereof corresponds to the TCR having specificity for an antigen derived from the PIK3CA p.E545K protein as defined in Table 2 below, wherein the peptide preferably comprises or consists of the sequence STRDPLSEITK and wherein the peptide is complexed to HLA A*11:01.

In an embodiment, the antigen recognizing construct according to the second aspect of the invention, wherein:
(a) The TCR alpha chain variable domains CDR1, CDR2 and CDR3 comprise, respectively, SEQ ID NO: 3, 4 and 5 or functionally equivalent variants thereof and the TCR beta chain variable domains CDR1, CDR2 and CDR3 comprise respectively, SEQ ID NO: 6, 7 and 8 or functionally equivalent variants thereof; or
(b) The TCR alpha chain variable domains CDR1, CDR2 and CDR3 comprise, respectively, SEQ ID NO: 15, 16 and 17 or functionally equivalent variants thereof and the TCR beta chain variable domains CDR1, CDR2 and CDR3 comprise respectively, SEQ ID NO: 18, 19 and 20 or functionally equivalent variants thereof; or
(c) The TCR alpha chain variable domains CDR1, CDR2 and CDR3 comprise, respectively, SEQ ID NO: 27, 28 and 29 or functionally equivalent variants thereof and the TCR beta chain variable domains CDR1, CDR2 and CDR3 comprise respectively, SEQ ID NO: 30, 31 and 32 or functionally equivalent variants thereof; or
(d) the TCR alpha chain variable domains CDR1, CDR2 and CDR3alpha comprise, respectively, SEQ ID NO: 39, 40 and 41 or functionally equivalent variants thereof and the TCR beta chain variable domains CDR1, CDR2 and CDR3 comprise respectively, SEQ ID NO: 42, 43 and 44 or functionally equivalent variants thereof; or
(e) the TCR alpha chain variable domains CDR1, CDR2 and CDR3 comprise, respectively, SEQ ID NO: 51, 52 and 53 or functionally equivalent variants thereof and the TCR beta chain variable domains CDR1, CDR2 and CDR3 comprise respectively, SEQ ID NO: 54, 55 and 56 or functionally equivalent variants thereof; or
(f) the TCR alpha chain variable domains CDR1, CDR2 and CDR3 comprise, respectively, SEQ ID NO: 63, 64 and 65 or functionally equivalent variants thereof and the TCR beta chain variable domains CDR1, CDR2 and CDR3 comprise respectively, SEQ ID NO: 66, 67 and 68 or functionally equivalent variants thereof; or
(g) the TCR alpha chain variable domains CDR1, CDR2 and CDR3 comprise, respectively, SEQ ID NO: 75, 76 and 77 or functionally equivalent variants thereof and the TCR beta chain variable domains CDR1, CDR2 and CDR3 comprise respectively, SEQ ID NO: 78, 79 and 80 or functionally equivalent variants thereof; or
(h) the TCR alpha chain variable domains CDR1, CDR2 and CDR3 comprise, respectively, SEQ ID NO: 87, 88 and 89 or functionally equivalent variants thereof and the TCR beta chain variable domains CDR1, CDR2 and CDR3 comprise respectively, SEQ ID NO: 90, 91 and 92 or functionally equivalent variants thereof; or
(i) the TCR alpha chain variable domains CDR1, CDR2 and CDR3 comprise, respectively, SEQ ID NO: 99, 100 and 101 or functionally equivalent variants thereof and the TCR beta chain variable domains CDR1, CDR2 and CDR3 comprise respectively, SEQ ID NO: 102, 103 and 104 or functionally equivalent variants thereof; or
(j) the TCR alpha chain variable domains CDR1, CDR2 and CDR3 comprise, respectively, SEQ ID NO: 111, 112 and 113 or functionally equivalent variants thereof and the TCR beta chain variable domains CDR1, CDR2 and CDR3 comprise respectively, SEQ ID NO: 114, 115 and 116 or functionally equivalent variants thereof.
(k) the TCR alpha chain variable domains CDR1, CDR2 and CDR3 comprise, respectively, SEQ ID NO: 123, 124 and 125 or functionally equivalent variants thereof and the TCR beta chain variable domains CDR1, CDR2 and CDR3 comprise respectively, SEQ ID NO: 126, 127 and 128 or functionally equivalent variants thereof.

The expression "functionally equivalent variant", when referred to an alpha chain variable domain, to a beta chain variable domain, to an alpha chain extracellular region, to a beta chain extracellular region, to an alpha chain or to a beta chain refers to a polypeptide resulting from the modification, deletion or insertion or one or more amino acids and which substantially preserves the specific binding activity of its reference polypeptide. If the variant results from the modification of one or more residues, then the modification can be a conservative amino acid substitution or not.

The term "specific binding," as well as values for the parameters used to measure binding (KD and EC50) have been defined above in the context of the functionally equivalent variant of the CDRs.

In a preferred embodiment of the invention, the functionally equivalent variant of the alpha chain variable domain as defined in any of SEQ ID NO: 9, 21, 33, 45, 57, 69, 81, 93, 105,117 or 129 is a polypeptide that shows at least 60%, at least 70%, at least 80%, at least 85%, at least 90%, at least 91%, at least 92%, at least 93%, at least 94%, at least 95%, at least 96%, at least 97%, at least 98%, at least 99%, at least 100% identity with the reference sequence.

In another preferred embodiment of the invention, the functionally equivalent variant of the beta chain variable domain as defined in any of SEQ ID NO: 10, 22, 34, 46, 58, 70, 82, 94, 106, 118 or 130 is a polypeptide that shows at least 60%, at least 70%, at least 80%, at least 85%, at least 90%, at least 91%, at least 92%, at least 93%, at least 94%, at least 95%, at least 96%, at least 97%, at least 98%, at least 99%, at least 100% identity with the reference sequence.

In another preferred embodiment of the invention, the functionally equivalent variant of the alpha chain extracellular domain as defined in any of SEQ ID NO: 11, 23, 35, 47, 59, 71, 83, 96, 107, 119 or 131 is a polypeptide that shows at least 60%, at least 70%, at least 80%, at least 85%, at least 90%, at least 91%, at least 92%, at least 93%, at least 94%, at least 95%, at least 96%, at least 97%, at least 98%, at least 99%, at least 100% identity with the reference sequence.

In another preferred embodiment of the invention, the functionally equivalent variant of the beta chain extracellular domain as defined in any of SEQ ID NO: 12, 24, 36, 48, 60, 72, 84, 97, 108, 120 or 132 is a polypeptide that shows at least 60%, at least 70%, at least 80%, at least 85%, at least 90%, at least 91%, at least 92%, at least 93%, at least 94%, at least 95%, at least 96%, at least 97%, at least 98%, at least 99%, at least 100% identity with the reference sequence.

In another preferred embodiment of the invention, the functionally equivalent variant of the alpha chain as defined in any of SEQ ID NO: 13, 25, 37, 49, 61, 73, 85, 98, 109, 121 or 133 is a polypeptide that shows at least 60%, at least 70%, at least 80%, at least 85%, at least 90%, at least 91%, at least 92%, at least 93%, at least 94%, at least 95%, at least 96%, at least 97%, at least 98%, at least 99%, at least 100% identity with the reference sequence.

In another preferred embodiment of the invention, the functionally equivalent variant of the beta chain as defined in any of SEQ ID NO: 14, 26, 38, 50, 62, 75, 86, 100, 110, 122 or 134 is a polypeptide that shows at least 60%, at least 70%, at least 80%, at least 85%, at least 90%, at least 91%, at least 92%, at least 93%, at least 94%, at least 95%, at least 96%, at least 97%, at least 98%, at least 99%, at least 100% identity with the reference sequence.

The degree of identity or similarity between two polypeptides or two polynucleotides is determined by using computer-implemented algorithms and methods that are widely known in the art. The identity and similarity between two sequences of amino acids is preferably determined using the BLASTP algorithm or determined using the Needleman-Wunsch algorithm to obtain an optimal alignment between two sequences. This analysis can be performed using an online software such as EMBOSS Needle (https://www.ebi.ac.uk/jdispatcher/psa/emboss_needle). In a preferred embodiment of the invention the degree of identity between two polypeptides is determined across the whole length of both polypeptides.

It will be understood that the functionally equivalent variants of the TCR alpha chain variable regions as defined in any of SEQ ID NO: 9, 21, 33, 45, 57, 69, 81, 93, 105,117 or 129 or the functionally equivalent variants of the TCR beta chain variable regions as defined in any of SEQ ID NO: 10, 22, 34, 46, 58, 70, 82, 94, 106, 118 or 130 may result from the modification, deletion or insertion of one or more amino acids at one or more of the FR regions, from the modification, deletion or insertion of one or more amino acids at one or more of the CDR or from the modification, deletion or insertion of one or more amino acids at one or more of the FRs and of the CDRs.

It will be understood that the functionally equivalent variants of the TCR alpha chain extracellular regions as defined in any of SEQ ID NO: 11, 23, 35, 47, 59, 71, 83, 95, 107, 119 or 131 or the functionally equivalent variants of the TCR beta chain extracellular regions as defined in any of SEQ ID NO: 12, 24, 36, 48, 60, 72, 84, 96, 108, 120 or 132 may result from the modification, deletion or insertion of one or more amino acids at one or more of the FR regions, from the modification, deletion or insertion of one or more amino acids at one or more of the CDR or from the modification, deletion or insertion of one or more amino acids at one or more of the FRs and of the CDRs.

It will be understood that the functionally equivalent variants of the TCR alpha chain as defined in any of SEQ ID NO: 13, 25, 37, 49, 61, 73, 85, 97, 109, 121 or 133 or the functionally equivalent variants of the TCR beta chain variable regions as defined in any of SEQ ID NO: 14, 26, 38, 50, 62, 74, 86, 98, 110,122 or 134 may result from the modification, deletion or insertion of one or more amino acids at one or more of the FR regions, from the modification, deletion or insertion of one or more amino acids at one or more of the CDR or from the modification, deletion or insertion of one or more amino acids at one or more of the FRs and of the CDRs.

In a particular embodiment, the antigen recognizing construct according to the second aspect of the invention, wherein the TCR comprises:
(i) an alpha chain variable region having the amino acid sequence of SEQ ID NO: 9 and a beta chain variable region having the amino acid sequence of SEQ ID NO: 10; or
(ii) an alpha chain variable region having the amino acid sequence of SEQ ID NO: 21 and a beta chain variable region having the amino acid sequence of SEQ ID NO: 22; or
(iii) an alpha chain variable region having the amino acid sequence of SEQ ID NO: 33 and a beta chain variable region having the amino acid sequence of SEQ ID NO: 34; or
(iv) an alpha chain variable region having the amino acid sequence of SEQ ID NO: 45 and a beta chain variable region having the amino acid sequence of SEQ ID NO: 46; or
(v) an alpha chain variable region having the amino acid sequence of SEQ ID NO: 57 and a beta chain variable region having the amino acid sequence of SEQ ID NO: 58; or
(vi) an alpha chain variable region having the amino acid sequence of SEQ ID NO: 69 and a beta chain variable region having the amino acid sequence of SEQ ID NO: 70; or
(vii) an alpha chain variable region having the amino acid sequence of SEQ ID NO: 81 and a beta chain variable region having the amino acid sequence of SEQ ID NO: 82; or
(viii) an alpha chain variable region having the amino acid sequence of SEQ ID NO: 93 and a beta chain variable region having the amino acid sequence of SEQ ID NO: 94; or
(ix) an alpha chain variable region having the amino acid sequence of SEQ ID NO: 105 and a beta chain variable region having the amino acid sequence of SEQ ID NO: 106; or
(x) an alpha chain variable region having the amino acid sequence of SEQ ID NO: 117 and a beta chain variable region having the amino acid sequence of SEQ ID NO: 118; or
(xi) an alpha chain variable region having the amino acid sequence of SEQ ID NO: 129 and a beta chain variable region having the amino acid sequence of SEQ ID NO: 130.

In another particular embodiment, the antigen recognizing construct according to the second aspect of the invention, wherein the TCR comprises:
(i) an alpha chain extracellular region having the amino acid sequence of SEQ ID NO: 11 and a beta chain extracellular region having the amino acid sequence of SEQ ID NO: 12; or
(ii) an alpha chain extracellular region having the amino acid sequence of SEQ ID NO: 23 and a beta chain extracellular region having the amino acid sequence of SEQ ID NO: 24; or
(iii) an alpha chain extracellular region having the amino acid sequence of SEQ ID NO: 35 and a beta chain extracellular region having the amino acid sequence of SEQ ID NO: 36; or
(iv) an alpha chain extracellular region having the amino acid sequence of SEQ ID NO: 47 and a beta chain extracellular region having the amino acid sequence of SEQ ID NO: 48; or
(v) an alpha chain extracellular region having the amino acid sequence of SEQ ID NO: 59 and a beta chain extracellular region having the amino acid sequence of SEQ ID NO: 60; or
(vi) an alpha chain extracellular region having the amino acid sequence of SEQ ID NO: 71 and a beta chain extracellular region having the amino acid sequence of SEQ ID NO: 72; or
(vii) an alpha chain extracellular region having the amino acid sequence of SEQ ID NO: 83 and a beta chain extracellular region having the amino acid sequence of SEQ ID NO: 84; or
(viii) an alpha chain extracellular region having the amino acid sequence of SEQ ID NO: 95 and a beta chain extracellular region having the amino acid sequence of SEQ ID NO: 96 or
(ix) an alpha chain extracellular region having the amino acid sequence of SEQ ID NO: 107 and a beta chain extracellular region having the amino acid sequence of SEQ ID NO: 108; or
(x) an alpha chain extracellular region having the amino acid sequence of SEQ ID NO: 119 and a beta chain extracellular region having the amino acid sequence of SEQ ID NO: 120; or
(xi) an alpha chain extracellular region having the amino acid sequence of SEQ ID NO: 131 and a beta chain extracellular region having the amino acid sequence of SEQ ID NO: 132.

In a more particular embodiment, the antigen recognizing construct according to the second aspect of the invention, wherein the TCR comprises:
(a) an alpha chain having the amino acid sequence of SEQ ID NO: 13 and a beta chain having the amino acid sequence of SEQ ID NO: 14; or
(b) an alpha chain having the amino acid sequence of SEQ ID NO: 25 and a beta chain having the amino acid sequence of SEQ ID NO: 26; or
(c) an alpha chain having the amino acid sequence of SEQ ID NO: 37 and a beta chain having the amino acid sequence of SEQ ID NO: 38; or
(d) an alpha chain having the amino acid sequence of SEQ ID NO: 49 and a beta chain having the amino acid sequence of SEQ ID NO: 50; or
(e) an alpha chain having the amino acid sequence of SEQ ID NO: 61 and a beta chain having the amino acid sequence of SEQ ID NO: 62; or
(f) an alpha chain having the amino acid sequence of SEQ ID NO: 73 and a beta chain having the amino acid sequence of SEQ ID NO: 74; or
(g) an alpha chain having the amino acid sequence of SEQ ID NO: 85 and a beta chain having the amino acid sequence of SEQ ID NO: 86; or
(h) an alpha chain having the amino acid sequence of SEQ ID NO: 97 and a beta chain having the amino acid sequence of SEQ ID NO: 98; or
(i) an alpha chain having the amino acid sequence of SEQ ID NO: 109 and a beta chain having the amino acid sequence of SEQ ID NO: 110; or
(j) an alpha chain having the amino acid sequence of SEQ ID NO: 121 and a beta chain having the amino acid sequence of SEQ ID NO: 122; or
(k) an alpha chain having the amino acid sequence of SEQ ID NO: 133 and a beta chain having the amino acid sequence of SEQ ID NO: 134.

The following table summarizes the sequences of the different TCRs from which the antigen recognizing constructs of the present invention derive.

**Table 1: CDR and VH and VL sequences of the antigen-recognizing constructs of the invention**

| **TCR number** | **Reactivity** | **SEQ ID NO:** | **Element** | **Sequence** |
|---|---|---|---|---|
| | | 3 | CDR1 alpha | TSDQSYG |
| | | 4 | CDR2 alpha | QGSYDEQN |
| | | 5 | CDR3 alpha | CAIVRTSYDKVIF |
| | | 6 | CDR1 beta | SGHNS |
| | | 7 | CDR2 beta | FNNNVP |
| | | 8 | CDR3 beta | CASSSPDLNTEAFF |
| | | 9 | alpha chain variable region | SLSSLLKVVTASLWLGPGIAQKITQTQPGMFVQEKEAVTLDCTYDTSDQSYGLFWYKQPSSGEMIFLIYQGSYDEQNAT EGRYSLNFQKARKSANLVISASQLGDSAMYFCAIVRTSYDKVIFGPGTSLSVIP |
| | | 10 | beta chain variable region | DSWTFCCVSLCILVAKHTDAGVIQSPRHEVTEMGQEVTLRCKPISGHNSLFWYRQTMMRGLELLIYFNNNVPIDDSGM PEDRFSAKMPNASFSTLKIQPSEPRDSAVYFCASSSPDLNTEAFFGQGTRLTVV |
| **1** | **TCR PIK3CA p.R88Q** | 11 | alpha chain extracelular region | SLSSLLKVVTASLWLGPGIAQKITQTQPGMFVQEKEAVTLDCTYDTSDQSYGLFWYKQPSSGEMIFLIYQGSYDEQNAT EGRYSLNFQKARKSANLVISASQLGDSAMYFCAIVRTSYDKVIFGPGTSLSVIPIQNPDPAVYQLRDSKSSDKSVCLFTDF DSQTNVSQSKDSDVYITDKTVLDMRSMDFKSNSAVAWSNKSDFACANAFNNSIIPEDTFFPSPESSCDVKLVEKSFETD TNLNFQNLS |
| | | 12 | beta chain extracelular region | DSWTFCCVSLCILVAKHTDAGVIQSPRHEVTEMGQEVTLRCKPISGHNSLFWYRQTMMRGLELLIYFNNNVPIDDSGM PEDRFSAKMPNASFSTLKIQPSEPRDSAVYFCASSSPDLNTEAFFGQGTRLTVVDLNKVFPPEVAVFEPSEAEISHTQKAT LVCLATGFFPDHVELSWWVNGKEVHSGVSTDPQPLKEQPALNDSRYCLSSRLRVSATFWQNPRNHFRCQVQFYGLSE NDEWTQDRAKPVTQIVSAEAWGRADCGFTSVSYQQGVLSATILYE |
| | | 13 | alpha chain | SLSSLLKVVTASLWLGPGIAQKITQTQPGMFVQEKEAVTLDCTYDTSDQSYGLFWYKQPSSGEMIFLIYQGSYDEQNAT EGRYSLNFQKARKSANLVISASQLGDSAMYFCAIVRTSYDKVIFGPGTSLSVIPIQNPDPAVYQLRDSKSSDKSVCLFTDF DSQTNVSQSKDSDVYITDKTVLDMRSMDFKSNSAVAWSNKSDFACANAFNNSIIPEDTFFPSPESSCDVKLVEKSFETD TNLNFQNLSVIGFRILLLKVAGFNLLMTLRLWSS |
| | | 14 | beta chain | DSWTFCCVSLCILVAKHTDAGVIQSPRHEVTEMGQEVTLRCKPISGHNSLFWYRQTMMRGLELLIYFNNNVPIDDSGM PEDRFSAKMPNASFSTLKIQPSEPRDSAVYFCASSSPDLNTEAFFGQGTRLTVVDLNKVFPPEVAVFEPSEAEISHTQKAT LVCLATGFFPDHVELSWWVNGKEVHSGVSTDPQPLKEQPALNDSRYCLSSRLRVSATFWQNPRNHFRCQVQFYGLSE NDEWTQDRAKPVTQIVSAEAWGRADCGFTSVSYQQGVLSATILYEILLGKATLYAVLVSALVLMAMVKRKDF |
| | | 1 | Target peptide | FFDETRQL |
| | | | HLA | C*04:01 |
| | | 15 | CDR1 alpha | SVFSS |
| | | 16 | CDR2 alpha | VVTGGEV |
| | | 17 | CDR3 alpha | CAGGEGYSTLTF |
| | | 18 | CDR1 beta | SGHVS |
| | | 19 | CDR2 beta | FQNEAQ |
| | | 20 | CDR3 beta | CASSLGYRGRIDYGYTF |
| | **TCR TP53 p.R248Q** | 21 | alpha chain variable region | VLKFSVSILWIQLAWVSTQLLEQSPQFLSIQEGENLTVYCNSSSVFSSLQWYQEPGEGPVLLVTVVTGGEVKKLKLTFQF GDAKDSSLHITAAQTGDTGLYLCAGGEGYSTLTFGKGTMLLVSP |
| | | 22 | beta chain variable region | GTRLLCWVVLGFLGTDHTGAGVSQSPRYKVAKRGQDVALRCDPISGHVSLFWYQQALGQGPEFLTYFQNEAQLDKSG LPSDRFFAERPEGSVSTLKIQRTQQEDSAVYLCASSLGYRGRIDYGYTFGSGTRLTVV |
| **2** | | 23 | alpha chain extracelular region | [?] VLKFSVSILWIQLAWVSTQLLEQSPQFLSIQEGENLTVYCNSSSVFSSLQWYQEPGEGPVLLVTVVTGGEVKKLKLTFQF GDAKDSSLHITAAQTGDTGLYLCAGGEGYSTLTFGKGTMLLVSPIQNPDPAVYQLRDSKSSDKSVCLFTDFDSQTNVSQ SKDSDVYITDKTVLDMRSMDFKSNSAVAWSNKSDFACANAFNNSIIPEDTFFPSPESSCDVKLVEKSFETDTNLNFQNLS |
| | | 24 | beta chain extracelular region | SKDSDVYITDKTVLDMRSMDFKSNSAVAWSNKSDFACANAFNNSIIPEDTFFPSPESSCDVKLVEKSFETDTNLNFQNLS GTRLLCWVVLGFLGTDHTGAGVSQSPRYKVAKRGQDVALRCDPISGHVSLFWYQQALGQGPEFLTYFQNEAQLDKSG LPSDRFFAERPEGSVSTLKIQRTQQEDSAVYLCASSLGYRGRIDYGYTFGSGTRLTVVDLNKVFPPEVAVFEPSEAEISHTQ KATLVCLATGFFPDHVELSWWVNGKEVHSGVSTDPQPLKEQPALNDSRYCLSSRLRVSATFWQNPRNHFRCQVQFYG LSENDEWTQDRAKPVTQIVSAEAWGRADCGFTSVSYQQGVLSATILYE |
| | | 25 | alpha chain | VLKFSVSILWIQLAWVSTQLLEQSPQFLSIQEGENLTVYCNSSSVFSSLQWYQEPGEGPVLLVTVVTGGEVKKLKLTFQF GDAKDSSLHITAAQTGDTGLYLCAGGEGYSTLTFGKGTMLLVSPIQNPDPAVYQLRDSKSSDKSVCLFTDFDSQTNVSQ SKDSDVYITDKTVLDMRSMDFKSNSAVAWSNKSDFACANAFNNSIIPEDTFFPSPESSCDVKLVEKSFETDTNLNFQNLS VIGFRILLLKVAGFNLLMTLRLWSS |
| | | 26 | beta chain | GTRLLCWVVLGFLGTDHTGAGVSQSPRYKVAKRGQDVALRCDPISGHVSLFWYQQALGQGPEFLTYFQNEAQLDKSG LPSDRFFAERPEGSVSTLKIQRTQQEDSAVYLCASSLGYRGRIDYGYTFGSGTRLTVVDLNKVFPPEVAVFEPSEAEISHTQ KATLVCLATGFFPDHVELSWWVNGKEVHSGVSTDPQPLKEQPALNDSRYCLSSRLRVSATFWQNPRNHFRCQVQFYG LSENDEWTQDRAKPVTQIVSAEAWGRADCGFTSVSYQQGVLSATILYEILLGKATLYAVLVSALVLMAMVKRKDF |
| | | 2 | Peptide | SCMGGMNQR |
| | | | HLA | A*11:01 |
| | | 27 | CDR1 alpha | DSASNY |
| | | 28 | CDR2 alpha | IRSNVGE |
| | | 29 | CDR3 alpha | CAASPNSGGYQKVTF |
| | | 30 | CDR1 beta | SGHAT |
| | | 31 | CDR2 beta | FQNNGV |
| | | 32 | CDR3 beta | CASSQHLAFTPNYGYTF |
| | **TCR ncRNA C5orf22** | 33 | alpha chain variable region | TSIRAVFIFLWLQLDLVNGENVEQHPSTLSVQEGDSAVIKCTYSDSASNYFPWYKQELGKRPQLIIDIRSNVGEKKDQRIAV TLNKTAKHFSLHITETQPEDSAVYFCAASPNSGGYQKVTFGIGTKLQVIP |
| | | 34 | beta chain variable region | GTRLLCWAALCLLGAELTEAGVAQSPRYKIIEKRQSVAFWCNPISGHATLYWYQQILGQGPKLLIQFQNNGVVDDSQLPK DRFSAERLKGVDSTLKIQPAKLEDSAVYLCASSQHLAFTPNYGYTFGSGTRLTVV |
| **3** | | 35 | alpha chain extracelular region | TSIRAVFIFLWLQLDLVNGENVEQHPSTLSVQEGDSAVIKCTYSDSASNYFPWYKQELGKRPQLIIDIRSNVGEKKDQRIAV TLNKTAKHFSLHITETQPEDSAVYFCAASPNSGGYQKVTFGIGTKLQVIPIQNPDPAVYQLRDSKSSDKSVCLFTDFDSQTN VSQSKDSDVYITDKTVLDMRSMDFKSNSAVAWSNKSDFACANAFNNSIIPEDTFFPSPESSCDVKLVEKSFETDTNLNFQ NLS |
| | | 36 | beta chain extracelular region | GTRLLCWAALCLLGAELTEAGVAQSPRYKIIEKRQSVAFWCNPISGHATLYWYQQILGQGPKLLIQFQNNGVVDDSQLPK DRFSAERLKGVDSTLKIQPAKLEDSAVYLCASSQHLAFTPNYGYTFGSGTRLTVVDLNKVFPPEVAVFEPSEAEISHTQKATL VCLATGFFPDHVELSWWVNGKEVHSGVSTDPQPLKEQPALNDSRYCLSSRLRVSATFWQNPRNHFRCQVQFYGLSEND EWTQDRAKPVTQIVSAEAWGRADCGFTSVSYQQGVLSATILYE |
| | | 37 | alpha chain | EWTQDRAKPVTQIVSAEAWGRADCGFTSVSYQQGVLSATILYE TSIRAVFIFLWLQLDLVNGENVEQHPSTLSVQEGDSAVIKCTYSDSASNYFPWYKQELGKRPQLIIDIRSNVGEKKDQRIAV TLNKTAKHFSLHITETQPEDSAVYFCAASPNSGGYQKVTFGIGTKLQVIPIQNPDPAVYQLRDSKSSDKSVCLFTDFDSQTN VSQSKDSDVYITDKTVLDMRSMDFKSNSAVAWSNKSDFACANAFNNSIIPEDTFFPSPESSCDVKLVEKSFETDTNLNFQ NLSVIGFRILLLKVAGFNLLMTLRLWSS |
| | | 38 | beta chain | NLSVIGFRILLLKVAGFNLLMTLRLWSS GTRLLCWAALCLLGAELTEAGVAQSPRYKIIEKRQSVAFWCNPISGHATLYWYQQILGQGPKLLIQFQNNGVVDDSQLPK DRFSAERLKGVDSTLKIQPAKLEDSAVYLCASSQHLAFTPNYGYTFGSGTRLTVVDLNKVFPPEVAVFEPSEAEISHTQKATL VCLATGFFPDHVELSWWVNGKEVHSGVSTDPQPLKEQPALNDSRYCLSSRLRVSATFWQNPRNHFRCQVQFYGLSEND EWTQDRAKPVTQIVSAEAWGRADCGFTSVSYQQGVLSATILYEILLGKATLYAVLVSALVLMAMVKRKDF |
| | | 167 | Peptide | STDLPILLK |
| | | | HLA | A*11:01 |
| | | 39 | CDR1 alpha | TRDTTYY |
| | | 40 | CDR2 alpha | RNSFDEQN |
| | | 41 | CDR3 alpha | CALRGGNTGKLIF |
| | | 42 | CDR1 beta | PRHDT |
| | | 43 | CDR2 beta | FYEKMQ |
| | | 44 | CDR3 beta | CASSPGPGGETQYF |
| **4** | **5'UTR HOXC13** | 45 | alpha chain variable region | LTASLLRAVIASICVVSSMAQKVTQAQTEISVVEKEDVTLDCVYETRDTTYYLFWYKQPPSGELVFLIRRNSFDEQNEISGRYSWNFQKSTS SFNFTITASQVVDSAVYFCALRGGNTGKLIFGQGTTLQVKP |
| | | 46 | beta chain variable region | LSPDLPDSAWNTRLLCRVMLCLLGAGSVAAGVIQSPRHLIKEKRETATLKCYPIPRHDTVYWYQQGPGQDPQFLISFYEKMQSDKGSIPD RFSAQQFSDYHSELNMSSLELGDSALYFCASSPGPGGETQYFGPGTRLLVL |
| | | 47 | alpha chain extracelular region | LTASLLRAVIASICVVSSMAQKVTQAQTEISVVEKEDVTLDCVYETRDTTYYLFWYKQPPSGELVFLIRRNSFDEQNEISGRYSWNFQKSTS SFNFTITASQVVDSAVYFCALRGGNTGKLIFGQGTTLQVKPIQNPDPAVYQLRDSKSSDKSVCLFTDFDSQTNVSQSKDSDVYITDKTVLD MRSMDFKSNSAVAWSNKSDFACANAFNNSIIPEDTFFPSPESSCDVKLVEKSFETDTNLNFQNLS |
| | | 48 | beta chain extracelular region | LSPDLPDSAWNTRLLCRVMLCLLGAGSVAAGVIQSPRHLIKEKRETATLKCYPIPRHDTVYWYQQGPGQDPQFLISFYEKMQSDKGSIPD RFSAQQFSDYHSELNMSSLELGDSALYFCASSPGPGGETQYFGPGTRLLVLDLKNVFPPEVAVFEPSEAEISHTQKATLVCLATGFYPDHV ELSWWVNGKEVHSGVSTDPQPLKEQPALNDSRYCLSSRLRVSATFWQNPRNHFRCQVQFYGLSENDEWTQDRAKPVTQIVSAEAWG RADCGFTSESYQQGVLSA |
| | | 49 | alpha chain | LTASLLRAVIASICVVSSMAQKVTQAQTEISVVEKEDVTLDCVYETRDTTYYLFWYKQPPSGELVFLIRRNSFDEQNEISGRYSWNFQKSTS SFNFTITASQVVDSAVYFCALRGGNTGKLIFGQGTTLQVKPIQNPDPAVYQLRDSKSSDKSVCLFTDFDSQTNVSQSKDSDVYITDKTVLD MRSMDFKSNSAVAWSNKSDFACANAFNNSIIPEDTFFPSPESSCDVKLVEKSFETDTNLNFQNLSVIGFRILLLKVAGFNLLMTLRLWSS |
| | | 50 | beta chain | LSPDLPDSAWNTRLLCRVMLCLLGAGSVAAGVIQSPRHLIKEKRETATLKCYPIPRHDTVYWYQQGPGQDPQFLISFYEKMQSDKGSIPD RFSAQQFSDYHSELNMSSLELGDSALYFCASSPGPGGETQYFGPGTRLLVLDLKNVFPPEVAVFEPSEAEISHTQKATLVCLATGFYPDHV ELSWWVNGKEVHSGVSTDPQPLKEQPALNDSRYCLSSRLRVSATFWQNPRNHFRCQVQFYGLSENDEWTQDRAKPVTQIVSAEAWG RADCGFTSESYQQGVLSATILYEILLGKATLYAVLVSALVLMAMVKRKDSRG |
| | | 168 | Peptide | SSLPLASGVFKK |
| | | | HLA | A*11:01 |

| **TCR number** | **Reactiv ity** | **SEQ ID NO:** | **Element** | **Sequence** |
|---|---|---|---|---|
| | | 51 | CDR1 alpha | ATGYPS |
| | | 52 | CDR2 alpha | ATKADDK |
| | | 53 | CDR3 alpha | CALSDLGIQGAQKLVF |
| | | 54 | CDR1 beta | MDHEN |
| | | 55 | CDR2 beta | SYDVKM |
| | | 56 | CDR3 beta | CASSLYEGTYYGYTF |
| | **5'UTR ZKSCA N1** | 57 | alpha chain variable region | NYSPGLVSLILLLLGRTRGNSVTQMEGPVTLSEEAFLTINCTYTATGYPSLFWYVQYPGEGLQLLLKATKADDKGSNKGFEATYRKETTSFH LEKGSVQVSDSAVYFCALSDLGIQGAQKLVFGQGTRLTINP |
| | | 58 | beta chain variable region | GIRLLCRVAFCFLAVGLVDVKVTQSSRYLVKRTGEKVFLECVQDMDHENMFWYRQDPGLGLRLIYFSYDVKMKEKGDIPEGYSVSREKKE RFSLILESASTNQTSMYLCASSLYEGTYYGYTFGSGTRLTVV |
| **5** | | 59 | alpha chain extracelular region | NYSPGLVSLILLLLGRTRGNSVTQMEGPVTLSEEAFLTINCTYTATGYPSLFWYVQYPGEGLQLLLKATKADDKGSNKGFEATYRKETTSFH LEKGSVQVSDSAVYFCALSDLGIQGAQKLVFGQGTRLTINPIQNPDPAVYQLRDSKSSDKSVCLFTDFDSQTNVSQSKDSDVYITDKTVLD MRSMDFKSNSAVAWSNKSDFACANAFNNSIIPEDTFFPSPESSCDVKLVEKSFETDTNLNFQNLS |
| | | 60 | beta chain extracelular region | GIRLLCRVAFCFLAVGLVDVKVTQSSRYLVKRTGEKVFLECVQDMDHENMFWYRQDPGLGLRLIYFSYDVKMKEKGDIPEGYSVSREKKE RFSLILESASTNQTSMYLCASSLYEGTYYGYTFGSGTRLTVVDLNKVFPPEVAVFEPSEAEISHTQKATLVCLATGFFPDHVELSWWVNGK EVHSGVSTDPQPLKEQPALNDSRYCLSSRLRVSATFWQNPRNHFRCQVQFYGLSENDEWTQDRAKPVTQIVSAEAWGRADCGFTSVS YQQGVLSATILYE |
| | | 61 | alpha chain | NYSPGLVSLILLLLGRTRGNSVTQMEGPVTLSEEAFLTINCTYTATGYPSLFWYVQYPGEGLQLLLKATKADDKGSNKGFEATYRKETTSFH LEKGSVQVSDSAVYFCALSDLGIQGAQKLVFGQGTRLTINPIQNPDPAVYQLRDSKSSDKSVCLFTDFDSQTNVSQSKDSDVYITDKTVLD MRSMDFKSNSAVAWSNKSDFACANAFNNSIIPEDTFFPSPESSCDVKLVEKSFETDTNLNFQNLSVIGFRILLLKVAGFNLLMTLRLWSS |
| | | 62 | beta chain | GIRLLCRVAFCFLAVGLVDVKVTQSSRYLVKRTGEKVFLECVQDMDHENMFWYRQDPGLGLRLIYFSYDVKMKEKGDIPEGYSVSREKKE RFSLILESASTNQTSMYLCASSLYEGTYYGYTFGSGTRLTVVDLNKVFPPEVAVFEPSEAEISHTQKATLVCLATGFFPDHVELSWWVNGK EVHSGVSTDPQPLKEQPALNDSRYCLSSRLRVSATFWQNPRNHFRCQVQFYGLSENDEWTQDRAKPVTQIVSAEAWGRADCGFTSVS YQQGVLSATILYEILLGKATLYAVLVSALVLMAMVKRKDF |
| | | 169 | Peptide | RVAEITGIVK |
| | | | HLA | A68*01 |

| **TCR number** | **Reactivity** | **SEQ ID NO:** | **Element** | **Sequence** |
|---|---|---|---|---|
| | | 63 | CDR1 alpha | DSAIYN |
| | | 64 | CDR2 alpha | IQSSQRE |
| | | 65 | CDR3 alpha | CAVRMEGGGADGLTF |
| | | 66 | CDR1 beta | MNHNS |
| | | 67 | CDR2 beta | SASEGT |
| | **MAGE A3_p167-176/MAG EA6_p.E16 8K** | 68 | CDR3 beta | CASSDFRGLGLDTQYF |
| | | 69 | alpha chain variable region | ETLLGLLILWLQLQWVSSKQEVTQIPAALSVPEGENLVLNCSFTDSAIYNLQWFRQDPGKGLTSLLLIQSSQREQTSGRLNASLDKSSGRSTLYI AASQPGDSATYLCAVRMEGGGADGLTFGKGTHLIIQP |
| **6** | | 70 | beta chain variable region | SIGLLCCVAFSLLWASPVNAGVTQTPKFQVLKTGQSMTLQCAQDMNHNSMYWYRQDPGMGLRLIYYSASEGTTDKGEVPNGYNVSRLNK REFSLRLESAAPSQTSVYFCASSDFRGLGLDTQYFGPGTRLTVL |
| | | 71 | alpha chain extracelular region | ETLLGLLILWLQLQWVSSKQEVTQIPAALSVPEGENLVLNCSFTDSAIYNLQWFRQDPGKGLTSLLLIQSSQREQTSGRLNASLDKSSGRSTLYI AASQPGDSATYLCAVRMEGGGADGLTFGKGTHLIIQPIQNPDPAVYQLRDSKSSDKSVCLFTDFDSQTNVSQSKDSDVYITDKTVLDMRSM DFKSNSAVAWSNKSDFACANAFNNSIIPEDTFFPSPESSCDVKLVEKSFETDTNLNFQNLS |
| | | 72 | beta chain extracelular region | SIGLLCCVAFSLLWASPVNAGVTQTPKFQVLKTGQSMTLQCAQDMNHNSMYWYRQDPGMGLRLIYYSASEGTTDKGEVPNGYNVSRLNK REFSLRLESAAPSQTSVYFCASSDFRGLGLDTQYFGPGTRLTVLDLKNVFPPEVAVFEPSEAEISHTQKATLVCLATGFYPDHVELSWWVNGK EVHSGVSTDPQPLKEQPALNDSRYCLSSRLRVSATFWQNPRNHFRCQVQFYGLSENDEWTQDRAKPVTQIVSAEAWGRADCGFTSESYQ QGVLSA |
| | | 73 | alpha chain | ETLLGLLILWLQLQWVSSKQEVTQIPAALSVPEGENLVLNCSFTDSAIYNLQWFRQDPGKGLTSLLLIQSSQREQTSGRLNASLDKSSGRSTLYI AASQPGDSATYLCAVRMEGGGADGLTFGKGTHLIIQPIQNPDPAVYQLRDSKSSDKSVCLFTDFDSQTNVSQSKDSDVYITDKTVLDMRSM DFKSNSAVAWSNKSDFACANAFNNSIIPEDTFFPSPESSCDVKLVEKSFETDTNLNFQNLSVIGFRILLLKVAGFNLLMTLRLWSS |
| | | 74 | beta chain | SIGLLCCVAFSLLWASPVNAGVTQTPKFQVLKTGQSMTLQCAQDMNHNSMYWYRQDPGMGLRLIYYSASEGTTDKGEVPNGYNVSRLNK REFSLRLESAAPSQTSVYFCASSDFRGLGLDTQYFGPGTRLTVLDLKNVFPPEVAVFEPSEAEISHTQKATLVCLATGFYPDHVELSWWVNGK EVHSGVSTDPQPLKEQPALNDSRYCLSSRLRVSATFWQNPRNHFRCQVQFYGLSENDEWTQDRAKPVTQIVSAEAWGRADCGFTSESYQ QGVLSATILYEILLGKATLYAVLVSALVLMAMVKRKDSRG |
| | | 170 | Peptide | MEVDPIGHLY |
| | | 75 | CDR1 alpha | ATGYPS |
| | | 76 | CDR2 alpha | ATKADDK |
| | | 77 | CDR3 alpha | CALSRTGRRALTF |
| | | 78 | CDR1 beta | MNHEY |
| | | 79 | CDR2 beta | SMNVEV |
| | | 80 | CDR3 beta | CASSFQGRNSPLHF |
| | | 81 | alpha chain variable region | NYSPGLVSLILLLLGRTRGNSVTQMEGPVTLSEEAFLTINCTYTATGYPSLFWYVQYPGEGLQLLLKATKADDKGSNKGFEATYRKETTS FHLEKGSVQVSDSAVYFCALSRTGRRALTFGSGTRLQVQP |
| | **MAGE A4** | 82 | beta chain variable region | GPQLLGYVVLCLLGAGPLEAQVTQNPRYLITVTGKKLTVTCSQNMNHEYMSWYRQDPGLGLRQIYYSMNVEVTDKGDVPEGYKVSR KEKRNFPLILESPSPNQTSLYFCASSFQGRNSPLHFGNGTRLTVT |
| **7** | | 83 | alpha chain extracelular region | NYSPGLVSLILLLLGRTRGNSVTQMEGPVTLSEEAFLTINCTYTATGYPSLFWYVQYPGEGLQLLLKATKADDKGSNKGFEATYRKETTS FHLEKGSVQVSDSAVYFCALSRTGRRALTFGSGTRLQVQPIQNPDPAVYQLRDSKSSDKSVCLFTDFDSQTNVSQSKDSDVYITDKTVL DMRSMDFKSNSAVAWSNKSDFACANAFNNSIIPEDTFFPSPESSCDVKLVEKSFETDTNLNFQNLS |
| | | 84 | beta chain extracelular region | GPQLLGYVVLCLLGAGPLEAQVTQNPRYLITVTGKKLTVTCSQNMNHEYMSWYRQDPGLGLRQIYYSMNVEVTDKGDVPEGYKVSR KEKRNFPLILESPSPNQTSLYFCASSFQGRNSPLHFGNGTRLTVTDLNKVFPPEVAVFEPSEAEISHTQKATLVCLATGFFPDHVELSW WVNGKEVHSGVSTDPQPLKEQPALNDSRYCLSSRLRVSATFWQNPRNHFRCQVQFYGLSENDEWTQDRAKPVTQIVSAEAWGRA DCGFTSVSYQQGVLSATILYE |
| | | 85 | alpha chain | NYSPGLVSLILLLLGRTRGNSVTQMEGPVTLSEEAFLTINCTYTATGYPSLFWYVQYPGEGLQLLLKATKADDKGSNKGFEATYRKETTS FHLEKGSVQVSDSAVYFCALSRTGRRALTFGSGTRLQVQPIQNPDPAVYQLRDSKSSDKSVCLFTDFDSQTNVSQSKDSDVYITDKTVL DMRSMDFKSNSAVAWSNKSDFACANAFNNSIIPEDTFFPSPESSCDVKLVEKSFETDTNLNFQNLSVIGFRILLLKVAGFNLLMTLRLW SS |
| | | 86 | beta chain | GPQLLGYVVLCLLGAGPLEAQVTQNPRYLITVTGKKLTVTCSQNMNHEYMSWYRQDPGLGLRQIYYSMNVEVTDKGDVPEGYKVSR KEKRNFPLILESPSPNQTSLYFCASSFQGRNSPLHFGNGTRLTVTDLNKVFPPEVAVFEPSEAEISHTQKATLVCLATGFFPDHVELSW WVNGKEVHSGVSTDPQPLKEQPALNDSRYCLSSRLRVSATFWQNPRNHFRCQVQFYGLSENDEWTQDRAKPVTQIVSAEAWGRA DCGFTSVSYQQGVLSATILYEILLGKATLYAVLVSALVLMAMVKRKDF |
| | | 87 | CDR1 alpha | DSASNY |
| | | 88 | CDR2 alpha | IRSNVGE |
| | | 89 | CDR3 alpha | CAAHGGGGSNYKLTF |
| | | 90 | CDR1 beta | SGHVS |
| | | 91 | CDR2 beta | FQNEAQ |
| | | 92 | CDR3 beta | CASSYSNQPQHF |
| | **MAGE A4_p145-154** | 93 | alpha chain variable region | TSIRAVFIFLWLQLDLVNGENVEQHPSTLSVQEGDSAVIKCTYSDSASNYFPWYKQELGKRPQLIIDIRSNVGEKKDQRIAVTLNKTAKH FSLHITETQPEDSAVYFCAAHGGGGSNYKLTFGKGTLLTVNP |
| | | 94 | beta chain variable region | GTRLLCWVVLGFLGTDHTGAGVSQSPRYKVAKRGQDVALRCDPISGHVSLFWYQQALGQGPEFLTYFQNEAQLDKSGLPSDRFFAE RPEGSVSTLKIQRTQKEDSAVYLCASSYSNQPQHFGDGTRLSIL |
| **8** | | 95 | alpha chain extracelular region | TSIRAVFIFLWLQLDLVNGENVEQHPSTLSVQEGDSAVIKCTYSDSASNYFPWYKQELGKRPQLIIDIRSNVGEKKDQRIAVTLNKTAKH FSLHITETQPEDSAVYFCAAHGGGGSNYKLTFGKGTLLTVNPIQNPDPAVYQLRDSKSSDKSVCLFTDFDSQTNVSQSKDSDVYITDKT VLDMRSMDFKSNSAVAWSNKSDFACANAFNNSIIPEDTFFPSPESSCDVKLVEKSFETDTNLNFQNLS |
| | | 96 | beta chain extracelular region | GTRLLCWVVLGFLGTDHTGAGVSQSPRYKVAKRGQDVALRCDPISGHVSLFWYQQALGQGPEFLTYFQNEAQLDKSGLPSDRFFAE RPEGSVSTLKIQRTQKEDSAVYLCASSYSNQPQHFGDGTRLSILDLNKVFPPEVAVFEPSEAEISHTQKATLVCLATGFFPDHVELSWW VNGKEVHSGVSTDPQPLKEQPALNDSRYCLSSRLRVSATFWQNPRNHFRCQVQFYGLSENDEWTQDRAKPVTQIVSAEAWGRADC GFTSVSYQQGVLSATILYE |
| | | 97 | alpha chain | TSIRAVFIFLWLQLDLVNGENVEQHPSTLSVQEGDSAVIKCTYSDSASNYFPWYKQELGKRPQLIIDIRSNVGEKKDQRIAVTLNKTAKH FSLHITETQPEDSAVYFCAAHGGGGSNYKLTFGKGTLLTVNPIQNPDPAVYQLRDSKSSDKSVCLFTDFDSQTNVSQSKDSDVYITDKT VLDMRSMDFKSNSAVAWSNKSDFACANAFNNSIIPEDTFFPSPESSCDVKLVEKSFETDTNLNFQNLSVIGFRILLLKVAGFNLLMTLR LWSS |
| | | 98 | beta chain | GTRLLCWVVLGFLGTDHTGAGVSQSPRYKVAKRGQDVALRCDPISGHVSLFWYQQALGQGPEFLTYFQNEAQLDKSGLPSDRFFAE RPEGSVSTLKIQRTQKEDSAVYLCASSYSNQPQHFGDGTRLSILDLNKVFPPEVAVFEPSEAEISHTQKATLVCLATGFFPDHVELSWW VNGKEVHSGVSTDPQPLKEQPALNDSRYCLSSRLRVSATFWQNPRNHFRCQVQFYGLSENDEWTQDRAKPVTQIVSAEAWGRADC GFTSVSYQQGVLSATILYEILLGKATLYAVLVSALVLMAMVKRKDF |
| | | 171 | Peptide | KRCFPVIFGK |
| | | | HLA | B*27:05 |
| | | 99 | CDR1 alpha | NSMFDY |
| | | 100 | CDR2 alpha | ISSIKDK |
| | | 101 | CDR3 alpha | CAASFGTDMRF |
| | | 102 | CDR1 beta | SEHNR |
| | | 103 | CDR2 beta | FQNEAQ |
| | | 104 | CDR3 beta | CASSPRTSGAKRSYN EQFF |
| | | 105 | alpha chain variable region | AMLLGASVLILWLQPDWVNSQQKNDDQQVKQNSPSLSVQEGRISILNCDYTNSMFDYFLWYKKYPAEGPTFLISISSIKDKNEDGRFT VFLNKSAKHLSLHIVPSQPGDSAVYFCAASFGTDMRFGAGTRLTVKP |
| | **MAGE A4_p145-154** | 106 | beta chain variable region | GTSLLCWMALCLLGADHADTGVSQDPRHKITKRGQNVTFRCDPISEHNRLYWYRQTLGQGPEFLTYFQNEAQLEKSRLLSDRFSAER PKGSFSTLEIQRTEQGDSAMYLCASSPRTSGAKRSYNEQFFGPGTRLTVL |
| **9** | | 107 | alpha chain extracelular region | AMLLGASVLILWLQPDWVNSQQKNDDQQVKQNSPSLSVQEGRISILNCDYTNSMFDYFLWYKKYPAEGPTFLISISSIKDKNEDGR FTVFLNKSAKHLSLHIVPSQPGDSAVYFCAASFGTDMRFGAGTRLTVKPIQNPDPAVYQLRDSKSSDKSVCLFTDFDSQTNVSQSKD SDVYITDKTVLDMRSMDFKSNSAVAWSNKSDFACANAFNNSIIPEDTFFPSPESSCDVKLVEKSFETDTNLNFQNLS |
| | | 108 | beta chain extracelular region | GTSLLCWMALCLLGADHADTGVSQDPRHKITKRGQNVTFRCDPISEHNRLYWYRQTLGQGPEFLTYFQNEAQLEKSRLLSDRFSAE RPKGSFSTLEIQRTEQGDSAMYLCASSPRTSGAKRSYNEQFFGPGTRLTVLDLKNVFPPEVAVFEPSEAEISHTQKATLVCLATGFYPD HVELSWWVNGKEVHSGVSTDPQPLKEQPALNDSRYCLSSRLRVSATFWQNPRNHFRCQVQFYGLSENDEWTQDRAKPVTQIVS AEAWGRADCGFTSESYQQGVLSA |
| | | 109 | alpha chain | AMLLGASVLILWLQPDWVNSQQKNDDQQVKQNSPSLSVQEGRISILNCDYTNSMFDYFLWYKKYPAEGPTFLISISSIKDKNEDGR FTVFLNKSAKHLSLHIVPSQPGDSAVYFCAASFGTDMRFGAGTRLTVKPIQNPDPAVYQLRDSKSSDKSVCLFTDFDSQTNVSQSKD SDVYITDKTVLDMRSMDFKSNSAVAWSNKSDFACANAFNNSIIPEDTFFPSPESSCDVKLVEKSFETDTNLNFQNLSVIGFRILLLKVA GFNLLMTLRLWSS |
| | | 110 | beta chain | GTSLLCWMALCLLGADHADTGVSQDPRHKITKRGQNVTFRCDPISEHNRLYWYRQTLGQGPEFLTYFQNEAQLEKSRLLSDRFSAE RPKGSFSTLEIQRTEQGDSAMYLCASSPRTSGAKRSYNEQFFGPGTRLTVLDLKNVFPPEVAVFEPSEAEISHTQKATLVCLATGFYPD HVELSWWVNGKEVHSGVSTDPQPLKEQPALNDSRYCLSSRLRVSATFWQNPRNHFRCQVQFYGLSENDEWTQDRAKPVTQIVS AEAWGRADCGFTSESYQQGVLSATILYEILLGKATLYAVLVSALVLMAMVKRKDSRG |
| | | 171 | Peptide | KRCFPVIFGK |
| | | | HLA | B*27:05 |
| | | 111 | CDR1 alpha | TTLSN |
| | | 112 | CDR2 alpha | LVKSGEV |
| | | 113 | CDR3 alpha | CAVCRMDSSYKLIF |
| | | 114 | CDR1 beta | SEHNR |
| | | 115 | CDR2 beta | FQNEAQ |
| | | 116 | CDR3 beta | CASSLVEGGPGGFGNNEQFF |
| | **PIK3CA p.E545K** | 117 | alpha chain variable region | LLITSMLVLWMQLSQVNGQQVMQIPQYQHVQEGEDFTTYCNSSTTLSNMQWYKQPGGHPVFLMQLVKSGEVKKQKLTFQFGEAKKNSSLHIT ATQTTDVGTYFCAVCRMDSSYKLIFGSGTLLVP |
| | | 118 | beta chain variable region | GTSLLCWMALCLLGADHADTGVSQDPHKITKGQNVTFCDPISEHNRLYWYRQTLGQGPEFLTYFQNEAQLEKSLLSDRFSAEPKGSFSTLEIQRTE QGDSAMYLCASSLVEGGPGGFGNNEQFFGPGTRLTVL |
| **10** | | 119 | alpha chain extracelular region | LLITSMLVLWMQLSQVNGQQVMQIPQYQHVQEGEDFTTYCNSSTTLSNMQWYKQPGGHPVFLMQLVKSGEVKKQKLTFQFGEAKKNSSLHIT ATQTTDVGTYFCAVCRMDSSYKLIFGSGTLLVPIQNPDPAVYQLRDSKSSDKSVCLFTDFDSQTNVSQSKDSDVYITDKTVLDMRSMDFKSNSAV AWSNKSDFACANAFNNSIIPEDTFFPSPESSCDVKLVEKSFETDTNLNFQNLS |
| | | 120 | beta chain extracelular region | GTSLLCWMALCLLGADHADTGVSQDPHKITKGQNVTFCDPISEHNRLYWYRQTLGQGPEFLTYFQNEAQLEKSLLSDRFSAEPKGS FSTLEIQRTEQGDSAMYLCASSLVEGGPGGFGNNEQFFGPGTRLTVLDLKNVFPPEVAVFEPSEAEISHTQKATLVCLATGFYPDHV ELSWWVNGKEVHSGVSTDPQPLKEQPALNDSRYCLSSRLRVSATFWQNPRNHFRCQVQFYGLSENDEWTQDRAKPVTQIVSAEA WGRADCGFTSESYQQGVLSA |
| | | 121 | alpha chain | LLITSMLVLWMQLSQVNGQQVMQIPQYQHVQEGEDFTTYCNSSTTLSNMQWYKQPGGHPVFLMQLVKSGEVKKQKLTFQFGE AKKNSSLHITATQTTDVGTYFCAVCRMDSSYKLIFGSGTLLVPIQNPDPAVYQLRDSKSSDKSVCLFTDFDSQTNVSQSKDSDVYITD KTVLDMRSMDFKSNSAVAWSNKSDFACANAFNNSIIPEDTFFPSPESSCDVKLVEKSFETDTNLNFQNLSVIGFRILLLKVAGFNLLM TLRLWSS |
| | | 122 | beta chain | GTSLLCWMALCLLGADHADTGVSQDPHKITKGQNVTFCDPISEHNRLYWYRQTLGQGPEFLTYFQNEAQLEKSLLSDRFSAEPKGS FSTLEIQRTEQGDSAMYLCASSLVEGGPGGFGNNEQFFGPGTRLTVLDLKNVFPPEVAVFEPSEAEISHTQKATLVCLATGFYPDHV ELSWWVNGKEVHSGVSTDPQPLKEQPALNDSRYCLSSRLRVSATFWQNPRNHFRCQVQFYGLSENDEWTQDRAKPVTQIVSAEA WGRADCGFTSESYQQGVLSATILYEILLGKATLYAVLVSALVLMAMVKRKDSRG |
| | | 172 | Peptide | STRDPLSEITK |
| | | | HLA | A*11:01 |
| | | 123 | CDR1 alpha | TSGFYG |
| | | 124 | CDR2 alpha | NALDGL |
| | | 125 | CDR3 alpha | CAVRDGNFNKFYF |
| | | 126 | CDR1 beta | GTSNPN |
| | | 127 | CDR2 beta | SVGIG |
| | **MAGE A4** | 128 | CDR3 beta | CAWTGGGGHQKRDTQYF |
| | | 129 | alpha chain variable region | WGAFLLYVSMKMGGTAGQSLEQPSEVTAVEGAIVQINCTYQTSGFYGLSWYQQHDGGAPTFLSYNALDGLEETGRFSSFLSRSDSYGYLL LQELQMKDSASYFCAVRDGNFNKFYFGSGTKLNVKP |
| **11** | | 130 | beta chain variable region | LCSLLALLLGTFFGVRSQTIHQWPATLVQPVGSPLSLECTVEGTSNPNLYWYRQAAGRGLQLLFYSVGIGQISSEVPQNLSASRPQDRQFIL SSKKLLLSDSGFYLCAWTGGGGHQKRDTQYFGPGTRLTVL |
| | | 131 | alpha chain extracelular region | WGAFLLYVSMKMGGTAGQSLEQPSEVTAVEGAIVQINCTYQTSGFYGLSWYQQHDGGAPTFLSYNALDGLEETGRFSSFLSRSDSYGYLL LQELQMKDSASYFCAVRDGNFNKFYFGSGTKLNVKPIQNPDPAVYQLRDSKSSDKSVCLFTDFDSQTNVSQSKDSDVYITDKTVLDMRS MDFKSNSAVAWSNKSDFACANAFNNSIIPEDTFFPSPESSCDVKLVEKSFETDTNLNFQNLS |
| | | 132 | beta chain extracelular region | LCSLLALLLGTFFGVRSQTIHQWPATLVQPVGSPLSLECTVEGTSNPNLYWYRQAAGRGLQLLFYSVGIGQISSEVPQNLSASRPQDRQFIL SSKKLLLSDSGFYLCAWTGGGGHQKRDTQYFGPGTRLTVLDLKNVFPPEVAVFEPSEAEISHTQKATLVCLATGFYPDHVELSWWVNGKE VHSGVSTDPQPLKEQPALNDSRYCLSSRLRVSATFWQNPRNHFRCQVQFYGLSENDEWTQDRAKPVTQIVSAEAWGRADCGFTSESYQ QGVLSA |
| | | 133 | alpha chain | WGAFLLYVSMKMGGTAGQSLEQPSEVTAVEGAIVQINCTYQTSGFYGLSWYQQHDGGAPTFLSYNALDGLEETGRFSSFLSRSDSYGYLL LQELQMKDSASYFCAVRDGNFNKFYFGSGTKLNVKPIQNPDPAVYQLRDSKSSDKSVCLFTDFDSQTNVSQSKDSDVYITDKTVLDMRS MDFKSNSAVAWSNKSDFACANAFNNSIIPEDTFFPSPESSCDVKLVEKSFETDTNLNFQNLSVIGFRILLLKVAGFNLLMTLRLWSS |
| | | 134 | beta chain | LCSLLALLLGTFFGVRSQTIHQWPATLVQPVGSPLSLECTVEGTSNPNLYWYRQAAGRGLQLLFYSVGIGQISSEVPQNLSASRPQDRQFIL SSKKLLLSDSGFYLCAWTGGGGHQKRDTQYFGPGTRLTVLDLKNVFPPEVAVFEPSEAEISHTQKATLVCLATGFYPDHVELSWWVNGKE VHSGVSTDPQPLKEQPALNDSRYCLSSRLRVSATFWQNPRNHFRCQVQFYGLSENDEWTQDRAKPVTQIVSAEAWGRADCGFTSESYQ QGVLSATILYEILLGKATLYAVLVSALVLMAMVKRKDSRG |
| | | | HLA | B*51:01 |

### Conjugates of sTCRs and therapeutic or diagnostic agents

In another embodiment, the soluble TCR is conjugated to an agent of interest, which may be a therapeutic agent and/or a diagnostic agent. This allows the delivery of the therapeutic or diagnostic agent to cells which show on their surface the complex of the HLA molecule and antigenic polypeptide to which the sTCR binds.

As used herein, the term "therapeutic agent" refers to a biological or chemical agent used for treatment, curing, mitigating, or preventing deleterious conditions in a subject. In some embodiments, the term "therapeutic agent" also encompasses any preventive or prophylactic agent. The term "therapeutic agent" also includes substances and agents for combating a disease, condition, or disorder of a subject, and includes drugs, diagnostics, and instrumentation. "Therapeutic agent" also includes anything used in medical diagnosis, or in restoring, correcting, or modifying physiological functions. The terms "therapeutic agent" and "pharmaceutically active agent" are used interchangeably herein.

A therapeutic agent can be selected according to the treatment objective and biological action desired. Thus, a therapeutic agent can be selected from any class suitable for the therapeutic objective. Further, the therapeutic agent may be selected or arranged to provide therapeutic activity over a period of time.

Examples of suitable therapeutic agents include, but are not limited to cytotoxic and/or cytostatic agents, i.e. agents known to inhibit the growth and/or replication of and/or kill cells, and in particular cancer and/or tumor cells. Numerous agents having cytotoxic and/or cytostatic properties are known in the literature. Non-limiting examples of classes of cytotoxic and/or cytostatic agents include, by way of example and not limitation, radionuclides, alkylating agents, DNA cross-linking agents, DNA intercalating agents (e.g., groove binding agents such as minor groove binders), cell cycle modulators, apoptosis regulators, kinase inhibitors, protein synthesis inhibitors, mitochondria inhibitors, nuclear export inhibitors, topoisomerase I inhibitors, topoisomerase II inhibitors, RNA/DNA antimetabolites and antimitotic agents.

Specific non-limiting examples of agents within certain of these various classes are provided below.

Alkylating Agents: asaley (L-Leucine, N-[N-acetyl-4-[bis-(2-chloroethyl)amino]-DL-phenylalanyl]-, ethylester); AZQ (1,4-cyclohexadiene-1,4-dicarbamic acid, 2,5-bis(1-aziridinyl)-3,6-dioxo-, diethyl ester); BCNU (N,N'-Bis(2-chloroethyl)-N-nitrosourea); busulfan (1,4-butanediol dimethanesulfonate); (carboxyphthalato)platinum; CBDCA (cis-(1,1-cyclobutanedicarboxylato)diammineplatinum(II))); CCNU (N-(2-chloroethyl)-N'-cyclohexyl-N-nitrosourea); CHIP (iproplatin; NSC 256927); chlorambucil; chlorozotocin (2-[[[(2-chloroethyl) nitrosoamino]carbonyl]amino]-2-deoxy-D-glucopyranose); cis-platinum (cisplatin); clomesone; cyanomorpholinodoxorubicin; cyclodisone; dianhydrogalactitol (5,6-diepoxydulcitol); fluorodopan ((5-[(2-chloroethyl)-(2-fluoroethyl)amino]-6-methyl-uracil); hepsulfam; hycanthone; indolinobenzodiazepine dimer DGN462; melphalan; methyl CCNU ((1-(2-chloroethyl)-3-(trans-4-methylcyclohexane)-1-nitrosourea); mitomycin C; mitozolamide; nitrogen mustard ((bis(2-chloroethyl) methylamine hydrochloride); PCNU ((1-(2-chloroethyl)-3-(2,6-dioxo-3-piperidyl)-1-nitrosourea)); piperazine alkylator ((1-(2-chloroethyl)-4-(3-chloropropyl)-piperazine dihydrochloride)); piperazinedione; pipobroman (N,N'-bis(3-bromopropionyl) piperazine); porfiromycin (N-methylmitomycin C); spirohydantoin mustard; teroxirone (triglycidylisocyanurate); tetraplatin; thio-tepa (N,N',N"-tri-1,2-ethanediylthio phosphoramide); triethylenemelamine; uracil nitrogen mustard (desmethyldopan); Yoshi-864 ((bis(3-mesyloxy propyl)amine hydrochloride).

DNA Alkylating-like Agents: Cisplatin; Carboplatin; Nedaplatin; Oxaliplatin; Satraplatin; Triplatin tetranitrate; Procarbazine; altretamine; dacarbazine; mitozolomide; temozolomide.

Alkylating Antineoplastic Agents: Carboquone; Carmustine; Chlornaphazine; Chlorozotocin; Duocarmycin; Evofosfamide; Fotemustine; Glufosfamide; Lomustine; Mannosulfan; Nimustine; Phenanthriplatin; Pipobroman; Ranimustine; Semustine; Streptozotocin; ThioTEPA; Treosulfan; Triaziquone; Triethylenemelamine; Triplatin tetranitrate.

DNA replication and repair inhibitors: Altretamine; Bleomycin; Dacarbazine; Dactinomycin; Mitobronitol; Mitomycin; Pingyangmycin; Plicamycin; Procarbazine; Temozolomide; ABT-888 (veliparib); olaparib; KU-59436; AZD-2281; AG-014699; BSI-201; BGP-15; INO-1001; ONO-2231.

Cell Cycle Modulators: Paclitaxel; Nab-Paclitaxel; Docetaxel; Vincristine; Vinblastine; ABT-348; AZD-1152; MLN-8054; VX-680; Aurora A-specific kinase inhibitors; Aurora B-specific kinase inhibitors and pan-Aurora kinase inhibitors; AZD-5438; BMI-1040; BMS-032; BMS-387; CVT-2584; flavopyridol; GPC-286199; MCS-5A; PD0332991; PHA-690509; seliciclib (CYC-202, R-roscovitine); ZK-304709; AZD4877, ARRY-520, GSK923295A.

Apoptosis Regulators: AT-101 ((-)gossypol); G3139 or oblimersen (Bcl-2-targeting antisense oligonucleotide); IPI-194; IPI-565; N-(4-(4-((4'-chloro(1,1'-biphenyl)-2-yl)methyl)piperazin-1-ylbenzoyl)-4-(((1R)-3-(dimethylamino)-1-((phenylsulfanyl)methyl)propyl)amino)-3-nitrobenzenesulfonamide); N-(4-(4-((2-(4-chlorophenyl)-5,5-dimethyl-1-cyclohex-1-en-1-yl)methyl)piperazin-1-yl)benzoyl)-4-(((1R)-3-(morpholin-4-yl)-1-((phenylsulfanyl)methyl)propyl)amino)-3-((trifluoromethyl)sulfonyl)benzenesulfonamide; GX-070 (Obatoclax^{®}; 1H-Indole, 2-(2-((3,5-dimethyl-1H-pyrrol-2-yl)methylene)-3-methoxy-2H-pyrrol-5-yl)-)); HGS1029; GDC-0145; GDC-0152; LCL-161; LBW-242; venetoclax; agents that target TRAIL or death receptors (e.g., DR4 and DR5) such as ETR2-ST01, GDC0145, HGS-1029, LBY-135, PRO-1762; drugs that target caspases, caspase-regulators, BCL-2 family members, death domain proteins, TNF family members, Toll family members, and/or NF-kappa-B proteins.

Angiogenesis Inhibitors: ABT-869; AEE-788; axitinib (AG-13736); AZD-2171; CP-547,632; IM-862; pegaptamib; sorafenib; BAY43-9006; pazopanib (GW-786034); vatalanib (PTK-787, ZK-222584); sunitinib; SU-11248; VEGF trap; vandetanib; ABT-165; ZD-6474; DLL4 inhibitors.

Proteasome Inhibitors: Bortezomib; Carfilzomib; Epoxomicin; Ixazomib; Salinosporamide A.

Kinase Inhibitors: Afatinib; Axitinib; Bosutinib; Crizotinib; Dasatinib; Erlotinib; Fostamatinib; Gefitinib; Ibrutinib; Imatinib; Lapatinib; Lenvatinib; Mubritinib; Nilotinib; Pazopanib; Pegaptanib; Sorafenib; Sunitinib; SU6656; Vandetanib; Vemurafenib; CEP-701 (lesaurtinib); XL019; INCB018424 (ruxolitinib); ARRY-142886 (selemetinib); ARRY-438162 (binimetinib); PD-325901; PD-98059; AP-23573; CCI-779; everolimus; RAD-001; rapamycin; temsirolimus; ATP-competitive TORC1/TORC2 inhibitors including PI-103, PP242, PP30, Torin 1; LY294002; XL-147; CAL-120; ONC-21; AEZS-127; ETP-45658; PX-866; GDC-0941; BGT226; BEZ235; XL765.

Protein Synthesis Inhibitors: Streptomycin; Dihydrostreptomycin; Neomycin; Framycetin; Paromomycin; Ribostamycin; Kanamycin; Amikacin; Arbekacin; Bekanamycin; Dibekacin; Tobramycin; Spectinomycin; Hygromycin B; Paromomycin; Gentamicin; Netilmicin; Sisomicin; Isepamicin; Verdamicin; Astromicin; Tetracycline; Doxycycline; Chlortetracycline; Clomocycline; Demeclocycline; Lymecycline; Meclocycline; Metacycline; Minocycline; Oxytetracycline; Penimepicycline; Rolitetracycline; Tetracycline; Glycylcyclines; Tigecycline; Oxazolidinone; Eperezolid; Linezolid; Posizolid; Radezolid; Ranbezolid; Sutezolid; Tedizolid; Peptidyl transferase inhibitors; Chloramphenicol; Azidamfenicol; Thiamphenicol; Florfenicol; Pleuromutilins; Retapamulin; Tiamulin; Valnemulin; Azithromycin; Clarithromycin; Dirithromycin; Erythromycin; Flurithromycin; Josamycin; Midecamycin; Miocamycin; Oleandomycin; Rokitamycin; Roxithromycin; Spiramycin; Troleandomycin; Tylosin; Ketolides; Telithromycin; Cethromycin; Solithromycin; Clindamycin; Lincomycin; Pirlimycin; Streptogramins; Pristinamycin; Quinupristin/dalfopristin; Virginiamycin.

Histone deacetylase inhibitors: Vorinostat; Romidepsin; Chidamide; Panobinostat; Valproic acid; Belinostat; Mocetinostat; Abexinostat; Entinostat; SB939 (pracinostat); Resminostat; Givinostat; Quisinostat; thioureidobutyronitrile (Kevetrin^{™}); CUDC-10; CHR-2845 (tefinostat); CHR-3996; 4SC-202; CG200745; ACY-1215 (rocilinostat); ME-344; sulforaphane.

Topoisomerase I Inhibitors: camptothecin; various camptothecin derivatives and analogs (for example, NSC 100880, NSC 603071, NSC 107124, NSC 643833, NSC 629971, NSC 295500, NSC 249910, NSC 606985, NSC 74028, NSC 176323, NSC 295501, NSC 606172, NSC 606173, NSC 610458, NSC 618939, NSC 610457, NSC 610459, NSC 606499, NSC 610456, NSC 364830, and NSC 606497); morpholinisoxorubicin; SN-38.

Topoisomerase II Inhibitors: doxorubicin; amonafide (benzisoquinolinedione); m-AMSA (4'-(9-acridinylamino)-3'-methoxymethanesulfonanilide); anthrapyrazole derivative ((NSC 355644); etoposide (VP-16); pyrazoloacridine ((pyrazolo[3,4,5-kl] acridine-2(6H)-propanamine, 9-methoxy-N, N-dimethyl-5-nitro-, monomethanesulfonate); bisantrene hydrochloride; daunorubicin; deoxydoxorubicin; mitoxantrone; menogaril; N,N-dibenzyl daunomycin; oxanthrazole; rubidazone; teniposide.

DNA Intercalating Agents: anthramycin; chicamycin A; tomaymycin; DC-81; sibiromycin; pyrrolobenzodiazepine derivative; SGD-1882 ((S)-2-(4-aminophenyl)-7-methoxy-8-(3-4(S)-7-methoxy-2-(4-methoxyphenyl)-5-oxo-5,11a-dihydro-1H-benzo[e]pyrrolo[1,2-a][1,4]diazepin-8-yl)oxy)propoxy)-1H-benzo[e]pyrrolo[1,2-a][1,4]diazepin-5(11aH)-one); SG2000 (SJG-136; (11aS,11a'S)-8,8'-(propane-1,3-diylbis(oxy))bis(7-methoxy-2-methylene-2,3-dihydro-1H-benzo[e]pyrrolo[1,2-a][1,4]diazepin-5(11aH)-one)).

RNA/DNA Antimetabolites: L-alanosine; 5-azacytidine; 5-fluorouracil; acivicin; aminopterin derivative N-[2-chloro-5[[(2,4-diamino-5-methyl-6-quinazolinyl)methyl]amino]benzoyl] L-aspartic acid (NSC 132483); aminopterin derivative N-[4-[[(2,4-diamino-5-ethyl-6-quinazolinyl)methyl]amino]benzoyl]L-aspartic acid; aminopterin derivative N-[2-chloro-4-[[(2,4-diamino-6-pteridinyl)methyl] amino]benzoyl] L-aspartic acid monohydrate; antifolate PT523 ((Nα -(4-amino-4-deoxypteroyl)-Nγ -hemiphthaloyl-L-ornithine)); Baker's soluble antifol (NSC 139105); dichlorallyl lawsone ((2-(3,3-dichloroallyl)-3-hydroxy-1,4-naphthoquinone); brequinar; ftorafur ((pro-drug; 5-fluoro-1-(tetrahydro-2-furyl)-uracil); 5,6-dihydro-5-azacytidine; methotrexate; methotrexate derivative (N-[[4-[[(2,4-diamino-6-pteridinyl)methyl]methylamino]-1-naphthalenyl]carbonyl]L-glutamic acid); PALA ((N-(phosphonoacetyl)-L-aspartate); pyrazofurin; trimetrexate.

DNA Antimetabolites: 3-HP; 2'-deoxy-5-fluorouridine; 5-HP; α-TGDR (α-2'-deoxy-6-thioguanosine); aphidicolin glycinate; ara C (cytosine arabinoside); 5-aza-2'-deoxycytidine; β-TGDR (β-2'-deoxy-6-thioguanosine); cyclocytidine; guanazole; hydroxyurea; inosine glycodialdehyde; macbecin II; pyrazoloimidazole; thioguanine; thiopurine.

Mitochondria Inhibitors: pancratistatin; phenpanstatin; rhodamine-123; edelfosine; d-alpha-tocopherol succinate; compound 11β; aspirin; ellipticine; berberine; cerulenin; GX015-070 (Obatoclax^{®}; 1H-Indole, 2-(2-((3,5-dimethyl-1H-pyrrol-2-yl)methylene)-3-methoxy-2H-pyrrol-5-yl)-); celastrol (tripterine); metformin; Brilliant green; ME-344.

Antimitotic Agents: allocolchicine; auristatins, such as MMAE (monomethyl auristatin E) and MMAF (monomethyl auristatin F); halichondrin B; cemadotin; colchicine; cholchicine derivative (N-benzoyl-deacetyl benzamide); dolastatin-10; dolastatin-15; maytansine; maytansinoids, such as DM1 (N2 '-deacetyl-N2 '-(3-mercapto-1-oxopropyl)-maytansine); rhozoxin; paclitaxel; paclitaxel derivative ((2'-N-[3-(dimethylamino)propyl]glutaramate paclitaxel); docetaxel; thiocolchicine; trityl cysteine; vinblastine sulfate; vincristine sulfate.

Nuclear Export Inhibitors: callystatin A; delactonmycin; KPT-185 (propan-2-yl (Z)-3-[3-[3-methoxy-5-(trifluoromethyl)phenyl]-1,2,4-triazol-1-yl]prop-2-enoate); kazusamycin A; leptolstatin; leptofuranin A; leptomycin B; ratjadone; Verdinexor ((Z)-3-[3-[3,5-bis(trifluoromethyl)phenyl]-1,2,4-triazol-1-yl]-N'-pyridin-2-ylprop-2-enehydrazide).

Hormonal Therapies: anastrozole; exemestane; arzoxifene; bicalutamide; cetrorelix; degarelix; deslorelin; trilostane; dexamethasone; flutamide; raloxifene; fadrozole; toremifene; fulvestrant; letrozole; formestane; glucocorticoids; doxercalciferol; sevelamer carbonate; lasofoxifene; leuprolide acetate; megesterol; mifepristone; nilutamide; tamoxifen citrate; abarelix; prednisone; finasteride; rilostane; buserelin; luteinizing hormone releasing hormone (LHRH); Histrelin; trilostane or modrastane; fosrelin; goserelin.

Any of these agents include, or that may be modified to include, a site of attachment to a sTCR.
Peptidic cytotoxins, i.e. proteins or fragments thereof with the ability to kill mammalian cells. For example, ricin, diphtheria toxin, pseudomonas bacterial exotoxin A, Dnase and Rnase;
Radio-nuclides, i.e. unstable isotopes of elements which decay with the concurrent emission of one or more of α or β particles, or γ rays. For example, iodine 131, rhenium 186, indium 1 1 1 , yttrium 90, bismuth 210 and 213, actinium 225 and astatine 213; chelating agents may be used to facilitate the association of these radio-nuclides to the high affinity TCRs, or multimers thereof;
Immuno-stimulants, i.e. immune effector molecules which stimulate immune response. For example, cytokines such as IL-2 and IFN-g,
Chemokines such as IL-8, platelet factor 4, melanoma growth stimulatory protein, etc;
The term "diagnostic agent" refers to a detectable molecule or substance, such as a fluorescent molecule, a radioactive molecule or any other labels known in the art that provide (either directly or indirectly) a signal.

The term "detectable marker or label", as it is used herein, refers to molecules that can generate a signal which is detectable by means of a method suitable for said detection. The term "label" or "labelling group" refers to any detectable label. In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

Suitable markers include, among others, fluorescein, rhodamine, eosin and other fluorophores, radioisotopes, gold, gadolinium and other lanthanides, paramagnetic iron, fluorine-18 and other positron-emitting radionuclides. Furthermore, the probes or labels may be bi- or multifunctional and can be detected by means of more than one of the listed methods. The marker or label can be, for example, a chromogenic, fluorogenic, magnetic, electrodense, radioactive, and/or chemiluminescent compound. In a preferred embodiment of the present invention, the detectable label or marker is selected from the list consisting of: fluorescein isothiocyanate (FITC), allophycocyanin (APC), or any other relevant fluorescent dye in flow cytometry, biotin, horseradish peroxidase (HRP), gold beads, and paramagnetic iron.

### Antigen recognizing constructs containing additional polypeptide regions providing additional or modified functionalities

In another embodiment, the invention relates to an antigen recognizing construct selected from the group consisting of
(i) A first polypeptide comprising the TCR alpha chain variable region and a second polypeptide comprising the TCR beta chain variable region wherein the first and second polypeptides are connected by a disulfide bond or
(ii) A first polypeptide comprising the TCR alpha chain extracellular region and a second polypeptide comprising the TCR beta chain extracellular region wherein the first and second are connected by a disulfide bond
wherein the first and/or the second polypeptide forms part of a fusion protein which comprises an heterologous polypeptide region.

The term "heterologous polypeptide region" refers to any polypeptide sequence that does not form part of a TCR as it appears in nature. In some embodiments, the "heterologous polypeptide region" is selected from the group consisting of immune effector polypeptide, a half-life extending domain and a T-cell signaling domain.

In an embodiment, the modified TCRs according to the invention comprises an additional polypeptide region which is an immune effector polypeptide. This results in the so-called Immune mobilising monoclonal T-cell receptors Against Cancer or ImmTAC. These molecules are a class of bispecific biological drug suitable for the treatment of cancer and viral infections which combines cancer-recognizing TCRs with immune activating complexes.

In an embodiment, the immune effector polypeptide appears forming part of the fusion protein with the first polypeptide of the antigen recognizing construct. In an embodiment, the immune effector polypeptide appears forming part of the fusion protein with the second polypeptide of the antigen recognizing construct. In another embodiment, the immune effector polypeptide appears forming part of fusions proteins with the first and second polypeptides of the antigen recognizing construct.

The immune effector polypeptide may be present at the N-terminus and/or at the C-terminus of the fusion protein or proteins which form the antigen recognizing construct.

As used herein, the term "immune effector polypeptide" generally refers to any molecule which induces or stimulates an immune response through direct or indirect activation of the humoral or cellular arm of the immune system, such as by activation of T-cells. Examples of immune effector polypeptides include, but are not limited to, IL-1, IL-la, IL-3, IL-4, IL-5, IL-6, IL-7, IL-10, IL-11, IL-12, IL-13, IL-15, IL-21, IL-23, TGF-beta, IFN-gamma, TNFa, anti-CD2 antibody, anti-CD3 antibody, anti-CD4 antibody, anti-CD8 antibody, anti-CD44 antibody, anti-CD45RA antibody, anti- CD45RB antibody, anti-CD45RO antibody, anti-CD49a antibody, anti-CD49b antibody, anti- CD49c antibody, anti-CD49d antibody, anti-CD49e antibody, anti-CD49f antibody, anti-CD 16 antibody, anti-CD28 antibody, anti-IL-2R antibodies, viral proteins and peptides, and bacterial proteins or peptides.

In an embodiment, the modified TCRs according to the invention comprises an additional polypeptide region which is a half-life extending domain.

A "half-life extending domain", as used herein, refers to a protein domain for extending the half-life of the multi-domain binding protein, relative to a multi-domain binding protein lacking the half-life extending domain. Examples of half-life extending domains include, without limitation, a fragment crystallizable region (Fc region); an immunoglobulin-binding domain B (IgBD); albumin; ubiquitin; a poly-glycine; an elastin-like polypeptide; polysialic acid; a transferrin domain; a C-terminal peptide; a non-exact repeat peptide sequence; and a gelatin-like protein. In a particular embodiment, the half-life extending domain is an immunoglobulin Fc domain or an albumin binding domain.

In an embodiment, the half-life extending domain appears forming part of the fusion protein with the first polypeptide of the antigen recognizing construct. In an embodiment, the half-life extending domain appears forming part of the fusion protein with the second polypeptide of the antigen recognizing construct. In another embodiment, the half-life extending domain appears forming part of fusions proteins with the first and second polypeptides of the antigen recognizing construct.

The half-life extending domain may be present at the N-terminus and/or at the C-terminus of the fusion protein or proteins which form the antigen recognizing construct.

In an embodiment, the modified TCRs according to the invention comprises an additional polypeptide region which is a T-cell signaling domain. These constructs correspond to the so-called TCR-CAR. Typically, the T-cell signaling domain comprises a transmembrane region and a cytosolic or intracellular costimulatory domain from a T-cell receptor which are different from the transmembrane and cytosolic domain of the TCR from which the TCR part of the TCR-CAR derives.

As used herein, "transmembrane domain" (TMD) refers to the area of TCR that crosses the cell membrane. The transmembrane that can be used in the TCR-CAR of the invention is the transmembrane domain of a transmembrane protein (e.g., a type I transmembrane protein), an artificial hydrophobic sequence, or a combination thereof. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the TCR-CAR is used. In some instances, the transmembrane domain can be selected or modified by amino acid substitution to avoid binding of such domains to the transmembrane domains of the same or different surface membrane proteins, e.g., to minimize interactions with other members of the receptor complex.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the TCR-CAR has bound to a target. Non limiting examples or transmembrane domains of particular use in this invention may include at least the transmembrane region(s) of e.g., the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154, CD3 zeta, KIRDS2, OX40, CD2, CD27, LFA-1 (CD1 la, CD18), ICOS (CD 278), 4-1BB (CD137), GITR, CD40, CTLA4, BAFFR, HVEM (LIGHTR), SLAMF7, NKp80 (KLRFI), CD160, CD19, IL2R beta, IL2R gamma, IL7Ra, ITGA1, VLA1, CD49a, ITGA4, IA4 CD49D, ITGA6, VLA-6, CD49f, ITGAD, CDGA, CDGA, CD103, ITGAL, CDLa, LFA-1, ITGAM, CDIIb, ITGAX, CDIc, ITGB1, CD29, ITGB2, CD18, LFA-1, LGA ITGB7, TNFR2, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), SLAMF6 (NTB-A), Lyl08), SLAM (SLAMF1, CD150, IPO-3), BLAME (SLAMF8), SELPLG (CD162), LTBR, PAG/Cbp, NKp44, Kp30, NKp46, including NKG2D, and/or a transmembrane domain selected from the transmembrane domain of NKG2C.

"Intracellular signaling domain" or "cytosolic domain" or "signaling tail" as the term is used here, refers to the intracellular portion of a molecule and more specifically to any oligopeptide or polypeptide known to function as a domain that transmits a signal to cause activation or inhibition of a biological process in a cell. The intracellular signaling domain generates a signal that stimulates the immune effector function of TCR-CAR containing cells.

Generally, the whole intracellular signaling domain can be used; however, it is appreciated that it is not necessary to use the entire domain, provided that whatever part of the signaling domain that is used is still capable of transducing the effector function signal. It will also be appreciated that variants of such intracellular signaling domains with substantially the same or greater functional capability may also be used. By this we include the meaning that the variants should have substantially the same or greater transduction of the effector functional signal. Typically, substantially the same or greater signal transduction includes at least 80%, 85%, 90%, 95%, 100%, 105%, 110%, 115%, or 120%, or more of the signal transduction of the unmodified intracellular signaling domain, wherein signal transduction of the unmodified intracellular signaling domain corresponds to 100%. Methods for assessing transduction of effector function signal are well known to those skilled in the art and include, for example, assessing the amounts and/or activity of molecules (e.g. proteins such as cytokines) that are indicative of the transduced signal. Thus, when the signal is the cytolytic function of a T-cell, the methods may involve measurement of one or more cytokines secreted by the T-cell, which cytokines are known to have a cytolytic activity (e.g. IFN gamma). Another means of assessing the cytolytic function is by CFSE staining and counting positive cells by Flow cytometry or by a chromium release assay as is well known in the art.

In a particular embodiment the intracellular signaling domain of the TCR-CAR comprises a costimulatory domain, a primary signaling domain, or any combination thereof.

A primary intracellular signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs (e.g. 2, 3, 4, 5 or more ITAMs). Thus, the intracellular signaling domain may comprise one or more ITAMs. It will be appreciated that one or more ITAMs of the intracellular signaling domain may be modified, for example by mutation. The modification may be used to increase or decrease the signaling function of the ITAM as compared to the native ITAM domain.

Examples of ITAM containing primary intracellular signaling domains that are of particular use in the invention include those of CD3 zeta, Fc receptor gamma, Fc receptor beta, CD3 gamma, CD3 delta, CD3 epsilon, CD5, CD22, CD79a, CD79b, and CD66d. In a particular embodiment the at least one intracellular signaling domain of the CAR expressed by the immune cell of the invention is selected from a group consisting of CD3 zeta, Fc receptor gamma, Fc receptor beta, CD3 gamma, CD3 delta, CD3 epsilon, CD5, CD22, CD79a, CD79b, and CD66d. In a more particular embodiment the at least one intracellular signaling domain of the CAR expressed by the immune cell of the invention is the CD3-zeta intracellular domain.

The term "CD3" refers to the human CD3 protein complex, which is composed of six distinct chains (a CD3y chain (SwissProt P09693), a CD3δ chain (SwissProt P04234), two CD3ε chains (SwissProt P07766), and one CD3 zeta chain homodimer (SwissProt P20963) (ε γ: ε δ:ζζ), and which is associated with the T cell receptor α and β chain. The term includes any CD3 variants, isoforms and species homologs which are naturally expressed by cells, including T cells, or are expressed on cells transfected with genes or cDNA encoding the aforementioned chains.

The term "zeta" or alternatively "zeta chain", "CD3-zeta" or "TCR-zeta" is defined as the protein represented by GenBank entry No. BAG36664.1, or equivalent residues from a non-human species, such as a mouse, rodent, monkey, primate, etc., and a "zeta stimulating domain" or alternatively a "CD3 zeta stimulating domain" or "TCR zeta stimulating domain" is defined as amino acid residues of the cytoplasmic domain of the zeta chain that are sufficient for functional transmission of the primary signal required to activate T cells etc. In one aspect, the zeta cytoplasmic domain comprises residues 52 through 164 inclusive of a GenBank entry protein of BAG36664.1, or equivalent residues from a non-human species, for example, a mouse, rodent, monkey, primate, and the like, which are their functional orthologists.

As mentioned above, the intracellular signaling domain may comprise a primary intracellular signaling domain by itself, or it may comprise a primary intracellular signaling domain in combination with one or more secondary intracellular signaling domains, such as one or more costimulatory signaling domains. Thus, the intracellular signaling domain of the CAR may comprise the CD3 zeta signaling domain by itself or in combination with one or more other intracellular signaling domains such as one or more costimulatory signaling domains.

The costimulatory signaling domain refers to a portion of the TCR-CAR comprising the intracellular domain of a costimulatory molecule.

The term "co-stimulating molecule" refers to a recognizable T-cell binding partner that specifically binds to a co-stimulating ligand, thereby mediating the co-stimulatory response exerted by the T-cell, such as, but not limited to, proliferation. Co-stimulating molecules are cell surface molecules other than antigen-specific receptors or their ligands, which are necessary for an effective immune response. A costimulatory molecule may be a cell surface molecule other than an antigen receptor or its ligands that is required for an efficient response of immune cells (eg lymphocytes) to an antigen. A costimulatory molecule can be represented in the following protein families: **TNF** receptor proteins, immunoglobulin-like proteins, cytokine receptors, integrins, lymphocyte activation signaling molecules (SLAM proteins) and NK cell activation receptors. Examples of such molecules include, but are not limited to OX40, ICOS, DAP10, CD27, CD28, CDS, CD30, CD137 (4-1BB), CD40, ICOS, lymphocyte function-associated antigen- 1 (LFA-1), CD2, CD7, LIGHT, NKG2C, GITR, NKG2C, SLAMF7, NKp80, BAFFR, HVEM, BTLA, ICAM-1, LFA-1 (CD11a/CD18), B7- H3, and a ligand that specifically binds with CD83, and the like. For example, CD27 co- stimulation has been demonstrated to enhance expansion, effector function, and survival of human CAR T cells in vitro and augments human T cell persistence and anti-tumour activity in vivo (Song et al. Blood. 2012; 1 19(3):696-706).

In another embodiment, the at least one intracellular signaling domain comprises the intracellular domain of the costimulatory molecules selected from OX40, CD70, CD27, CD28, CD5, ICAM-1, LFA-1 (CD11a/CD18), ICOS (CD278), DAP10, DAP 12, and 4-1BB (CD137), or any combination thereof.

In an embodiment, the T-cell signaling domain appears forming part of the fusion protein with the first polypeptide of the antigen recognizing construct. In an embodiment, the T-cell signaling domain appears forming part of the fusion protein with the second polypeptide of the antigen recognizing construct. In another embodiment, the T-cell signaling domain appears forming part of fusions proteins with the first and second polypeptides of the antigen recognizing construct.

In another embodiment, the TCR-CAR of the invention further comprises a hinge domain between the sTCR part and the transmembrane domain.

As used herein, "hinge domain", "hinge region" or "spacer" refers to an amino acid region that allows for separation and flexibility of the binding moiety and the T cell membrane. The length of the flexible hinges also allows for better binding to relatively inaccessible epitopes, e.g., longer hinge domains are allowed for optimal binding. One skilled in the art will be able to determine the appropriate hinge for the given TCR-CAR target.

The hinge domain can have a length of from about 10 amino acids to about 200 amino acids, preferably, between 50 and 150 amino acids, more preferably between 75 and 125 amino acids. In some cases, the hinge domain is a domain region polypeptide derived from a receptor (e.g., a CD8-derived hinge domain). Exemplary hinge domains include glycine polymers (G) n, glycine-serine polymers (including, for example, (GS)n, (GSGGS)n and (GGGS)n, where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains. Exemplary spacers can comprise amino acid sequences including, but not limited to, GGSG, GGSGG, GSGSG, GSGGG, GGGSG, GSSSG and the like.

In some cases, the hinge domain in the TCR-CAR of the invention includes at least one cysteine. For example, in some cases, the hinge domain can include the sequence Cys-Pro-Pro-Cys.

In some cases, the hinge domain is an immunoglobulin heavy chain hinge domain. Immunoglobulin hinge domain amino acid sequences are known in the art; see, e.g., Tan et al. (1990) Proc. Natl. Acad. Sci. USA 87:162; and Huck et al. (1986) Nucl. Acids Res. 14:1779. As non-limiting examples, an immunoglobulin hinge domain can include one of the following amino acid sequences: DKTHT; CPPC; CPEPKSCDTPPPCPR; ELKTPLGDTTHT; KSCDKTHTCP; KCCVDCP; KYGPPCP; EPKSCDKTHTCPPCP (human IgG1 hinge); ERKCCVECPPCP (human IgG2 hinge); ELKTPLGDTTHTCPRCP (human IgG3 hinge); SPNMVPHAHHAQ (human IgG4 hinge); and the like.

The hinge domain can comprise an amino acid sequence of a human IgG1, IgG2, IgG3, or IgG4, hinge domain. The hinge domain can include one or more amino acid substitutions and/or insertions and/or deletions compared to a wild-type (naturally-occurring) hinge domain. For example, His 229 of human IgG1 hinge can be substituted with Tyr, so that the hinge domain comprises the sequence EPKSCDKTYTCPPCP (SEQ ID NO: 152); see, e.g., Yan et al. (2012) J. Biol. Chem. 287:5891).

### Nucleic acid and nucleic acids compositions

In a third aspect, the present invention relates to a nucleic acid encoding the antigen recognizing construct according to the previous aspect of the invention.

As used interchangeably herein, the terms "nucleic acid", "polynucleotide" and "nucleotide sequence" relate to any polymeric form of nucleotides of any length and composed of ribonucleotides or deoxyribonucleotides. The terms include both single-stranded and double-stranded polynucleotides, as well as modified polynucleotides (e.g., methylated, protected). Typically, the nucleic acid is a "coding sequence" which, as used herein, refers to a DNA sequence that is transcribed and translated into a polypeptide in a host cell when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A transcription termination sequence will usually be located 3' to the coding sequence.

In an embodiment, the nucleic acid construct according to the invention, wherein the nucleic acid construct is selected from the group consisting of:
(i) a nucleic acid construct comprising a first region encoding the alpha chain of a TCR and a second region encoding the beta chain of said TCR,
(ii) a nucleic acid construct comprising a first region encoding a fusion protein comprising the alpha chain of a TCR and a second polypeptide and a second region encoding the beta chain of said TCR,
(iii) a nucleic acid construct comprising a first region encoding the alpha chain of a TCR and a second region encoding a fusion protein comprising the beta chain of said TCR and a second polypeptide,
(iv) a nucleic acid construct comprising a first region encoding the variable region of the alpha chain of a TCR and a second region encoding the variable region of the beta chain of said TCR, optionally wherein said first and second region form a single open reading frame,
(v) a nucleic acid construct comprising a first region encoding a TCR alpha chain extracellular region sequence and a second region encoding the TCR beta chain extracellular region sequence, optionally wherein said first and second region form a single open reading frame.

The term "nucleic acid construct", as used herein, refers to a DNA or RNA molecule that comprises a polynucleotide sequence which encodes a target protein and which may include initiation and termination signals operably linked to regulatory elements including a promoter and polyadenylation signal capable of directing expression in the cells of a subject.

In some embodiments, the nucleic acid encoding the antigen recognizing constructs of the invention or the different polypeptides that form the antigen recognizing construct is RNA, e.g., in vitro synthesized RNA. Methods for in vitro synthesis of RNA are known in the art; any known method can be used to synthesize RNA comprising a nucleotide sequence encoding the antigen recognizing constructs of the invention or the different polypeptides that form the antigen recognizing constructs of the invention. Methods for introducing RNA into a host cell are known in the art. Introducing RNA comprising a nucleotide sequence encoding the first and/or the second polypeptide of a heterodimeric, conditionally active CAR of the present disclosure into a host cell can be carried out in vitro or ex vivo or in vivo. For example, a host cell (e.g., an NK cell, a cytotoxic T lymphocyte, etc.) can be electroporated in vitro or ex vivo with RNA comprising a nucleotide sequence encoding the first and/or the second polypeptide of a heterodimeric, conditionally active CAR of the present disclosure.

In an embodiment, the different nucleic acids encoding the antigen-recognizing constructs of the invention further comprise sequences encoding signal sequences that are translated in the same open reading frame as the different polypeptides encoded by the nucleic acids. This results in that the polypeptides encoded by the nucleic acids are produced as precursor forms that are processed intracellularly in the secretory pathway resulting in the cleavage of the signal sequence in the mature polypeptide.

Thus, in preferred embodiments:
- If a nucleic acid construct comprises a first region encoding a fusion protein comprising the alpha chain of a TCR and a second polypeptide and a second region encoding the beta chain of said TCR, the nucleic acid further comprises a first additional sequence encoding a first signal sequence and a second additional sequence encoding a second signal sequence, wherein the first signal sequence is found in the same open reading frame as the alpha chain of the TCR and the second signal sequence is found in the same open reading frame as the beta chain of the TCR,
- If the nucleic acid construct comprising a first region encoding a fusion protein comprising the alpha chain of a TCR and a second polypeptide and a second region encoding the beta chain of said TCR, the nucleic acid further comprises a first additional sequence encoding a first signal sequence and a second additional sequence encoding a second signal sequence, wherein the first signal sequence is found in the same open reading frame as the fusion protein comprising the alpha chain of a TCR and a second polypeptide and the second signal sequence is found in the same open reading frame as the beta chain of the TCR,
- If the nucleic acid construct comprises a first region encoding the alpha chain of a TCR and a second region encoding a fusion protein comprising the beta chain of said TCR and a second polypeptide, the nucleic acid further comprises a first additional sequence encoding a first signal sequence and a second additional sequence encoding a second signal sequence, wherein the first signal sequence is found in the same open reading frame as the alpha chain of a TCR and the second signal sequence is found in the same open reading frame as the fusion protein comprising the beta chain of a TCR and a second polypeptide the beta chain of the TCR,
- If the nucleic acid construct comprises a first region encoding the variable region of the alpha chain of the TCR and a second region encoding the variable region of the beta chain of said TCR, the nucleic acid further comprises a first additional sequence encoding a first signal sequence and a second additional sequence encoding a second signal sequence, wherein the first signal sequence is found in the same open reading frame as the alpha chain variable region and the second signal sequence is found in the same open reading frame as the beta chain variable region,
- If the nucleic acid construct comprises a first region encoding the variable region of the alpha chain of the TCR and a second region encoding the variable region of the beta chain of said TCR wherein both regions form a fusion protein, the nucleic acid further comprises an additional sequence encoding a signal sequence which is found in the same open reading frame as the fusion protein,
- If the nucleic acid construct comprises a first region encoding the extracellular region of the alpha chain of the TCR and a second region encoding the extracellular region of the beta chain of said TCR wherein both regions form a fusion protein, the nucleic acid further comprises an additional sequence encoding a signal sequence which is found in the same open reading frame as the fusion protein,
- If the nucleic acid construct comprises a first region encoding a TCR alpha chain extracellular region sequence and a second region encoding the TCR beta chain extracellular region sequence, the nucleic acid further comprises a first additional sequence encoding a first signal sequence and a second additional sequence encoding a second signal sequence, wherein the first signal sequence is found in the same open reading frame as the TCR alpha chain extracellular region sequence and the second signal sequence is found in the same open reading frame as the TCR beta chain extracellular region sequence.
- If the nucleic acid construct comprises a first region encoding a TCR alpha chain extracellular region sequence and a second region encoding the TCR beta chain extracellular region sequence wherein both regions form a fusion protein, the nucleic acid further comprises an additional sequence encoding a signal sequence which is found in the same open reading frame as the fusion protein.

The term "signal sequence", also known as "leader peptide", is used herein according to its ordinary meaning in the art and refers to a peptide having a length of about 5-30 amino acids. A leader peptide is present at the N-terminus of newly synthesized proteins that form part of the secretory pathway. Proteins of the secretory pathway include, but are not limited to proteins that reside either inside certain organelles (the endoplasmic reticulum, Golgi or endosomes), are secreted from the cell, or are inserted into a cellular membrane. In some embodiments, the leader peptide forms part of the transmembrane domain of a protein.

In a particular embodiment the signal which is encoded by the nucleic acids according to the invention is selected form the group comprising the CD8 signal sequence and the IgK signal sequence.

It will be evident to the expert in the field that the nucleic acid construct of the invention, which comprises two regions encoding each a different polypeptide (e.g, the alpha and beta chains of the TCR or the alpha and beta chain variable domains) allows two separate polypeptides to be obtained. Several mechanisms are available to produce two or more polypeptides from the same nucleic acid construct. One such way is the existence in said nucleic acid of different transcription promoting elements, e.g., promoters and enhancers, for each of the different coding regions (see aspects and embodiments further below). Another way is the existence of single promoting elements which originate a unique messenger RNA (mRNA) which encodes for both polypeptides. In said case the nucleic acid can be monocistronic, i.e., both regions are contained in the same open reading frame of the mRNA, or bicistronic wherein the mRNA contains two different opening reading frames, one encoding for the first polypeptide and the other encoding for the second polypeptide.

In a particular embodiment the nucleic acid construct of the invention is a monocistronic construct wherein the region encoding the first polypeptide and the region encoding the second polypeptide are found in the same open-reading frame and are separated by a self-cleaving peptide region.

As used herein, the term "self-cleaving peptide region" means a peptide sequence having a cleavage activity that occurs between two amino acid residues in the peptide sequence itself. Examples of the self-cleaving peptide include a 2A peptide and a 2A-like peptide. For example, in 2A or 2A-like peptides, cleavage occurs between glycine and proline residues on these peptides. This is caused by the "ribosome skipping mechanism", which prevents the formation of normal peptide bonds between glycine residues and proline residues during translation, and does not affect downstream translation. The ribosome skip mechanism is known in the art and is used for the expression of multiple proteins encoded by a single molecule of mRNA. The self-cleaving peptide used in the present invention can be obtained from a viral 2A peptide or a 2A-like peptide having an equivalent function. Examples of 2A self-cleaving peptides include 2A peptides (F2A) derived from foot-and-mouth disease virus (FMDV) (VKQTLNFDLLKLAGDVESNPGP, 2A peptides derived from horse rhinitis A virus (ERAV), 2A peptide (P2A) derived from Porcine teschovirus (PTV-1), and 2A peptides (T2A) derived from Thosea signa virus (TaV) 64)). Mutations may be appropriately introduced into the self-cleaving peptide domain as long as its activity is not significantly impaired.

In a particular embodiment the nucleic acid construct of the invention is a monocistronic construct wherein the region encoding the first polypeptide and the region encoding the second polypeptide are found in the same open-reading frame and are separated by a cis acting hydrolase element.

A "cis-acting hydrolase element" or "CHYSEL" refers to a peptide sequence that causes a ribosome to release the growing polypeptide chain that it is being synthesized without dissociation from the mRNA. In this respect, the ribosome continues translating and therefore produces a second polypeptide. Peptides such as the FMDV 2A sequence (GSGSRVTELL YRM KRAETYC PRPLLAIHPTEARHKQKIVAPVKQLLNFDLLKLAGDVESNPGP), Sponge (Amphimedon queenslandica) 2A sequence (LLCFLLLLLSGDVELNPGP or HHFMFLLLLLAGDIELNPGP); acorn worm (Saccoglossus kowalevskii) (WFLVLLSFILSGDIEVNPGP) 2A sequence; amphioxus (Branchiostoma floridae) (KNCAMYMLLLSGDVETNPGP or MVISQLMLKLAGDVEENPGP) 2A sequence Porcine teschovirus-1 (GSGATNFSLLKQAGDVEENPGP) 2A sequence; Thoseaa signa virus (GSGEGRGSLLTCGDVEENPGP) 2A sequence; and Equine rhinitis A virus (GSGQCTNYALLKLAGDVESNPGP) 2A sequence are CHYSELs of use in this invention. In some embodiments, the 2A sequence is a naturally occurring or synthetic sequence that includes the 2A consensus sequence D-X-E-X-NPGP (SEQ ID NO: 166), in which X is any amino acid residue.

In a particular embodiment the nucleic acid construct of the invention is a multicistronic construct wherein the first region and the second region are found in different open-reading frames and are separated by an internal ribosome entry site or a cis acting hydrolase element.

The term "internal ribosome entry site" or "IRES" defines a sequence motif that promotes attachment of ribosomes to that motif on internal mRNA sequences. Consequently, an mRNA containing an IRES sequence motif results in two translational products, one initiating from the 5'-end of the mRNA and the other by an internal translation mechanism mediated by the IRES. A number of IRES have been described and can be used in the nucleic acid construct of this invention. See, e.g., US 8,192,984; WO 2010/119257; and US 2005/0112095.

### Expression vector

In a fourth aspect, the present invention relates to an expression vector comprising a nucleic acid according to the third aspect of the invention.

An "expression vector" as used herein refers to a nucleic acid molecule capable of replication and expressing a gene of interest when transformed, transfected or transduced into a host cell. The expression vectors comprise one or more phenotypic selectable markers and an origin of replication to ensure maintenance of the vector and to, if desired, provide amplification within the host. Selectable markers include, for example, sequences conferring antibiotic resistance markers, which may be used to obtain successful transformants by selection, such as ampicillin, tetracycline and kanamycin resistance sequences, or supply critical nutrients not available from complex media. Suitable expression vectors may be plasmids derived, for example, from pBR322 or various pUC plasmids, which are commercially available. Other expression vectors may be derived from bacteriophage, phagemid, or cosmid expression vectors, all of which are described in sections 1.12-1.20 of Sambrook et al., (Molecular Cloning: A Laboratory Manual. 3rd edn., 2001, Cold Spring Harbor Laboratory Press). Isolated plasmids and DNA fragments are cleaved, tailored, and ligated together in a specific order to generate the desired vectors, as is well known in the art (see, for example, Sambrook et al., ibis).

Large numbers of suitable vectors and promoters are known to those of skill in the art; many are commercially available for generating a subject recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

Expression vectors generally have convenient restriction sites located near the promoter sequence to provide for the insertion of nucleic acid sequences encoding heterologous proteins. A selectable marker operative in the expression host may be present. Suitable expression vectors include, but are not limited to, viral vectors (e.g. viral vectors based on vaccinia virus; poliovirus; adenovirus; adeno-associated virus; SV40; herpes simplex virus; human immunodeficiency virus; a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus); and the like.

The term "promoter", as used herein, refers to a DNA sequence that determines the site of transcription initiation for an RNA polymerase. Promoter sequences comprise motifs which are recognized and bound by polypeptides, i.e. transcription factors. The said transcription factors shall upon binding recruit RNA polymerases II, preferably, RNA polymerase I, II or III, more preferably, RNA polymerase II or III, and most preferably, RNA polymerase II. Thereby will be initiated the expression of a nucleic acid operatively linked to the transcription control sequence. It is to be understood that dependent on the type of nucleic acid to be expressed, expression as meant herein may comprise transcription of DNA sequences into RNA polynucleotides (as suitable for, e.g., antisense approaches, RNAi approaches or ribozyme approaches) or may comprise transcription of DNA sequences into RNA polynucleotides followed by translation of the said RNA polynucleotides into polypeptides (as suitable for, e.g., gene expression and recombinant polypeptide production approaches). In order to govern expression of a nucleic acid sequence, the transcription control sequence may be located immediately adjacent to the nucleic acid to be expressed, i.e. physically linked to the said nucleic acid at its 5' end. Alternatively, it may be located in physical proximity. In the latter case, however, the sequence must be located so as to allow functional interaction with the nucleic acid to be expressed.

In order to assess the expression of the nucleic acid construct according to the invention, the expression vector to be introduced into a cell can also contain either a selectable marker gene or a reporter gene or both to facilitate identification and selection of expressing cells from the population of cells sought to be transfected or infected through viral vectors; in other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic -resistance genes, such as neo and the like. Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Examples of promoters/regulatory sequences suitable for the present invention are, without limitation human cytomegalovirus promoter (CMV) (Hosseini Rad et al, PLoS One, 2020, 15(7): e0232915), Chinese Hamster Elongation Factor-1α (CHEF-1) (Ebadat, et al, PLoS One, 2017, 12(10):e018596), Human elongation factor-1 alpha (EF-1) (Hosseini Rad et al, PLoS One, 2020, 15(7): e0232915), Human Phosphoglycerate Kinase (hPGK) (Hosseini Rad et al, PLoS One, 2020, 15(7): e0232915), RPBSA (Hosseini Rad et al, PLoS One, 2020, 15(7): e0232915), MND promoter (WO2018085690),murine stem cell virus (MSCV) long terminal repeat (LTR) (Hughes, M. et al., Hum Gene Ther. 2005 Apr; 16(4): 457-472) and the NFAT-minimal IL2 promoter (Hoojberg et al., Blood, 2000 Jul; 96(2): 459-466.

A vector preferably contains one or more restriction sites and may be capable of autonomous replication in a defined host cell including a target cell or tissue or a progenitor cell or tissue thereof, or be partially or entirely integrable with the genome of the defined host such that the cloned sequence is reproducible. Accordingly, the vector may be an autonomously replicating vector, i.e. a vector that exists as an extrachromosomal entity, the replication of which is independent of chromosomal replication, e.g. a linear or closed circular plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome/s into which it has been integrated. The choice of the vector will typically depend on the compatibility of the vector with the host cell into which the vector is to be introduced.

The vector may further comprise one or more nucleic acid sequences encoding selectable marker such as auxotrophic markers (e.g., LEU2 , URA3, TRP 1 or HIS3) , detectable labels such as fluorescent or luminescent proteins (e.g., GFP, eGFP, DsRed, CFP) , or protein conferring resistance to a chemical /toxic compound (e.g., MGMT gene conferring resistance to temozolomide) . These markers can be used to select or detect host cells comprising the vector and can be easily chosen by the skilled person according to the host cell.

### Nucleic acid compositions

In a fifth aspect, the present invention relates to a nucleic acid composition encoding the antigen recognizing construct according to the first and second aspect of the invention selected from the group consisting of:
(i) a composition comprising a first nucleic acid encoding the alpha chain of a TCR and a second nucleic acid encoding the beta chain of said TCR,
(ii) a composition comprising a first nucleic acid encoding a fusion protein comprising the alpha chain of a TCR and a second nucleic acid encoding the beta chain of said TCR,
(iii) a composition comprising a first nucleic acid encoding the alpha chain of a TCR and a second nucleic acid encoding a fusion protein comprising the beta chain of said TCR and a second polypeptide.

As used herein, the term "nucleic acid composition" refers to any composition comprising nucleic acids. The nucleic acids within the nucleic acid composition may be organized into chromosomes, wherein the proteins (i.e., for example, histones) may become associated with the chromosomes having a regulatory function.

In a particular embodiment, the nucleic acid composition according to the invention, wherein the first and/or the second nucleic acids are provided in an expression vector.

All the terms and embodiments previously described are equally applicable to this aspect of the invention.

### Cells

In a sixth aspect, the present invention relates to a cell comprising an antigen recognizing construct according to the first and second aspect of the invention, a nucleic acid construct according to the third aspect of the invention, a vector according to the fourth aspect of the invention or the nucleic acid composition according to the fifth aspect of the invention.

The term "cell" refers to "host cell" and means any cell of any organism that is modified, transformed, or manipulated by addition or modification of a gene, a DNA or a RNA sequence, or a protein or a polypeptide. It also refers to the progeny of such cells. Host cells or genetically engineered cells of the present invention include isolated immune cells.

As used herein, "immune cell" refers to a cell that plays a role in the immune response. Immune cells are of hematopoietic origin, and include lymphocytes, such as B cells and T cells; natural killer cells; myeloid cells, such as monocytes, macrophages, eosinophils, mast cells, basophils, and granulocytes. In some embodiments, the cell is a T cell; a NK cell; a NKT cell; lymphocytes, such as B cells and T cells; and myeloid cells, such as monocytes, macrophages, eosinophils, mast cells, basophils, and granulocytes.

In a particular embodiment of the method of the invention the immune cell is a T cell or a natural killer (NK) cell.

As used herein, the term "T cell" refers to a type of lymphocyte that matures in the thymus. T cells play an important role in cell-mediated immunity and are distinguished from other lymphocytes such as B lymphocytes by the presence of T cell receptors on the cell surface. T cells can also be isolated or obtained from commercially available sources. T cells are of any type expressing CD3, including helper T cells (CD4+ cells), cytotoxic T cells (CD8+ cells), natural killer T cells, regulatory T cells (Tregs) and gamma-delta T cells. "Cytotoxic cells" include CD8+ T cells, natural-killer (NK) cells, and neutrophils capable of mediating a cytotoxic response. The terms "T cell" and "T lymphocyte" are interchangeable and are used interchangeably herein.

Natural killer cells or "NK cells" are well known in the art. In one embodiment, natural killer cells include cell lines, such as NK- 92 cells. Further examples of NK cell lines include NKG, YT, NK-YS, HANK-1, YTS cells, and NKL cells. NK cells can be detected by specific surface markers, such as CD16, CD56, and CD8 in humans. NK cells do not express T-cell antigen receptors, the pan T marker CD3, or surface immunoglobulin B cell receptors.

Natural killer T (NKT) cells are a heterogeneous group of T cells that share properties of both T cells and natural killer cells. Thus, NKT cells are a subset of T cells that coexpress an αβ T-cell receptor, but also express a variety of molecular markers that are typically associated with NK cells, such as NK1. Many of these cells recognize the non-polymorphic CD1d molecule, an antigen-presenting molecule that binds self and foreign lipids and glycolipids. They constitute only approximately 0.1% of all peripheral blood T cells. Natural killer T cells should not be confused with natural killer cells.

In another embodiment, the cell according to the invention is a human cell.

All the terms and embodiments previously described are equally applicable to this aspect of the invention.

### Pharmaceutical composition

In a seventh aspect, the present invention relates to a pharmaceutical composition comprising an antigen recognizing construct according to the first and second aspect of the invention, a nucleic acid construct according to the third aspect of the invention, a vector according to the fourth aspect of the invention or the nucleic acid composition according to the fifth aspect of the invention, together with one or more pharmaceutically acceptable carriers or excipients.

As used herein, the term "pharmaceutical composition" refers to a form that allows the biological activity of the active ingredient contained to be effective and has an acceptable toxicity for the subject to which the composition is administered.

The term "pharmaceutically effective amount", as used herein, relates to the sufficient amount of a compound (i.e. of the combination of the invention) to provide the desired effect and it will generally be determined, by among other causes, the characteristics of the compound itself and the therapeutic effect to be achieved. It will also depend on the subject to be treated, the severity of the disease suffered by said subject, the chosen dosage form, administration route, etc.

The term "excipient" refers to a substance that aids the absorption of any of the components or compounds of the pharmaceutical composition of the invention, or stabilises the components or compounds and/or aids the preparation of the pharmaceutical composition in the sense of giving it consistency or flavours to make it more palatable. Thus, excipients may have the function, by way of example, but not limited to, binding the components (e.g. starches, sugars or cellulose), sweetening, colouring, protecting the active substance (e.g. to insulate it from air and/or moisture), filling a pill, capsule or any other presentation or a disintegrating function to facilitate the dissolution of the components, not excluding other excipients not listed in this paragraph. The term 'excipient' is therefore defined as a material which, included in the dosage forms, is added to the active substances or their associations to enable their preparation and stability, to modify their organoleptic properties or to determine the physical and chemical properties of the pharmaceutical composition and their bioavailability.

The expression "pharmaceutically acceptable excipient", as used herein, includes any and all solvents, dispersion media, coatings, antibacterial and antifungal agents, isotonic and absorption delaying agents that are physiologically compatible with the composition of the invention.

All the terms and embodiments previously described are equally applicable to this aspect of the invention.

### In vitro method of detecting cancer cells

In an eight aspect, the present invention relates to an *in vitro* method of detecting cancer in a biological sample comprising:
(a) contacting the biological sample with the antigen recognizing construct of the invention, and
(b) detecting binding of the antigen recognizing construct to the biological sample which has a cancer that express a tumoral antigen which comprises the antigenic peptide recognized by the antigen recognizing construct when the antigenic peptide is linked with a HLA.

In particular embodiments, such detection and/or determination methods can be practiced as a method of diagnosis, such as a method of diagnosis whether a mammalian subject (such as a human subject or patient) has a disease, disorder or condition (such as one described above), in particular a proliferative disorder such as a cancer or tumour (or has a risk of developing such a disease, disorder or condition) that is associated with the expression of the antigen which is recognized by the antigen recognition constructs of the invention

In certain embodiments, the biological sample is one obtained from a mammalian subject like a human patient. The term "biological sample" is used in its broadest sense and can refer to a bodily sample obtained from the subject (e.g., a human patient). For example, the biological sample can include a clinical sample, i.e., a sample derived from a subject. Such samples can include, but are not limited to: peripheral bodily fluids, which may or may not contain cells, e.g., blood, urine, plasma, mucous, bile pancreatic juice, supernatant fluid, and serum; tissue or fine needle biopsy samples; tumour biopsy samples or sections (or cells thereof), and archival samples with known diagnosis, treatment and/or outcome history. Biological samples may also include sections of tissues, such as frozen sections taken for histological purposes. The term "biological sample" can also encompass any material derived by processing the sample. Derived materials can include, but are not limited to, cells (or their progeny) isolated from the biological sample, nucleic acids and/or proteins extracted from the sample. Processing of the biological sample may involve one or more of, filtration, distillation, extraction, amplification, concentration, fixation, inactivation of interfering components, addition of reagents, and the like.

In some embodiments of these detection, determination and/or diagnosis methods, the biological sample is a tissue sample from the subject, such as a sample of a tumour or a cancer from the subject. Such a sample may contain tumour cells and/or blood cells (eg monocytes and T cells). As described above, such tissue sample may be a biopsy sample of the tumour or a cancer such as a needle biopsy samples, or a tumour biopsy sections or an archival sample thereof. Such a tissue sample may comprise living, dead or fixed cells, such as from the tumour or a cancer, and such cells may be suspected of expressing (e.g. aberrantly or localised) the applicable biomarker to be determined.

In a particular embodiment, the term antigen recognizing construct refers to the conjugates in which the antigen recognizing construct is coupled to a detectable target or label as explained above.

Marker detection methods include, among others, fluorescence, light, confocal and electron microscopy, magnetic resonance and spectroscopy; fluoroscopy, computerized tomography and positron emission tomography, enzyme-linked immunosorbent assays, size exclusion chromatography by fluorescence detection, lateral flow test, radioimmunoassay, radiolabeling, Western blot, immunohistochemistry, immunofluorescence, immunocytochemistry, flow cytometry, fluorescence-activated cell sorting, immunoprecipitation, and enzyme-type immunospot.

As used herein, the term "cancer" or "tumor" or "tumor disease" refers to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. As used herein, the term cancer includes, but is not limited to breast cancer, ovarian cancer, pancreatic cancer, prostate cancer, lung cancer, colorectal cancer, stomach/gastric cancer, endometrial/uterine/cervical cancer, bladder cancer, head and neck cancer, leukemia, cancer of the heart, of the small intestine, spleen, kidney, brain, skin, bone, bone marrow, blood, thymus, womb, testicles, hepatobiliary system and liver, sarcoma, cholangiocarcinoma, glioblastoma, multiple myeloma, lymphoma, adenoma, angiosarcoma, astrocytoma, epithelial carcinoma, germinoma, glioma, hemangioendothelioma, hemangiosarcoma, hematoma, hepatoblastoma, medulloblastoma, melanoma, neuroblastoma, hepatobiliary cancer, osteosarcoma, retinoblastoma, rhabdomyosarcoma, sarcoma and teratoma. Furthermore, this term includes acrolentiginous melanoma, actinic keratosis adenocarcinoma, adenoid cystic carcinoma, adenomas, adenosarcoma, adenosquamus carcinoma, astrocytic tumors, Bartholin gland carcinoma, basal cell carcinoma, bronchial gland carcinoma, capillary carcinoid, carcinoma, carcinosarcoma, cystadenoma, endodermal sinus tumor, endometrial hyperplasia, endometrial stromal sarcoma, endometrioid adenocarcinoma, ependymal sarcoma, Ewing sarcoma, focal nodular hyperplasia, germ cell tumors, glucagonoma, hemangioblastoma, hemagioendothelioma, hemagioma, hepatic adenoma, hepatic adenomastosis, hepatocellular carcinoma, hepatobiliary cancer, insulinoma, intraepithelial neoplasia, squamous cell intraepithelial neoplasia, invasive squamous-cell carcinoma, large cell carcinoma, leiomyosarcoma, melanoma, malignant melonoma, malignant mesothelial tumor, medulloblastoma, medulloepithelioma, mucoepidermoid carcinoma, neuroblastoma, neuroepithelial adenocarcinoma, nodular melanoma, osteosarcoma, papillary serous adenocarcinoma, pituitary tumors, plasmacytoma, pseudosarcoma, pulmonary blastoma, renal cell carcinoma, retinoblastoma, rhabdomyosarcoma, serous carcinoma, microcytic carcinoma, soft tissue carcinoma, somatostatin secreting tumor, squamous carcinoma, squamous cell carcinoma, undifferentiated carcinoma, uveal melanoma, verrucous carcinoma, vipoma, Wilm tumor, intracerebral cancer, rectal cancer, astrocytoma, microcytic cancer and non-microcytic cancer, metastatic melanoma, androgen-independent metastatic prostate cancer, androgen-dependent metastatic prostate cancer. The term cancer includes both primary tumors as well as metastatic tumors.

The term "sample" or "biological sample", as used herein, refers to biological material isolated from a subject. The biological sample contains any biological material suitable for detecting cancer. In a particular embodiment, the sample is either a tumor sample or a biofluid. The sample can be isolated from any suitable tissue or biological fluid such as, for example blood, saliva, plasma, serum, urine, cerebrospinal liquid (CSF), feces, a surgical specimen, a specimen obtained from a biopsy, and a tissue sample embedded in paraffin. Methods for isolating samples are well known to those skilled in the art.

All the terms and embodiments previously described are equally applicable to this aspect of the invention.

### Medical uses of the composition of the invention in the treatment of cancer

In a ninth aspect, the present invention relates to the antigen recognizing construct according to the first and second aspect of the invention, the nucleic acid construct according to the third aspect of the invention, the vector according to the fourth aspect of the invention, the nucleic acid composition according to the fifth aspect of the invention or the cell according to the sixth aspect of the invention for use in medicine.

In a preferred embodiment, the construct according to the invention selected for use in medicine comprise a therapeutic agent as defined previously in the first aspect of the invention.

In a tenth aspect, the present invention relates to the antigen recognizing construct according to the first and second aspect of the invention, the nucleic acid construct according to the third aspect of the invention, the vector according to the fourth aspect of the invention, the nucleic acid composition according to the fifth aspect of the invention or the cell according to the sixth aspect of the invention for use in the treatment of cancer.

As used herein, the terms "therapy", "treat", "treatment", or "amelioration" are used interchangeably. The term refers to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (i.e, not aggravated) condition of the disease. These include, but are not limited to, delayed or slowed progression, amelioration or alleviation of the disease state, and remission (partial or total). The term "treatment" of a disease also includes providing relief from symptoms or side effects of the disease (including symptomatic treatment).

In a particular embodiment, the cancer express a tumoral antigen which comprises the peptide recognized by the antigen recognizing construct when the antigen is provided as a complex with a HLA.

In a particular embodiment, the cancer may be: acute myeloid leukemia cancer, adenoid cystic carcinoma cancer, adrenal gland cancer, B cell lymphoma cancer, biliary tract cancer, bladder/urinary tract cancer, blood cancer, bone cancer, bowel cancer, breast cancer, cervical cancer, cholangiocarcinoma, CNS/brain cancer, colon cancer, endometrial cancer, esophageal cancer, glioblastoma, glioma, head and neck cancer, kideny renal clear cell carcinoma, kidney renal papillary cell carcinoma, kidney cancer, kidney chromophoe cancer, liver cancer, lung cancer, lung adenocarcinoma cancer, lung squamous cell carcinoma cancer, lymph cancer, melanoma cancer, ovarian cancer, fallopian tube cancer, pancreas cancer, pheocrhomocytoma and paraganglioma, prostate cancer, rectum cancer, sarcoma cancer, skin cancer, soft tissue cancer, stomach cancer, testicular cancer, thymoma, thyroid cancer, synovial sarcoma or uterine cancer.

In some embodiment, the antigen recognizing construct is used in the treatment of cancer characterized by the expression in the cancer cells of an antigen having the sequence FFDETRQL, preferably when the antigen is presented in the form of a complex with HLA C*04:01. In some embodiments, the antigen recognizing construct contains a CDR3 alpha having the sequence of SEQ ID NO: 5 and a CDR3 beta having the sequence of SEQ ID NO: 8.

In some embodiments, where the antigen recognizing construct of the invention specifically binds to a peptide from the TP53_ PIK3CA_R88Q, protein preferably the peptide having the sequence FFDETRQL, the antigen recognizing construct is used for the treatment of the cancer selected from the group consisting of: biliary tract cancer, bladder/urinary tract cancer, bowel cancer, breast cancer, CNS/brain cancer, esophagus/stomach cancer, head and neck cancer, lung cancer, ovary/fallopian tube cancer, prostate cancer or uterus cancer.

In some embodiment, the antigen recognizing construct is used in the treatment of cancer characterized by the expression in the cancer cells of an antigen having the sequence SCMGGMNQR, preferably when the antigen is presented in the form of a complex with HLA A*11:01. In some embodiments, the antigen recognizing construct contains a CDR3 alpha having the sequence of SEQ ID NO: 17 and a CDR3 beta having the sequence of SEQ ID NO: 20.

In some embodiments, where the antigen recognizing construct of the invention specifically binds to a peptide from TP53_R248Q, preferably the peptide having the sequence SCMGGMNQR, the antigen recognizing construct is used for the treatment of the cancer selected from the group consisting of: bladder/urinary tract, blood cancer, bone cancer, bowel cancer, breast cancer, CNS/brain cancer, esophagus/stomach cancer, head and neck cancer, kidney cancer, liver cancer, lung cancer, lymph cancer, ovary/fallopian tube cancer, pancreas cancer, prostate cancer, skin cancer, soft tissue cancer or uterus cancer.

In some embodiment, the antigen recognizing construct is used in the treatment of cancer characterized by the expression in the cancer cells of an antigen having the sequence STDLPILLK, preferably when the antigen is presented in the form of a complex with HLA A11*01. In some embodiments, the antigen recognizing construct contains a CDR3 alpha having the sequence of SEQ ID NO: 29 and a CDR3 beta having the sequence of SEQ ID NO: 32.

In some embodiments, where the antigen recognizing construct of the invention specifically binds to a peptide from C5orf22, preferably the peptide having the sequence STDLPILLK, the antigen recognizing construct is used for the treatment of the cancer selected from the group consisting of: adenoid cystic carcinoma, bladder cancer, breast cancer, cervical cancer, cholangiocarcinoma cancer, colon cancer, b cell lymphoma cancer, esophageal cancer, glioblastoma cancer, head and neck cancer, kidney chromophoe cancer, kidney renal clear cell carcinoma, kidney renal papillary cell carcinoma, acute myeloid leukemia, glioma, liver cancer, lung adenocarcinoma, lung squamous cell carcinoma, ovarian cancer, pheocrhomocytoma and paraganglioma, prostate cancer, rectum cancer, sarcoma, melanoma, stomach cancer, testicular cancer, thyroid cancer, thymoma, endometrial cancer or uterine cancer.

In particular embodiment, the antigen recognizing construct is used in the treatment of cancer characterized by the expression in the cancer cells of an antigen having the sequence SSLPLASGVFKK, preferably when the antigen is presented in the form of a complex with HLA A11*01. In some embodiments, the antigen recognizing construct contains a CDR3 alpha having the sequence of SEQ ID NO: 41 and a CDR3 beta having the sequence of SEQ ID NO: 44.

In more particular embodiment, where the antigen recognizing construct of the invention specifically binds to a peptide from HOXC13, preferably the peptide having the sequence SSLPLASGVFKK, the antigen recognizing construct is used for the treatment of the cancer selected from the group consisting of: bladder cancer, breast cancer, cervical cancer, esophageal cancer, head and neck cancer, lung squamous cell carcinoma or melanoma.

In some embodiment, the antigen recognizing construct is used in the treatment of cancer characterized by the expression in the cancer cells of an antigen having the sequence RVAEITGIVK, preferably when the antigen is presented in the form of a complex with HLA A68*01. In some embodiments, the antigen recognizing construct contains a CDR3 alpha having the sequence of SEQ ID NO: 53 and a CDR3 beta having the sequence of SEQ ID NO: 56.

In some embodiments, where the antigen recognizing construct of the invention specifically binds to a peptide from ZKSCAN1, preferably the peptide having the sequence RVAEITGIVK, the antigen recognizing construct is used for the treatment of the cancer selected from the group consisting of: adenoid cystic carcinoma, bladder cancer, breast cancer, cervical cancer, cholangiocarcinoma cancer, colon cancer, b cell lymphoma cancer, esophageal cancer, glioblastoma cancer, head and neck cancer, kidney chromophoe cancer, kidney renal clear cell carcinoma, kidney renal papillary cell carcinoma, acute myeloid leukemia, glioma, liver cancer, lung adenocarcinoma, lung squamous cell carcinoma, ovarian cancer, pheocrhomocytoma and paraganglioma, prostate cancer, rectum cancer, sarcoma, melanoma, stomach cancer, testicular cancer, thyroid cancer, thymoma, endometrial cancer or uterine cancer.

In some embodiment, the antigen recognizing construct is used in the treatment of cancer characterized by the expression in the cancer cells of an antigen having the sequence MEVDPIGHLY. In some embodiments, the antigen recognizing construct contains a CDR3 alpha having the sequence of SEQ ID NO: 65 and a CDR3 beta having the sequence of SEQ ID NO: 68.

In some embodiments, where the antigen recognizing construct of the invention specifically binds to a peptide from MAGE-A3, preferably the peptide having the sequence MEVDPIGHLY, the antigen recognizing construct is used for the treatment of the cancer selected from the group consisting of: breast cancer, cervical cancer, colon cancer, esophageal cancer, head and neck cancer, liver cancer, lung cancer, ovarian cancer, sarcoma cancer, melanoma cancer, stomach cancer, endometrial cancer, bladder cancer, pancreatic cancer or uterine cancer.

In some embodiment, the antigen recognizing construct is used in the treatment of cancer characterized by the expression in the cancer cells of an antigen having the sequence KRCFPVIFGK, preferably when the antigen is presented in the form of a complex with HLA B*27:05. In some embodiments, the antigen recognizing construct contains a CDR3 alpha having the sequence of SEQ ID NO: 89 and a CDR3 beta having the sequence of SEQ ID NO: 92.

In some embodiments, where the antigen recognizing construct of the invention specifically binds to a peptide from MAGE A4_p142-156, preferably the peptide having the sequence KRCFPVIFGK, the antigen recognizing construct is used for the treatment of the cancer selected from the group consisting of: synovial sarcoma cancer, myxoid round cell cancer, liposarcoma cancer, endometrial cancer, gastric cancer, esophageal cancer, head and neck cancer, cervical cancer, prostate cancer, colorectal cancer, lung cancer or ovarian cancer.

In some embodiment, the antigen recognizing construct is used in the treatment of cancer characterized by the expression in the cancer cells of an antigen having the sequence KRCFPVIFGK, preferably when the antigen is presented in the form of a complex with HLA B*27:05. In some embodiments, the antigen recognizing construct contains a CDR3 alpha having the sequence of SEQ ID NO: 101 and a CDR3 beta having the sequence of SEQ ID NO: 104.

In some embodiments, where the antigen recognizing construct of the invention specifically binds to a peptide from MAGE A4_p142-156, preferably the peptide having the sequence KRCFPVIFGK, the antigen recognizing construct is used for the treatment of the cancer selected from the group consisting of: synovial sarcoma cancer, myxoid round cell cancer, liposarcoma cancer, endometrial cancer, gastric cancer, esophageal cancer, head and neck cancer, cervical cancer, prostate cancer, colorectal cancer, lung cancer or ovarian cancer.

In some embodiment, the antigen recognizing construct is used in the treatment of cancer characterized by the expression in the cancer cells of an antigen, preferably when the antigen is presented in the form of a complex with HLA B* 51:01. In some embodiments, the antigen recognizing construct contains a CDR3 alpha having the sequence of SEQ ID NO: 125 and a CDR3 beta having the sequence of SEQ ID NO: 128.

In some embodiments, where the antigen recognizing construct of the invention specifically binds to a peptide from MAGE A4, preferably the peptide being presented in the form of a complex with HLA B* 51:01, the antigen recognizing construct is used for the treatment of the cancer selected from the group consisting of: synovial sarcoma cancer, myxoid round cell cancer, liposarcoma cancer, endometrial cancer, gastric cancer, esophageal cancer, head and neck cancer, cervical cancer, prostate cancer, colorectal cancer, lung cancer or ovarian cancer.

In some embodiment, the antigen recognizing construct is used in the treatment of cancer characterized by the expression in the cancer cells of an antigen having the sequence STRDPLSEITK, preferably when the antigen is presented in the form of a complex with HLA A*11:01. In some embodiments, the antigen recognizing construct contains a CDR3 alpha having the sequence of SEQ ID NO: 113 and a CDR3 beta having the sequence of SEQ ID NO: 116.

In some embodiments, where the antigen recognizing construct of the invention specifically binds to a peptide from TP53_ PIK3CA_E545K, preferably the peptide having the sequence STRDPLSEITK, the antigen recognizing construct is used for the treatment of the cancer selected from the group consisting of: biliary tract cancer, bladder/urinary tract cancer, bowel cancer, breast cancer, cervix cancer, CNS/brain cancer, esophagus/stomach cancer, head and neck cancer, kidney cancer, liver cancer, lung cancer, ovary/fallopian tube cancer, pancreas cancer, prostate cancer, skin cancer, testis cancer, thyroid cancer or uterus cancer.

All the terms and embodiments previously described are equally applicable to this aspect of the invention.

### Further aspects of the invention

1. An antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein:
   (a) the antigenic peptide comprises the amino acid sequence FFDETRQL (SEQ ID NO: 1) and the HLA molecule is HLA C*04:01; or
   (b) the antigenic peptide comprises the amino acid sequence SCMGGMNQR ( SEQ ID NO: 2) and the HLA molecule is HLA A*11:01.
2. The antigen recognizing construct according to aspect 1, wherein:
   (a) a TCR alpha chain variable domain comprising a complementarity determining region 3 (CDR3) comprising SEQ ID NO: 5 or functionally equivalent variant thereof and a TCR beta chain variable domain comprising a complementarity determining region 3 (CDR3) comprising SEQ ID NO: 8 or a functionally equivalent variant thereof; or
   (b) a TCR alpha chain variable domain comprising a CDR3 comprising SEQ ID NO: 17 or a functionally equivalent variant thereof and a TCR beta chain variable domain comprising a CDR3 comprising SEQ ID NO: 20 or a functionally equivalent variant thereof.
3. The antigen recognizing construct according to aspect 2, wherein:
   (a) the TCR alpha chain variable domains CDR1, CDR2 and CDR3 comprise, respectively, SEQ ID NO: 3, 4 and 5 or functionally equivalent variants thereof and the TCR beta chain variable domains CDR1, CDR2 and CDR3 comprise respectively, SEQ ID NO:6, 7 and 8 or functionally equivalent variants thereof; or
   (b) the TCR alpha chain variable domains CDR1, CDR2 and CDR3 comprise, respectively, SEQ ID NO:15, 16 and 17 or functionally equivalent variants thereof and the TCR beta chain variable domains CDR1, CDR2 and CDR3 comprise respectively, SEQ ID NO:18, 19 and 20 or functionally equivalent variants thereof.
4. The antigen recognizing construct according to aspect 3, wherein the TCR comprises:
   (a) an alpha chain variable region having the amino acid sequence of SEQ ID NO: 9 and a beta chain variable region having the amino acid sequence of SEQ ID NO: 10; or
   (b) an alpha chain variable region having the amino acid sequence of SEQ ID NO: 21 and a beta chain variable region having the amino acid sequence of SEQ ID NO: 22.
5. The antigen recognizing construct according to aspect 4, wherein the TCR comprises:
   (a) an alpha chain extracellular region having the amino acid sequence of SEQ ID NO: 11 and a beta chain extracellular region having the amino acid sequence of SEQ ID NO: 12 or
   (b) an alpha chain extracellular region having the amino acid sequence of SEQ ID NO: 23 and a beta chain extracellular region having the amino acid sequence of SEQ ID NO: 24.
6. The antigen recognizing construct according to aspect 5, wherein the TCR comprises:
   (a) an alpha chain having the amino acid sequence of SEQ ID NO: 13 and a beta chain having the amino acid sequence of SEQ ID NO: 14; or
   (b) an alpha chain having the amino acid sequence of SEQ ID NO: 25 and a beta chain having the amino acid sequence of SEQ ID NO: 26.
7. An antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein the antigen recognizing construct comprises:
   (a) a TCR alpha chain variable domain comprising a CDR3 comprising SEQ ID NO: 5 or functionally equivalent variant thereof and a TCR beta chain variable domain comprising a CDR3 comprising SEQ ID NO: 8 or a functionally equivalent variant thereof; or
   (b) a TCR alpha chain variable domain comprising a CDR3 comprising SEQ ID NO: 17 or functionally equivalent variant thereof and a TCR beta chain variable domain comprising a CDR3 comprising SEQ ID NO: 20 or a functionally equivalent variant thereof; or
   (c) a TCR alpha chain variable domain comprising a CDR3 comprising SEQ ID NO: 29 or functionally equivalent variant thereof and a TCR beta chain variable domain comprising a CDR3 comprising SEQ ID NO: 32 or a functionally equivalent variant thereof; or
   (d) a TCR alpha chain variable domain comprising a CDR3 comprising SEQ ID NO: 41 or a functionally equivalent variant thereof and a TCR beta chain variable domain comprising a CDR3 comprising SEQ ID NO: 44 or a functionally equivalent variant thereof; or
   (e) a TCR alpha chain variable domain comprising a CDR3 comprising SEQ ID NO: 53 or a functionally equivalent variant thereof and a TCR beta chain variable domain comprising a CDR3 comprising SEQ ID NO: 56 or a functionally equivalent variant thereof; or
   (f) a TCR alpha chain variable domain comprising a CDR3 comprising SEQ ID NO: 65 or a functionally equivalent variant thereof and a TCR beta chain variable domain comprising a CDR3 comprising SEQ ID NO: 68 or a functionally equivalent variant thereof; or
   (g) a TCR alpha chain variable domain comprising a CDR3 comprising SEQ ID NO: 77 or a functionally equivalent variant thereof and a TCR beta chain variable domain comprising a CDR3 comprising SEQ ID NO: 80 or a functionally equivalent variant thereof; or
   (h) a TCR alpha chain variable domain comprising a CDR3 comprising SEQ ID NO: 89 or a functionally equivalent variant thereof and a TCR beta chain variable domain comprising a CDR3 comprising SEQ ID NO: 92 or a functionally equivalent variant thereof; or
   (i) a TCR alpha chain variable domain comprising a CDR3 comprising SEQ ID NO: 101 or a functionally equivalent variant thereof and a TCR beta chain variable domain comprising a CDR3 comprising SEQ ID NO: 104 or a functionally equivalent variant thereof; or
   (j) a TCR alpha chain variable domain comprising a CDR3 comprising SEQ ID NO: 113 or a functionally equivalent variant thereof and a TCR beta chain variable domain comprising a CDR3 comprising SEQ ID NO: 116 or a functionally equivalent variant thereof; or
   (k) a TCR alpha chain variable domain comprising a CDR3 comprising SEQ ID NO: 125 or a functionally equivalent variant thereof and a TCR beta chain variable domain comprising a CDR3 comprising SEQ ID NO: 128 or a functionally equivalent variant thereof.
8. The antigen recognizing construct according to aspect 6, wherein:
   (a) The TCR alpha chain variable domains CDR1, CDR2 and CDR3 comprise, respectively, SEQ ID NO: 3, 4 and 5 or functionally equivalent variants thereof and the TCR beta chain variable domains CDR1, CDR2 and CDR3 comprise respectively, SEQ ID NO: 6, 7 and 8 or functionally equivalent variants thereof; or
   (b) The TCR alpha chain variable domains CDR1, CDR2 and CDR3 comprise, respectively, SEQ ID NO: 15, 16 and 17 or functionally equivalent variants thereof and the TCR beta chain variable domains CDR1, CDR2 and CDR3 comprise respectively, SEQ ID NO: 18, 19 and 20 or functionally equivalent variants thereof; or
   (c) The TCR alpha chain variable domains CDR1, CDR2 and CDR3 comprise, respectively, SEQ ID NO: 27, 28 and 29 or functionally equivalent variants thereof and the TCR beta chain variable domains CDR1, CDR2 and CDR3 comprise respectively, SEQ ID NO: 30, 31 and 32 or functionally equivalent variants thereof; or
   (d) the TCR alpha chain variable domains CDR1, CDR2 and CDR3alpha comprise, respectively, SEQ ID NO: 39, 40 and 41 or functionally equivalent variants thereof and the TCR beta chain variable domains CDR1, CDR2 and CDR3 comprise respectively, SEQ ID NO: 42, 43 and 44 or functionally equivalent variants thereof; or
   (e) the TCR alpha chain variable domains CDR1, CDR2 and CDR3 comprise, respectively, SEQ ID NO: 51, 52 and 53 or functionally equivalent variants thereof and the TCR beta chain variable domains CDR1, CDR2 and CDR3 comprise respectively, SEQ ID NO: 54, 55 and 56 or functionally equivalent variants thereof; or
   (f) the TCR alpha chain variable domains CDR1, CDR2 and CDR3 comprise, respectively, SEQ ID NO: 63, 64 and 65 or functionally equivalent variants thereof and the TCR beta chain variable domains CDR1, CDR2 and CDR3 comprise respectively, SEQ ID NO: 66, 67 and 68 or functionally equivalent variants thereof; or
   (g) the TCR alpha chain variable domains CDR1, CDR2 and CDR3 comprise, respectively, SEQ ID NO: 75, 76 and 77 or functionally equivalent variants thereof and the TCR beta chain variable domains CDR1, CDR2 and CDR3 comprise respectively, SEQ ID NO: 78, 79 and 80 or functionally equivalent variants thereof; or
   (h) the TCR alpha chain variable domains CDR1, CDR2 and CDR3 comprise, respectively, SEQ ID NO: 87, 88 and 89 or functionally equivalent variants thereof and the TCR beta chain variable domains CDR1, CDR2 and CDR3 comprise respectively, SEQ ID NO: 90, 91 and 92 or functionally equivalent variants thereof; or
   (i) the TCR alpha chain variable domains CDR1, CDR2 and CDR3 comprise, respectively, SEQ ID NO: 99, 100 and 101 or functionally equivalent variants thereof and the TCR beta chain variable domains CDR1, CDR2 and CDR3 comprise respectively, SEQ ID NO: 102, 103 and 104 or functionally equivalent variants thereof; or
   (j) the TCR alpha chain variable domains CDR1, CDR2 and CDR3 comprise, respectively, SEQ ID NO: 111, 112 and 113 or functionally equivalent variants thereof and the TCR beta chain variable domains CDR1, CDR2 and CDR3 comprise respectively, SEQ ID NO: 114, 115 and 116 or functionally equivalent variants thereof; or
   (k) the TCR alpha chain variable domains CDR1, CDR2 and CDR3 comprise, respectively, SEQ ID NO: 123, 124 and 125 or functionally equivalent variants thereof and the TCR beta chain variable domains CDR1, CDR2 and CDR3 comprise respectively, SEQ ID NO: 126, 127 and 128 or functionally equivalent variants thereof.
9. The antigen recognizing construct according to aspect 7, wherein the TCR comprises:
   (a) an alpha chain variable region having the amino acid sequence of SEQ ID NO: 9 and a beta chain variable region having the amino acid sequence of SEQ ID NO: 10; or
   (b) an alpha chain variable region having the amino acid sequence of SEQ ID NO: 21 and a beta chain variable region having the amino acid sequence of SEQ ID NO: 22; or
   (c) an alpha chain variable region having the amino acid sequence of SEQ ID NO: 33 and a beta chain variable region having the amino acid sequence of SEQ ID NO: 34; or
   (d) an alpha chain variable region having the amino acid sequence of SEQ ID NO: 45 and a beta chain variable region having the amino acid sequence of SEQ ID NO: 46; or
   (e) an alpha chain variable region having the amino acid sequence of SEQ ID NO: 57 and a beta chain variable region having the amino acid sequence of SEQ ID NO: 58; or
   (f) an alpha chain variable region having the amino acid sequence of SEQ ID NO: 69 and a beta chain variable region having the amino acid sequence of SEQ ID NO: 70; or
   (g) an alpha chain variable region having the amino acid sequence of SEQ ID NO: 81 and a beta chain variable region having the amino acid sequence of SEQ ID NO: 82; or
   (h) an alpha chain variable region having the amino acid sequence of SEQ ID NO: 93 and a beta chain variable region having the amino acid sequence of SEQ ID NO: 94; or
   (i) an alpha chain variable region having the amino acid sequence of SEQ ID NO: 105 and a beta chain variable region having the amino acid sequence of SEQ ID NO: 106; or
   (j) an alpha chain variable region having the amino acid sequence of SEQ ID NO: 117 and a beta chain variable region having the amino acid sequence of SEQ ID NO: 118; or
   (k) an alpha chain variable region having the amino acid sequence of SEQ ID NO: 129 and a beta chain variable region having the amino acid sequence of SEQ ID NO: 130.
10. The antigen recognizing construct according to aspect 7, wherein the TCR comprises:
   (a) an alpha chain extracellular region having the amino acid sequence of SEQ ID NO: 11 and a beta chain extracellular region having the amino acid sequence of SEQ ID NO: 12; or
   (b) an alpha chain extracellular region having the amino acid sequence of SEQ ID NO: 23 and a beta chain extracellular region having the amino acid sequence of SEQ ID NO: 24; or
   (c) an alpha chain extracellular region having the amino acid sequence of SEQ ID NO: 35 and a beta chain extracellular region having the amino acid sequence of SEQ ID NO: 36; or
   (d) an alpha chain extracellular region having the amino acid sequence of SEQ ID NO: 47 and a beta chain extracellular region having the amino acid sequence of SEQ ID NO: 48; or
   (e) an alpha chain extracellular region having the amino acid sequence of SEQ ID NO: 59 and a beta chain extracellular region having the amino acid sequence of SEQ ID NO: 60; or
   (f) an alpha chain extracellular region having the amino acid sequence of SEQ ID NO: 71 and a beta chain extracellular region having the amino acid sequence of SEQ ID NO: 72; or
   (g) an alpha chain extracellular region having the amino acid sequence of SEQ ID NO: 83 and a beta chain extracellular region having the amino acid sequence of SEQ ID NO: 84; or
   (h) an alpha chain extracellular region having the amino acid sequence of SEQ ID NO: 95 and a beta chain extracellular region having the amino acid sequence of SEQ ID NO: 96; or
   (i) an alpha chain extracellular region having the amino acid sequence of SEQ ID NO: 107 and a beta chain extracellular region having the amino acid sequence of SEQ ID NO: 108; or
   (j) an alpha chain extracellular region having the amino acid sequence of SEQ ID NO: 119 and a beta chain extracellular region having the amino acid sequence of SEQ ID NO: 120; or
   (k) an alpha chain extracellular region having the amino acid sequence of SEQ ID NO: 131 and a beta chain extracellular region having the amino acid sequence of SEQ ID NO: 132.
11. The antigen recognizing construct according to aspect 8, wherein the TCR comprises:
   (a) an alpha chain having the amino acid sequence of SEQ ID NO: 13 and a beta chain having the amino acid sequence of SEQ ID NO: 14; or
   (b) an alpha chain having the amino acid sequence of SEQ ID NO: 25 and a beta chain having the amino acid sequence of SEQ ID NO: 26; or
   (c) an alpha chain having the amino acid sequence of SEQ ID NO: 37 and a beta chain having the amino acid sequence of SEQ ID NO: 38; or
   (d) an alpha chain having the amino acid sequence of SEQ ID NO: 49 and a beta chain having the amino acid sequence of SEQ ID NO: 50; or
   (e) an alpha chain having the amino acid sequence of SEQ ID NO: 61 and a beta chain having the amino acid sequence of SEQ ID NO: 62; or
   (f) an alpha chain having the amino acid sequence of SEQ ID NO: 73 and a beta chain having the amino acid sequence of SEQ ID NO: 74; or
   (g) an alpha chain having the amino acid sequence of SEQ ID NO: 85 and a beta chain having the amino acid sequence of SEQ ID NO: 86; or
   (h) an alpha chain having the amino acid sequence of SEQ ID NO: 97 and a beta chain having the amino acid sequence of SEQ ID NO: 98; or
   (i) an alpha chain having the amino acid sequence of SEQ ID NO: 109 and a beta chain having the amino acid sequence of SEQ ID NO: 110; or
   (j) an alpha chain having the amino acid sequence of SEQ ID NO: 121 and a beta chain having the amino acid sequence of SEQ ID NO: 122.
   (k) an alpha chain having the amino acid sequence of SEQ ID NO: 133 and a beta chain having the amino acid sequence of SEQ ID NO: 134.
12. The antigen recognizing construct according to any of aspects 1 to 11 wherein the antigen recognizing construct is a TCR.
13. The antigen recognizing construct according to any of aspects 1 to 11 which is a soluble TCR wherein the soluble TCR is
   (i) a fusion protein comprising the TCR alpha chain variable region and the TCR beta chain variable region,
   (ii) a fusion protein comprising a first region comprising the TCR alpha chain extracellular region and a second region comprising the TCR beta chain extracellular sequence,
   (iii) A first polypeptide comprising the TCR alpha chain variable region and a second polypeptide comprising the TCR beta chain variable region wherein the first and second polypeptides are connected by a disulfide bond or
   (iv) A first polypeptide comprising the TCR alpha chain extracellular region and a second polypeptide comprising the TCR beta chain extracellular region wherein the first and second are connected by a disulfide bond
14. The antigen recognizing construct according to aspect 13 wherein the soluble TCR is conjugated to an agent of interest.
15. The antigen recognizing construct according to aspect 14 wherein the agent of interest is a therapeutic or diagnostic agent.
16. The antigen recognizing construct according to aspect 13(iii) or 13(iv) wherein the first and/or the second polypeptide forms part of a fusion protein which comprises an heterologous polypeptide region.
17. The antigen recognizing construct according to aspect 16 wherein the heterologous polypeptide region is selected from the group consisting of an T-cell engaging immune effector polypeptide, a half-life extending domain and a T-cell signaling domain.
18. The antigen recognizing construct according to aspect 15 wherein
   (a) the T-cell engaging immune effector polypeptide is a CD3 effector domain,
   (b) the half-life extending domain is an immunoglobulin Fc domain or an albumin binding domain and
   (c) the signaling domain is a transmembrane and cytosolic domain from a T-cell receptor.
19. The antigen recognizing construct according to aspects 1 to 16 wherein the construct comprises a TCR alpha chain polypeptide and a TCR beta chain polypeptide and wherein at least the TCR alpha chain polypeptide or the TCR beta chain polypeptide further comprise a second polypeptide region.
20. A nucleic acid encoding the antigen recognizing construct according to any one of aspects 1 to 19.
21. The nucleic acid construct according to aspect 20 wherein the nucleic acid construct is selected from the group consisting of:
   (i) a nucleic acid construct comprising a first region encoding the alpha chain of a TCR and a second region encoding the beta chain of said TCR,
   (ii) a nucleic acid construct comprising a first region encoding a fusion protein comprising the alpha chain of a TCR and a second polypeptide and a second region encoding the beta chain of said TCR,
   (iii) a nucleic acid construct comprising a first region encoding the alpha chain of a TCR and a second region encoding a fusion protein comprising the beta chain of said TCR and a second polypeptide,
   (iv) a nucleic acid construct comprising a first region encoding the variable region of the alpha chain of a TCR and a second region encoding the variable region of the beta chain of said TCR, optionally wherein said first and second region form a single open reading frame,
   (v) a nucleic acid construct comprising a first region encoding a TCR alpha chain extracellular region sequence and a second region encoding the TCR beta chain extracellular region sequence, optionally wherein said first and second region form a single open reading frame.
22. The nucleic acid construct according to aspect 21 wherein each region encoding a polypeptide further comprises a sequence encoding a signal sequence which is located in the same open reading frame as the polypeptide encoded by the region
23. An expression vector comprising a nucleic acid according to aspects 21 or 22.
24. A nucleic acid composition encoding the antigen recognizing construct according to any one of aspects 1 to 19 selected from the group consisting of:
   (i) a composition comprising a first nucleic acid encoding the alpha chain of a TCR and a second nucleic acid encoding the beta chain of said TCR,
   (ii) a composition comprising a first nucleic acid encoding a fusion protein comprising the alpha chain of a TCR and a second nucleic acid encoding the beta chain of said TCR,
   (iii) a composition comprising a first nucleic acid encoding the alpha chain of a TCR and a second nucleic acid encoding a fusion protein comprising the beta chain of said TCR and a second polypeptide.
25. The nucleic acid composition according to aspects 23 or 24 wherein the first and/or the second nucleic acids are provided in an expression vector.
26. A cell comprising an antigen recognizing construct of any one of aspect 1 to 19, a nucleic acid construct according to any of aspects 20 to 22, a vector according to aspect 23 or the nucleic acid composition according to any of aspects 23 to 25.
27. The cell according to aspect 26, wherein the cell is a human cell.
28. A pharmaceutical composition comprising an antigen recognizing construct of any one of aspect 1 to 19, a nucleic acid construct according to any of aspects 20 to 22, a vector according to aspect 23 or the nucleic acid composition according to aspects 24 or 25, together with one or more pharmaceutically acceptable carriers or excipients.
29. An in vitro method of detecting cancer cells in a biological sample comprising:
   (a) contacting the biological sample with the antigen recognizing construct of any of aspects 1 to 19, and
   (b) detecting binding of the antigen recognizing construct to cells in the biological sample wherein the cancer cells express a tumoral antigen which comprises the antigenic peptide recognized by the antigen recognizing construct when the antigenic peptide is linked with a HLA.
30. The antigen recognizing construct of any one of aspect 1 to 19, the nucleic acid construct according to any of aspects 20 to 22, a vector according to aspect 23, the nucleic acid composition according to aspects 24 or 25 or the cell according to aspects 26 or 27, for use in medicine.
31. The antigen recognizing construct of any one of aspect 1 to 19, the nucleic acid construct according to any of aspects 20 to 22, a vector according to aspect 23, the nucleic acid composition according to aspects 24 or 25 or the cell according to aspects 26 or 27, for use in the treatment of cancer.
32. The antigen recognizing construct, nucleic acid and/or cell for use according to aspect 31, wherein the cancer express a tumoral antigen which comprises the peptide recognized by the antigen recognizing construct when the antigen is linked with a HLA.

The invention is described below by means of the following examples which are to be construed as merely illustrative and not limitative of the scope of the invention.

### EXAMPLES

### Materials and methods

### Cloning, in vitro transcription of RNA, and electroporation

The HLA sequences of interest, the full-length cancer germline antigen MAGE A4 were cloned into pcDNA3.1 using BamH1 and EcoR1. HLA-I sequences were obtained from IPD-IMGT/HLA and codon-optimized. Alternatively, the full-length sequence for the oncogenic drivers PIK3CA and TP53 (both mutated and Wt versions) were cloned into pcDNA3.1+v2, which included a P2A and ZsGreen sequences, with Hindlll and BamHI. The sequences of the full-length proteins were obtained from NCBI and were manually mutated to introduce the desired mutated aminoacid.

The plasmids were linearized with Not-I followed by phenol-chloroform extraction and precipitation with sodium acetate and ethanol. Next, 1µg of DNA was used as a template to generate RNA by IVT using HiScribe# T7 ARCA mRNA Kit with tailing (New England) following manufacturer's instructions. RNA was precipitated using LiCl2, resuspended at 1µg/ul in molecular grade H2O, and stored at -80° until use.

Tumor cell lines, healthy human cells, and B cells were harvested and resuspended in 100ul of Opti-MEM media (Gibco) and transferred into a sterile 0.2 cm cuvette (VWR electroporation cuvettes). From 4 to 8 µg of RNA encoding for the sequence of interest were added for electroporation. Cells were electroporated at 150V, 20 ms, and 1 pulse using an ECM 830 BTX-Electroporator. After electroporation, cells were resuspended in pre-warmed specific media containing DNAse (Pulmozyme, Roche). B cells were cultured in 5mL polystyrene tubes. TCL and human healthy cells were cultured in tissue-treated 6 well plates with the corresponding media at 37°C and 5% CO₂. After 20 h, cells were harvested, washed with PBS, and used in co-culture assays.

### Peptide synthesis and peptide-pulsing

The amino acid sequences of the immunogenic peptides were supplied by JPT Peptide Technologies (Berlin, Germany) with HPLC purity. The synthetic peptides were resuspended in DMSO, diluted, and typically pulsed at 1 µg/mL into antigen presenting cells (CD4+ or B cells) or tumor cell lines for 2h. Cells where then washed and used as targets in co-culture assays.

### Co-culture assays: IFN-y enzyme-linked immunospot (ELISPOT) and detection of activation marker 4-1BB by flow cytometry.

Effector T-cell populations of interest (from 2e4 to 5e4 cells) were co-incubated with target cells (from 5e4 to 2e5) and T-cell reactivity was evaluated after 20-24 h by measuring IFN-γ release by ELISPOT and by detection of 4-1BB (CD137) expression on the CD8+ T cells by flow cytometry. T cells were thawed into T-cell medium supplemented with 3,000 IU IL-2 (Proleukin) and DNAse (Pulmozyme, Roche) three to four days before coincubation with target T cells. All co-cultures were performed in the absence of exogenously added cytokines. Cells were stained with CD3-APCH7 (BD, clone SK7, 0.3:40), CD8-PECy7 (BD, clone RPA-T8, 0.1:40), CD4-PE (BD, RPA-T4, 0.3:40) and CD137-APC (BD, clone 4B4-1, 0.5:40), and in some cases mTRB-FITC (eBiosciences, clone H57-597, 0.2:40) for 30 minutes at 4°C, washed with staining buffer containing PI (1:2000) and acquired in BD FACSLyric^{™}, BD FACSCanto^{™} or BD FACSCelesta^{™}. In parallel, IFN-y secretion was detected using IFN-y capture and detection antibodies (MABtech technologies) assessed by ELISPOT assay following manufacturer instructions. ELISPOT plates were analyzed and counted in ELISPOT reader. For all the assays, plate-bound OKT3 (1 µg/mL; Biolegend) was used as a positive control. Media, and/or autologous APC pulsed with irrelevant peptides were used as negative controls.

### HLA-I restriction with COS7 cells

To determine which HLA alleles presented the immunogenic peptides identified, COS7 cells were transfected with pcDNA3.1 plasmids encoding the individual HLA molecules using Lipofectamine 2000 (Life Technologies). COS7 cells were either co-transduced with the antigen of interest or rested overnight and pulsed with the corresponding peptide for 2h. Cells were then washed and used as targets in co-culture assays.

### TCR sequencing

TCR sequencing was performed following two different strategies. For TCR targeting cryptic and cancer germline antigens, the TCR locus was sequenced by multiplex single-cell RNA sequencing of enriched antigen-specific T-cell populations. The samples were multiplexed using TotalSeq^{™} barcodes. Sequencing was done on an Illumina NS6000 with an S1 flowcell and v1 chemistry. Mapping, quantification, and clonotype definitions were done using cell ranger multi software (version 6.1.1 using the reference vdj_GRCh38_alts_ensembl-5.0.0). Demultiplexing and subsequent analysis was done in R using the packages Seurat (version 4.0.3) and scRepertoire (version 1.3.5); Seurat::HTODemux was run using default parameters to obtain singlets.

For TCR recognizing hotspot neoantigens, cells were stained with TotalSeq-C hashtag antibodies (BioLegend) after FcR blockade with human TruStain FcX (BioLegend) for sample multiplexing. Barcoded cells were separated using a Next GEM chip N on the 10x Chromium X, and libraries were prepared using the Chromium next GEM single cell 5' HT reagent kit v2 (dual index) with feature barcode technology for cell surface protein and immune receptor mapping (10x Genomics). Libraries were then pooled and sequenced on the Illumina NovaSeq 6000 sequencing platform (Illumina) according to the manufacturer's instructions for paired-end 150 bp (PE150) reads using v1.0 reagent kits (Novogene). Mapping, quantification, and clonotype definitions were performed using Cell Ranger multi-pipeline version 7.1.0 with reference vdj-GRCh38-alts-ensembl-7.0.0. Demultiplexing and downstream analyses were performed in R using Seurat version 5.0.1 and a dedicated pipeline.

In this case, to verify the most abundant TCR clonotypes inferred from the single-cell data from enriched populations targeting hotspot-derived neoantigens, TCR-β deep sequencing was also carried out. Genomic DNA was extracted from the target populations according to the Human TCRB Assay Sample Preparation Guidelines and submitted to the Adaptive Immunosequencing hsTCRB_v4b Service (UltraDeep) (Adaptive Biotechnologies). Processed samples were downloaded from the immunoSEQ analyzer and TCR frequencies were calculated from productive rearrangements in R. The most enriched TCR-β sequences consistently identified by the two sequencing techniques were paired with the corresponding TCR-α sequences according to single-cell RNA sequencing and selected for further studies.

TRA V-J-encoding sequences and TRB V-D-J-encoding sequences were combined to sequences encoding the mouse constant TRA and TRB chains (Cohen et al., 2006), respectively. Mouse constant regions were modified, as previously described (Cohen et al., 2007; Haga-Friedman et al., 2012). The full-length TRB and TRA chains were cloned, in this orientation and separated by a furin SGSG P2A linker, into pMSGV1 retroviral vector (GenScript). The use of mouse TCR constant regions promotes pairing of the introduced TCR and also facilitates the identification of positively transduced human T cells by flow cytometry using an antibody specific for the mouse TCR-β chain.

### PBL transduction

To generate transient retroviral rich supernatants, the retroviral vector MSGV1 encoding the antigen-specific TCR and the envelope encoding plasmid RD114 were co-transfected into the retroviral packaging cell line 293GP (plated the day prior to transfection) using Lipofectamine 2000 (Life Technologies). Retroviral supernatants were collected at 42-48 h after transfection, diluted 1:1 with DMEM media (Gibco), and then centrifuged onto Retronectin-coated (10 µg/ml, Takara), non-tissue culture-treated 24-well plates at 2,000 g for 2 h at 32°C. Donor PBMC activated 2 days before with 50 ng/ml soluble OKT3 in T-cell media, were harvested, added onto the retrovirus plates, and centrifuged for 10 min at 300 g. Activated T cells were transduced overnight, removed from the plates, and further cultured in IL-2 containing T-cell media. Cells were typically assayed 10-14 days post-retroviral transduction.

### Dextramer generation and staining

U-Load Dextramers (Immudex) were loaded independently with an HLA monomer corresponding to one of the patient's HLA-I alleles (HLA-A*11:01, HLA-A*68:01 or HLA-C*04:01) and a peptide of interest at a time. easYmers HLA-I peptide monomers and loading of U-Load dextramers were prepared following manufacturer instructions. Flow cytometry-based quality control assay to monitor peptide loading efficiency was performed at least once for each of the peptide- HLA combinations evaluated.

Custom HLA dextramers conjugated to PE, were used to stain T cells in combination with CD3-APCH7 (BD, clone SK7, 0.3:20), CD8-PECy7 (BD, clone RPA-T8, 0.1:20) and CD4-BV510 (BD, SK3, 0.2:20) for 30 minutes at 4°C, washed with staining buffer containing PI (1:2000) and acquired in BD FACSCanto^{™}.

T-cell recognition was considered positive when the percentage of dextramer-binding cells was double the percentage of cells binding to dextramers loaded with the irrelevant control peptides.

### Rapid expansion protocol (REP)

T cells underwent a rapid expansion protocol (REP) of 14 days using irradiated allogeneic PBMC (50 Gy) pooled from three donors as feeder cells in T-cell medium RPMI 1640 plus (Lonza):AIM-V (Gibco) containing 5% human AB serum (BST), 100 U/mL penicillin (Lonza), 100 µg/mL streptomycin (Lonza), 2mM L-Glutamine (Lonza), 12.5 mM HEPES (Thermo Fisher Scientific) supplemented with 30 ng/ml anti-CD3 (OKT3, Biolegend) and 3000 IU of IL-2 (Proleukin). After day 6, half of the medium was replaced with fresh T-cell medium containing IL-2 every other day. Cells were split when confluent, harvested on day 14, and cryopreserved until use.

### Enrichment of antigen-specific T cells and mTCR transduced cells

To enrich for antigen-specific T cells, PBLs or TILs were FACS sorted either based on the upregulation of the activation marker 4-1BB after coculture with antigen presenting cells expressing the antigen of interest (either peptide-pulsed or RNA electroporated), or by using dextramers loaded with the specific peptides. Same antibodies and dilutions as described for co-cultures (above) or dextramer (above), scaling up to the number of cells were used.

In some instances, PBL transduced with the desired TCRs were FACS sorted based on mTRB-FITC (eBiosciences, clone H57-597, 0.2:40) and CD8-PECy7 (BD, clone RPA-T8, 0.1:40) expression to obtain a pure CD8⁺ TCR transduced population.

Cells were sorted in BD FACS AriaTM or BD Influx^{™} and expanded using a REP as previously specified.

### Healthy human cell lines

Healthy human cell lines were ordered from Promocell. Cells were thawed and cultured following manufacturer's instructions in the recommended media without antibiotics. Cells were split when confluent with Dettaching kit (Promocell). Cells were cultured at the recommended concentration and expanded no more than 4 passages until use. HCM-c (Cat: C-128810) were cultured in myocyte growth medium (Cat n°: C-39275). HREpC-c (CatC-12665) cultured in Renal Epithelial Cell GM media (Cat n°: C-39606). HSAEpC-c (Cat: C-12642) cultured in Small Airway Epithelial cell GM (Cat n°: C-39175). NHEM.f-c (Cat: C-12400) cultured in Melanocyte growth medium (Cat n°: C-39415).

### Generation of autologous B cells

To generate autologous antigen-presenting cells (APC), B cells were isolated from cryopreserved PBMC by positive selection using CD19+ microbeads (Miltenyi Biotec). These primary B cells were expanded through CD40-CD40L stimulation by culturing cells for 4-5 days with irradiated NIH3T3 feeder cells constitutively expressing CD40L at 37°C in 5% CO2 in B cell medium (Iscove's IMDM media (Gibco) containing 10% human AB serum (Biowest), 100 U/mL penicillin (Lonza), 100ug/mL streptomycin (Lonza), 2mM L-Glutamine (Lonza) and supplemented with 200 U/mL IL-4 (Prepotech)). Up to three rounds of stimulation and expansion were performed consecutively. At day 5-6, B cells were either used for generation of snap-frozen pellets for HLA-I immunopeptidomics, used in co-culture experiments or cryopreserved until use. When used after cryopreservation, B cells were thawed in B cell medium containing DNAse (Pulmozyme, Roche) 20h before use in co-culture assays.

### PBMC isolation

Peripheral blood mononuclear cells (PBMC) were separated by centrifugation in a Ficoll density gradient (Lymphoprep, Stem cell) from leukapheresis or from whole blood. Cell aliquots were cryopreserved with FCS/DMSO 10% for later expansion of B cells or in vitro sensitization experiments. Alternatively, PBMCs from healthy donors were used for TCR-transduction.

### Patient samples

Patients were chosen on the basis of the availability of peripheral blood samples and/or tumor biopsy and their HLA haplotype. All patients signed the corresponding informed consent. Peripheral blood samples were used to isolate PBMCs as previously specified, to subsequently isolate and expand B cells or CD4+ and CD8+ T cells. A small fragment (2-4 mm3) of tumor biopsies or surgically resected tumor was used for the establishment of patient-derived tumor cell lines.

Buffy coats from healthy donor (HD) blood were supplied by the Blood and Tissue Banc of Catalonia.

### Results

### Example 1 - Identification of TCRs recognizing different types of tumor antigens

By performing personalized immunological screenings in metastatic cancer patients with different tumor types, we have identified multiple TCRs targeting different tumor antigens. This includes TCRs targeting non-canonical or cryptic antigens, cancer germline antigens and neoantigens derived from hotspot mutations. Table 2 includes a summary of all the TCRs identified, their TCR α and β sequences with CDR3 regions, the specific tumor antigen targeted, and, if determined, their HLA restriction element. In total, we have detected 11 TCRs targeting distinct tumor antigens, all holding potential for the development of off-the-shelf T-cell therapies with TCR-engineered T cells or for the generation of bi-specific T-cell engagers.

**Table 2: Summary of the reactivities, TRAV/TRAJs, sequences of the alpha and beta chains, HLA and target epitope sequences of the identified TCRs**

| **TCR ID** | **Reactivity** | **TRAC** | **TRAV/TRAJ** | **TCR alpha cain VJ AA seq (CDR3 highlighted)** | **TRBC** | **TRBV/TRBJ** | **TCR beta chain VDJ AA seq (CDR3 highlighted)** | **HLA** | **Peptide sequence** |
|---|---|---|---|---|---|---|---|---|---|
| TCR_nc RNA C5orf22 | ncRNA C5orf22 | TRAC, clonotype 6 | TRAV13-1*02/ TRAJ13*01 | TSIRAVFIFLWLQLDLVNGENVEQHPSTLSVQ EGDSAVIKCTYSDSASNYFPWYKQELGKRPQ LIIDIRSNVGEKKDQRIAVTLNKTAKHFSLHITE TQPEDSAVYFCAASPNSGGYQKVTFGIGTKL QVIP | TRBC1 | TRBV11-2*01/ TRBJ1-2*01 | GTRLLCWAALCLLGAELTEAGVAQSPRY KIIEKRQSVAFWCNPISGHATLYWYQQIL GQGPKLLIQFQNNGVVDDSQLPKDRFSA ERLKGVDSTLKIQPAKLEDSAVYLCASS QHLAFTPNYGYTFGSGTRLTVV | A11* 01 | STDLPILLK |
| TCR_5' UTR HOXC1 3 | 5'UTR HOXC13 | TRAC, clonotype 7 | TRAV19*01/ TRAJ37*01 | LTASLLRAVIASICVVSSMAQKVTQAQTEISVV EKEDVTLDCVYETRDTTYYLFWYKQPPSGEL VFLIRRNSFDEQNEISGRYSWNFQKSTSSFNF TITASQVVDSAVYFCALRGGNTGKLIFGQGTT LQVKP | TRBC2 | TRBV13*01/ TRBJ2-5*01 | LSPDLPDSAWNTRLLCRVMLCLLGAGSV AAGVIQSPRHLIKEKRETATLKCYPIPRH DTVYWYQQGPGQDPQFLISFYEKMQSD KGSIPDRFSAQQFSDYHSELNMSSLELG DSALYFCASSPGPGGETQYFGPGTRLLV L | A11* 01 | SSLPLASGVFKK |
| TCR_5' UTR ZKSCA N1 | 5'UTR ZKSCAN1 | TRAC, clonotype 2 | TRAV9-2*01/ TRAJ54*01 | NYSPGLVSLILLLLGRTRGNSVTQMEGPVTLS EEAFLTINCTYTATGYPSLFWYVQYPGEGLQL LLKATKADDKGSNKGFEATYRKETTSFHLEK GSVQVSDSAVYFCALSDLGIQGAQKLVFGQG TRLTINP | TRBC1 | TRBV28*01/ TRBJ1-2*01 | GIRLLCRVAFCFLAVGLVDVKVTQSSRYL VKRTGEKVFLECVQDMDHENMFWYRQ DPGLGLRLIYFSYDVKMKEKGDIPEGYS VSREKKERFSLILESASTNQTSMYLCASS LYEGTYYGYTFGSGTRLTVV | A68* 01 | RVAEITGIVK |
| TCR_M AGE A3_p16 7-176/ MAGEA 6_p.E16 8K | MAGE A3_p167-176/ MAGEA6_ p.E168K | TRAC | TRAV21*01/ TRAJ45*01 | ETLLGLLILWLQLQWVSSKQEVTQIPAALSVPE GENLVLNCSFTDSAIYNLQWFRQDPGKGLTS LLLIQSSQREQTSGRLNASLDKSSGRSTLYIAA SQPGDSATYLCAVRMEGGGADGLTFGKGTH LIIQP | TRBC2 | TRBV6-1*01/ TRBJ2-3*01 | SIGLLCCVAFSLLWASPVNAGVTQTPKF QVLKTGQSMTLQCAQDMNHNSMYWYR QDPGMGLRLIYYSASEGTTDKGEVPNG YNVSRLNKREFSLRLESAAPSQTSVYFC ASSDFRGLGLDTQYFGPGTRLTVL | unk. | MEVDPIGHLY |
| MAGE A4_TCR 1 | MAGE A4 | TRAC | TRAV9-2*01/ TRAJ5*01 | NYSPGLVSLILLLLGRTRGNSVTQMEGPVTLS EEAFLTINCTYTATGYPSLFWYVQYPGEGLQL LLKATKADDKGSNKGFEATYRKETTSFHLEK GSVQVSDSAVYFCALSRTGRRALTFGSGTRL QVQP | TRBC1 | TRBV27*01/ \|TRBJ1-6*01 | GPQLLGYVVLCLLGAGPLEAQVTQNPRY LITVTGKKLTVTCSQNMNHEYMSWYRQ DPGLGLRQIYYSMNVEVTDKGDVPEGYK VSRKEKRNFPLILESPSPNQTSLYFCASS FQGRNSPLHFGNGTRLTVT | unk. | unk. |
| MAGE A4_TCR 2 | MAGE A4_p.145-154 | TRAC | TRAV13-1 *02/ITRAJ53* 01 | TSIRAVFIFLWLQLDLVNGENVEQHPSTLSVQ EGDSAVIKCTYSDSASNYFPWYKQELGKRPQ LIIDIRSNVGEKKDQRIAVTLNKTAKHFSLHITE TQPEDSAVYFCAAHGGGGSNYKLTFGKGTLL TVNP | TRBC1 | TRBV7-8*02/\|TRBJ1 -5*01 | GTRLLCWVVLGFLGTDHTGAGVSQSPR YKVAKRGQDVALRCDPISGHVSLFWYQ QALGQGPEFLTYFQNEAQLDKSGLPSD RFFAERPEGSVSTLKIQRTQKEDSAVYL CASSYSNQPQHFGDGTRLSIL | B*27 :05 | KRCFPVIFGK |
| MAGE A4_TCR 3 | MAGE A4_p.145-154 | TRAC | TRAV29/DV5* 01\|TRAJ43*01 | AMLLGASVLILWLQPDWVNSQQKNDDQQVK QNSPSLSVQEGRISILNCDYTNSMFDYFLWYK KYPAEGPTFLISISSIKDKNEDGRFTVFLNKSA KHLSLHIVPSQPGDSAVYFCAASFGTDMRFG AGTRLTVKP | TRBC2 | TRBV7-9*03/ \|TRBJ2-1*01 | GTSLLCWMALCLLGADHADTGVSQDPR HKITKRGQNVTFRCDPISEHNRLYWYRQ TLGQGPEFLTYFQNEAQLEKSRLLSDRF SAERPKGSFSTLEIQRTEQGDSAMYLCA SSPRTSGAKRSYNEQFFGPGTRLTVL | B*27 :05 | KRCFPVIFGK |
| MAGE A4_TCR 4 | MAGE A4 | TRAC | TRAV1-1*01/\|TRAJ21* 01 | WGAFLLYVSMKMGGTAGQSLEQPSEVTAVE GAIVQINCTYQTSGFYGLSWYQQHDGGAPTF LSYNALDGLEETGRFSSFLSRSDSYGYLLLQE LQMKDSASYFCAVRDGNFNKFYFGSGTKLNV KP | TRBC2 | TRBV30*01/ \|TRBJ2-3*01 | LCSLLALLLGTFFGVRSQTIHQWPATLVQ PVGSPLSLECTVEGTSNPNLYWYRQAA GRGLQLLFYSVGIGQISSEVPQNLSASRP QDRQFILSSKKLLLSDSGFYLCAWTGGG GHQKRDTQYFGPGTRLTVL | B*51 :01 | unk. |
| TCR_PI K3CA p.E545K | PIK3CA p.E545K | TRAC | TRAV25*01 F / TRAJ12*01 F | LLITSMLVLWMQLSQVNGQQVMQIPQYQHVQ EGEDFTTYCNSSTTLSNMQWYKQPGGHPVF LMQLVKSGEVKKQKLTFQFGEAKKNSSLHITA TQTTDVGTYFCAVCMDSSYKLIFGSGTLLVP | TRBC2 | TRBV7-9*03 F/ TRBJ2-1*01 F | GTSLLCWMALCLLGADHADTGVSQDPH KITKGQNVTFCDPISEHNRLYWYRQTLG QGPEFLTYFQNEAQLEKSLLSDRFSAEP KGSFSTLEIQRTEQGDSAMYLCASSLVE GGPGGFGNNEQFFGPGTRLTVL | A*11 :01 | STRDPLSEITK |
| TCR_PI K3CA p. R88Q | PIK3CA p. R88Q | TRAC | TRAV14/DV4* 02F / TRAJ50*01 F | SLSSLLKVVTASLWLGPGIAQKITQTQPGMFV QEKEAVTLDCTYDTSDQSYGLFWYKQPSSGE MIFLIYQGSYDEQNATEGRYSLNFQKARKSAN LVISASQLGDSAMYFCAIVRTSYDKVIFGPGTS LSVIP | TRBC1 | TRBV12-3*01 F/ TRBJ1-1*01 F | DSWTFCCVSLCILVAKHTDAGVIQSPRH EVTEMGQEVTLRCKPISGHNSLFWYRQT MMRGLELLIYFNNNVPIDDSGMPEDRFS AKMPNASFSTLKIQPSEPRDSAVYFCAS SSPDLNTEAFFGQGTRLTVV | C*0 4:01 | FFDETRQL |
| TCR_TP 53 p. R248 Q | TP53 p.R248Q | TRAC | TRAV27*03 F/TRAJ11*01 F | VLKFSVSILWIQLAWVSTQLLEQSPQFLSIQEG ENLTVYCNSSSVFSSLQWYQEPGEGPVLLVT VVTGGEVKKLKLTFQFGDAKDSSLHITAAQTG DTGLYLCAGGEGYSTLTFGKGTMLLVSP | TRBC1 | TRBV7-8*01 F/TRBJ1-2*01 F | GTRLLCWVVLGFLGTDHTGAGVSQSPR YKVAKRGQDVALRCDPISGHVSLFWYQ QALGQGPEFLTYFQNEAQLDKSGLPSD RFFAERPEGSVSTLKIQRTQQEDSAVYL CASSLGYRGRIDYGYTFGSGTRLTVV | A*11 :01 | SCMGGMNQR |

### Example 2 - Identification of TCR recognizing cryptic antigens

Figure 3 shows the in-depth characterization of the specificity and functional traits of the three TCRs (i.e. TCR_ncRNA C5orf22, TCR_5'UTR HOX13 and TCR_5'UTR ZKSCAN), each targeting a different cryptic antigen. These TCRs were isolated from T-cell populations enriched with cryptic antigen-reactive T cells. Two of the recognized cryptic tumor antigens were derived from 5'-UTR regions of the canonical genes HOXC13 (5'U-HOXC13) and ZKSCAN1 (5'U-ZKSCAN1). In contrast, the third cryptic antigen mapped to a noncoding spliced variant of the C5orf22 gene (nc-C5orf22). 5'U-HOXC13 and nc-C5orf22 were recognized in the context of HLA-A*11:01 whilst ZKSCAN1 was recognized in the context of HLA-A*68:01 (Figure 1A). These cryptic antigens were detected in patient-derived tumor cell lines from different tumor origins established in the lab (not shown).

By performing single-cell RNA sequencing of the three T-cell populations enriched with cryptic antigen-reactive T cells, we detected a clear predominant TCR clonotype per population (Figure 1B). Thereafter, the most frequent α/β pairs of each of the populations were cloned into a retroviral vector and were used to transduce peripheral blood lymphocytes (PBL). Each TCR was constructed using the murine TRA and TRB constant region to reduce mispairing of the endogenous TCR and enable us to track the TCR-transduced cells using an anti-mouse TRB antibody. Based on this, all PBL TCR transduced cells were sorted on the basis of CD8 and mTCR expression (CD8+mTCR+). Dextramer staining demonstrated that the three TCRs recognized their corresponding cryptic antigen in the expected HLA context based on (a) (Figure 1C). Moreover, peptide titration experiments to measure the functional avidity of TCR transduced cells evidenced that nc-C5orf22 and 5'U-HOXC13-specific TCRs required higher concentrations of minimal peptide to become activated, while 5'U-ZKSCAN1 TCR transduced cells were more sensitive at detecting their cognate antigen (Figure 1D).

Finally, to gain further insights into the selective expression of these cryptic antigens in tumor cells and their applicability as targets for cancer immunotherapy, we empirically evaluated the expression and translation of the selected immunogenic cryptic antigens in several human healthy cell types by exploiting the ability of TCR transduced cells to detect their cognate peptides with high sensitivity. To this end, the three cryptic antigen-specific CD8+mTCR+ cells were cocultured with human cells of different origins, including normal melanocytes, cardiac myocytes, renal epithelial, and fibroblast, as well as some melanoma cell lines generated in the lab. The expression of the specific HLA alleles restricting antigen recognition of the TCRs evaluated was exogenously enforced in the target cells and T-cell activation was evaluated by measuring 41BB upregulation in mTCR+ cells by flow cytometry following coculture (Figure 1E). These results demonstrated that cryptic antigen-reactive TCR transduced cells were capable of recognizing melanoma cell lines that naturally express the cognate antigen whilst healthy cells were barely recognized. In the case of 5'U-ZKSCAN1 TCR transduced cells (5'U-ZKS. CD8+mTCR+ cells) displayed preferential recognition of melanoma cells compared with normal melanocytes and cardiac myocytes when target cells were electroporated with the relevant allele HLA-A*68:01. Importantly, 5'U-HOXC13 TCR transduced cells (5'U-HOX. CD8+ mTCR+ cells) displayed recognition of two of the four melanoma cell lines included but did not recognize cardiac myocytes and barely recognized normal melanocytes when electroporated with the corresponding allele HLA-A*11:01. The lower sensitivity of the cryptic C5orf22 TCR transduced cells (nc-C5orf. CD8+mTCR+ cells) and/or limited expression of the antigen precluded us from reaching a conclusion regarding the tumor specific expression of this antigen. Overall, these results indicate that the 5'U-HOXC13 peptide is the cryptic antigen with the highest tumor specificity followed by 5'U-ZKSCAN1. Nevertheless, the three TCR recognizing these cryptic antigens would be promising candidates for the development of personalized T-cell engineered T-cell therapies.

### Example 3 - Identification of TCR recognizing cancer germline antigens

A similar strategy was used for the detection of TCRs targeting cancer germline antigens (CGA) (i.e. TCR_MAGEA3_p167-176/MAGEA6_p.E168K, MAGEA4_TCR1, TCR2, TCR3 and TCR4). In this case, the immunological screening of an ovarian cancer patient and a melanoma patient against selected CGAs led to the identification of four and one TCRs targeting the CGAs MAGE-A4 and MAGE-A3, respectively (Figure 2). The four MAGE-A4 TCRs recognized MAGE-A4 when full-length MAGE A4 RNA was electroporated into autologous CD4+ T cells (Figure 2A). By testing different small peptides covering the entire MAGE-A4 sequence, we observed that one of the four TCRs recognized the MAGEA4_{p.109-123} peptide while two of the four TCRs recognized the MAGEA4_{p.142-156} peptide (Figure 2A). Importantly, three out of four TCRs (TCR#2, #3 and #4) recognized the autologous tumor cell line treated with IFN-y (Figure 2A). Therefore, we identified four TCRs capable of recognizing MAGE A4 CGA and three of them were shown to recognize the autologous tumor.

We also performed experiments to identify the HLA restriction element of each of the TCRs. As observed in Figure 2B, TCR#2 and TCR#3 recognized MAGE-A4 in the context of HLA-B*27:05 while TCR#4 recognize MAGE-A4 in the context of HLA-B*51:01. The specific restriction element presenting the peptide recognized by TCR#1 is still unknown.

Similar experiments were performed with T cells transduced with TCR recognizing a peptide from MAGE-A3 CGA. As observed in Figure 2C, TCR-transduced T cells were recognizing the MAGE-A3 peptide and were also able to recognize the autologous tumor cell line. The specific restriction element presenting the peptide recognized by this TCR is still unknown.

### Example 4 - Identification of TCR recognizing neoantigens derived from hotspot mutations

A similar strategy was used to identify TCRs that target neoantigens derived from hotspot mutations presented by highly prevalent HLA-I alleles (i.e. TCR:PIK3CAp.E545K, TCR_PIK3CA_p.R88Q and TCR_TP53p.R248Q), as determined by immunopeptidomics studies conducted on HLA-I monoallelic cells in the laboratory (data not shown). In this instance, by using PBL from a HLA-A*11:01⁺HLA-C*04:01⁺ breast cancer patient, we identified two HLA-A*11:01-restricted T-cell responses specific for PIK3CA_{p.E545K} (Figure 3A) and TP53_{p.R248Q} (Figure 3B), as well as one HLA-C*04:01-restricted response specific for PIK3CA_{p.R88Q} (Figure 3A).

We performed TCR-β deep sequencing (Figure 3C) and single-cell RNA sequencing in the three enriched T-cell populations recognizing hotspot-derived neoantigens to identify the most dominant TCR clonotypes. We tested the top three most dominant TCRs isolated from the PIK3CA_{p.E545K} and TP53_{p.R248Q} specific T-cell populations (data not shown), along with the top five most abundant TCRs from the PIK3CA_{p.R88Q} specific population (Figure 3D). Of note, only one TCR from each enriched T-cell population was reactive against its corresponding neoantigen.

Peptide titration experiments were conducted to evaluate the functional avidity of TCR-transduced cells. The results showed that T cells transduced with the PIK3CA_{pE545K}-specific TCR demonstrated the highest avidity, whereas those with the PIK3CA_{p.R88Q}-specific TCR exhibited the lowest avidity (Figure 3E). Importantly, all three TCRs displayed high specificity for the mutant forms of the neoantigens when compared to their wild-type counterparts (Figure 3E).

Finally, we investigated whether these TCRs could recognize their targets when processed and presented by tumor cells. Since none of our in-house patient-derived tumor cell lines (TCLs) expressed the relevant hotspot mutations along with the corresponding HLA-I alleles, we used the VHIO-029 melanoma patient-derived TCL, which naturally expresses HLA-A*11:01 and HLA-C*04:01. To mimic the expression of the neoantigens, we either pulsed the cells with the mutant peptides or transfected them with RNA encoding the full-length mutant proteins PIK3CA_{p.E54K}, TP53_{p.R248Q}, or PIK3CA_{p.R88Q}. We then assessed T-cell activation by measuring 4-1BB upregulation in mTCR+ cells using flow cytometry after coculture (Figure 3F).

These experiments showed that TCR-transduced T cells, targeting three hotspot-derived neoantigens, successfully recognized VHIO-29 TCL when pulsed or transfected with the corresponding neoantigens (Figure 3F). This supports that these neoepitopes can be generated from the processing of the full-length mutant proteins and presented by endogenous HLA-I alleles on tumor cells. These three TCRs show promise as candidates for the development of off-the-shelf T-cell therapies targeting hotspot-derived neoantigens.

## Claims

1. An antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein:
(a) the antigenic peptide comprises the amino acid sequence FFDETRQL (SEQ ID NO: 1) and the HLA molecule is HLA C*04:01; or
(b) the antigenic peptide comprises the amino acid sequence SCMGGMNQR ( SEQ ID NO: 2) and the HLA molecule is HLA A*11:01.

2. An antigen recognizing construct specifically binding to a complex of an antigenic peptide and a HLA molecule, wherein said antigen recognizing construct is selected from a T cell receptor (TCR) or an antigen-binding fragment thereof, a fusion protein comprising a TCR or an antigen-binding fragment thereof and a single chain TCR wherein the antigen recognizing construct comprises:
(a) a TCR alpha chain variable domain comprising a CDR3 comprising SEQ ID NO: 5 or functionally equivalent variant thereof and a TCR beta chain variable domain comprising a CDR3 comprising SEQ ID NO: 8 or a functionally equivalent variant thereof; or
(b) a TCR alpha chain variable domain comprising a CDR3 comprising SEQ ID NO: 17 or functionally equivalent variant thereof and a TCR beta chain variable domain comprising a CDR3 comprising SEQ ID NO: 20 or a functionally equivalent variant thereof; or
(c) a TCR alpha chain variable domain comprising a CDR3 comprising SEQ ID NO: 29 or functionally equivalent variant thereof and a TCR beta chain variable domain comprising a CDR3 comprising SEQ ID NO: 32 or a functionally equivalent variant thereof; or
(d) a TCR alpha chain variable domain comprising a CDR3 comprising SEQ ID NO: 41 or a functionally equivalent variant thereof and a TCR beta chain variable domain comprising a CDR3 comprising SEQ ID NO: 44 or a functionally equivalent variant thereof; or
(e) a TCR alpha chain variable domain comprising a CDR3 comprising SEQ ID NO: 53 or a functionally equivalent variant thereof and a TCR beta chain variable domain comprising a CDR3 comprising SEQ ID NO: 56 or a functionally equivalent variant thereof; or
(f) a TCR alpha chain variable domain comprising a CDR3 comprising SEQ ID NO: 65 or a functionally equivalent variant thereof and a TCR beta chain variable domain comprising a CDR3 comprising SEQ ID NO: 68 or a functionally equivalent variant thereof; or
(g) a TCR alpha chain variable domain comprising a CDR3 comprising SEQ ID NO: 77 or a functionally equivalent variant thereof and a TCR beta chain variable domain comprising a CDR3 comprising SEQ ID NO: 80 or a functionally equivalent variant thereof; or
(h) a TCR alpha chain variable domain comprising a CDR3 comprising SEQ ID NO: 89 or a functionally equivalent variant thereof and a TCR beta chain variable domain comprising a CDR3 comprising SEQ ID NO: 92 or a functionally equivalent variant thereof; or
(i) a TCR alpha chain variable domain comprising a CDR3 comprising SEQ ID NO: 101 or a functionally equivalent variant thereof and a TCR beta chain variable domain comprising a CDR3 comprising SEQ ID NO: 104 or a functionally equivalent variant thereof; or
(j) a TCR alpha chain variable domain comprising a CDR3 comprising SEQ ID NO: 113 or a functionally equivalent variant thereof and a TCR beta chain variable domain comprising a CDR3 comprising SEQ ID NO: 116 or a functionally equivalent variant thereof; or
(k) a TCR alpha chain variable domain comprising a CDR3 comprising SEQ ID NO: 125 or a functionally equivalent variant thereof and a TCR beta chain variable domain comprising a CDR3 comprising SEQ ID NO: 128 or a functionally equivalent variant thereof.

3. The antigen recognizing construct according to claim 2, wherein:
(a) The TCR alpha chain variable domains CDR1, CDR2 and CDR3 comprise, respectively, SEQ ID NO: 3, 4 and 5 or functionally equivalent variants thereof and the TCR beta chain variable domains CDR1, CDR2 and CDR3 comprise respectively, SEQ ID NO: 6, 7 and 8 or functionally equivalent variants thereof; or
(b) The TCR alpha chain variable domains CDR1, CDR2 and CDR3 comprise, respectively, SEQ ID NO: 15, 16 and 17 or functionally equivalent variants thereof and the TCR beta chain variable domains CDR1, CDR2 and CDR3 comprise respectively, SEQ ID NO: 18, 19 and 20 or functionally equivalent variants thereof; or
(c) The TCR alpha chain variable domains CDR1, CDR2 and CDR3 comprise, respectively, SEQ ID NO: 27, 28 and 29 or functionally equivalent variants thereof and the TCR beta chain variable domains CDR1, CDR2 and CDR3 comprise respectively, SEQ ID NO: 30, 31 and 32 or functionally equivalent variants thereof; or
(d) the TCR alpha chain variable domains CDR1, CDR2 and CDR3alpha comprise, respectively, SEQ ID NO: 39, 40 and 41 or functionally equivalent variants thereof and the TCR beta chain variable domains CDR1, CDR2 and CDR3 comprise respectively, SEQ ID NO: 42, 43 and 44 or functionally equivalent variants thereof; or
(e) the TCR alpha chain variable domains CDR1, CDR2 and CDR3 comprise, respectively, SEQ ID NO: 51, 52 and 53 or functionally equivalent variants thereof and the TCR beta chain variable domains CDR1, CDR2 and CDR3 comprise respectively, SEQ ID NO: 54, 55 and 56 or functionally equivalent variants thereof; or
(f) the TCR alpha chain variable domains CDR1, CDR2 and CDR3 comprise, respectively, SEQ ID NO: 63, 64 and 65 or functionally equivalent variants thereof and the TCR beta chain variable domains CDR1, CDR2 and CDR3 comprise respectively, SEQ ID NO: 66, 67 and 68 or functionally equivalent variants thereof; or
(g) the TCR alpha chain variable domains CDR1, CDR2 and CDR3 comprise, respectively, SEQ ID NO: 75, 76 and 77 or functionally equivalent variants thereof and the TCR beta chain variable domains CDR1, CDR2 and CDR3 comprise respectively, SEQ ID NO: 78, 79 and 80 or functionally equivalent variants thereof; or
(h) the TCR alpha chain variable domains CDR1, CDR2 and CDR3 comprise, respectively, SEQ ID NO: 87, 88 and 89 or functionally equivalent variants thereof and the TCR beta chain variable domains CDR1, CDR2 and CDR3 comprise respectively, SEQ ID NO: 90, 91 and 92 or functionally equivalent variants thereof; or
(i) the TCR alpha chain variable domains CDR1, CDR2 and CDR3 comprise, respectively, SEQ ID NO: 99, 100 and 101 or functionally equivalent variants thereof and the TCR beta chain variable domains CDR1, CDR2 and CDR3 comprise respectively, SEQ ID NO: 102, 103 and 104 or functionally equivalent variants thereof; or
(j) the TCR alpha chain variable domains CDR1, CDR2 and CDR3 comprise, respectively, SEQ ID NO: 111, 112 and 113 or functionally equivalent variants thereof and the TCR beta chain variable domains CDR1, CDR2 and CDR3 comprise respectively, SEQ ID NO: 114, 115 and 116 or functionally equivalent variants thereof; or
(k) the TCR alpha chain variable domains CDR1, CDR2 and CDR3 comprise, respectively, SEQ ID NO: 123, 124 and 125 or functionally equivalent variants thereof and the TCR beta chain variable domains CDR1, CDR2 and CDR3 comprise respectively, SEQ ID NO: 126, 127 and 128 or functionally equivalent variants thereof.

4. The antigen recognizing construct according to claim 2, wherein the TCR comprises:
(a) an alpha chain variable region having the amino acid sequence of SEQ ID NO: 9 and a beta chain variable region having the amino acid sequence of SEQ ID NO: 10; or
(b) an alpha chain variable region having the amino acid sequence of SEQ ID NO: 21 and a beta chain variable region having the amino acid sequence of SEQ ID NO: 22; or
(c) an alpha chain variable region having the amino acid sequence of SEQ ID NO: 33 and a beta chain variable region having the amino acid sequence of SEQ ID NO: 34; or
(d) an alpha chain variable region having the amino acid sequence of SEQ ID NO: 45 and a beta chain variable region having the amino acid sequence of SEQ ID NO: 46; or
(e) an alpha chain variable region having the amino acid sequence of SEQ ID NO: 57 and a beta chain variable region having the amino acid sequence of SEQ ID NO: 58; or
(f) an alpha chain variable region having the amino acid sequence of SEQ ID NO: 69 and a beta chain variable region having the amino acid sequence of SEQ ID NO: 70; or
(g) an alpha chain variable region having the amino acid sequence of SEQ ID NO: 81 and a beta chain variable region having the amino acid sequence of SEQ ID NO: 82; or
(h) an alpha chain variable region having the amino acid sequence of SEQ ID NO: 93 and a beta chain variable region having the amino acid sequence of SEQ ID NO: 94; or
(i) an alpha chain variable region having the amino acid sequence of SEQ ID NO: 105 and a beta chain variable region having the amino acid sequence of SEQ ID NO: 106; or
(j) an alpha chain variable region having the amino acid sequence of SEQ ID NO: 117 and a beta chain variable region having the amino acid sequence of SEQ ID NO: 118; or
(k) an alpha chain variable region having the amino acid sequence of SEQ ID NO: 129 and a beta chain variable region having the amino acid sequence of SEQ ID NO: 130.

5. The antigen recognizing construct according to claim 2, wherein the TCR comprises:
(a) an alpha chain extracellular region having the amino acid sequence of SEQ ID NO: 11 and a beta chain extracellular region having the amino acid sequence of SEQ ID NO: 12; or
(b) an alpha chain extracellular region having the amino acid sequence of SEQ ID NO: 23 and a beta chain extracellular region having the amino acid sequence of SEQ ID NO: 24; or
(c) an alpha chain extracellular region having the amino acid sequence of SEQ ID NO: 35 and a beta chain extracellular region having the amino acid sequence of SEQ ID NO: 36; or
(d) an alpha chain extracellular region having the amino acid sequence of SEQ ID NO: 47 and a beta chain extracellular region having the amino acid sequence of SEQ ID NO: 48; or
(e) an alpha chain extracellular region having the amino acid sequence of SEQ ID NO: 59 and a beta chain extracellular region having the amino acid sequence of SEQ ID NO: 60; or
(f) an alpha chain extracellular region having the amino acid sequence of SEQ ID NO: 71 and a beta chain extracellular region having the amino acid sequence of SEQ ID NO: 72; or
(g) an alpha chain extracellular region having the amino acid sequence of SEQ ID NO: 83 and a beta chain extracellular region having the amino acid sequence of SEQ ID NO: 84; or
(h) an alpha chain extracellular region having the amino acid sequence of SEQ ID NO: 95 and a beta chain extracellular region having the amino acid sequence of SEQ ID NO: 96; or
(i) an alpha chain extracellular region having the amino acid sequence of SEQ ID NO: 107 and a beta chain extracellular region having the amino acid sequence of SEQ ID NO: 108; or
(j) an alpha chain extracellular region having the amino acid sequence of SEQ ID NO: 119 and a beta chain extracellular region having the amino acid sequence of SEQ ID NO: 120; or
(k) an alpha chain extracellular region having the amino acid sequence of SEQ ID NO: 131 and a beta chain extracellular region having the amino acid sequence of SEQ ID NO: 132.

6. The antigen recognizing construct according to claim 3, wherein the TCR comprises:
(a) an alpha chain having the amino acid sequence of SEQ ID NO: 13 and a beta chain having the amino acid sequence of SEQ ID NO: 14; or
(b) an alpha chain having the amino acid sequence of SEQ ID NO: 25 and a beta chain having the amino acid sequence of SEQ ID NO: 26; or
(c) an alpha chain having the amino acid sequence of SEQ ID NO: 37 and a beta chain having the amino acid sequence of SEQ ID NO: 38; or
(d) an alpha chain having the amino acid sequence of SEQ ID NO: 49 and a beta chain having the amino acid sequence of SEQ ID NO: 50; or
(e) an alpha chain having the amino acid sequence of SEQ ID NO: 61 and a beta chain having the amino acid sequence of SEQ ID NO: 62; or
(f) an alpha chain having the amino acid sequence of SEQ ID NO: 73 and a beta chain having the amino acid sequence of SEQ ID NO: 74; or
(g) an alpha chain having the amino acid sequence of SEQ ID NO: 85 and a beta chain having the amino acid sequence of SEQ ID NO: 86; or
(h) an alpha chain having the amino acid sequence of SEQ ID NO: 97 and a beta chain having the amino acid sequence of SEQ ID NO: 98; or
(i) an alpha chain having the amino acid sequence of SEQ ID NO: 109 and a beta chain having the amino acid sequence of SEQ ID NO: 110; or
(j) an alpha chain having the amino acid sequence of SEQ ID NO: 121 and a beta chain having the amino acid sequence of SEQ ID NO: 122.
(k) an alpha chain having the amino acid sequence of SEQ ID NO: 133 and a beta chain having the amino acid sequence of SEQ ID NO: 134.

7. The antigen recognizing construct according to any of claims 1 to 6 which is a soluble TCR wherein the soluble TCR is
(i) a fusion protein comprising the TCR alpha chain variable region and the TCR beta chain variable region,
(ii) a fusion protein comprising a first region comprising the TCR alpha chain extracellular region and a second region comprising the TCR beta chain extracellular sequence,
(iii) A first polypeptide comprising the TCR alpha chain variable region and a second polypeptide comprising the TCR beta chain variable region wherein the first and second polypeptides are connected by a disulfide bond or
(iv) A first polypeptide comprising the TCR alpha chain extracellular region and a second polypeptide comprising the TCR beta chain extracellular region wherein the first and second are connected by a disulfide bond

8. The antigen recognizing construct according to claim 7 wherein
(a) the T-cell engaging immune effector polypeptide is a CD3 effector domain,
(b) the half-life extending domain is an immunoglobulin Fc domain or an albumin binding domain and
(c) the signaling domain is a transmembrane and cytosolic domain from a T-cell receptor.

9. A nucleic acid encoding the antigen recognizing construct according to any one of claims 1 to 8.

10. An expression vector comprising a nucleic acid according to claim 9.

11. A nucleic acid composition encoding the antigen recognizing construct according to any one of claims 1 to 8 selected from the group consisting of:
(i) a composition comprising a first nucleic acid encoding the alpha chain of a TCR and a second nucleic acid encoding the beta chain of said TCR,
(ii) a composition comprising a first nucleic acid encoding a fusion protein comprising the alpha chain of a TCR and a second nucleic acid encoding the beta chain of said TCR,
(iii) a composition comprising a first nucleic acid encoding the alpha chain of a TCR and a second nucleic acid encoding a fusion protein comprising the beta chain of said TCR and a second polypeptide.

12. A cell comprising an antigen recognizing construct of any one of claim 1 to 8, a nucleic acid construct according to any of claim 9, a vector according to claim 10 or the nucleic acid composition according to claim 11.

13. A pharmaceutical composition comprising an antigen recognizing construct of any one of claim 1 to 8, a nucleic acid construct according to claim 9, a vector according to claim 10 or the nucleic acid composition according to claim 11, together with one or more pharmaceutically acceptable carriers or excipients.

14. An in vitro method of detecting cancer cells in a biological sample comprising:
(a) contacting the biological sample with the antigen recognizing construct of any of claims 1 to 8, and
(b) detecting binding of the antigen recognizing construct to cells in the biological sample wherein the cancer cells express a tumoral antigen which comprises the antigenic peptide recognized by the antigen recognizing construct when the antigenic peptide is linked with a HLA.

15. The antigen recognizing construct of any one of claim 1 to 8, the nucleic acid construct according to claim 9, a vector according to claim 10, the nucleic acid composition according to claim 11 or the cell according to claim 12, for use in medicine.

16. The antigen recognizing construct of any one of claim 1 to 8, the nucleic acid construct according to claim 9, a vector according to claim 10, the nucleic acid composition according to claim 11 or the cell according to claim 12, for use in the treatment of cancer.
